(19) Europäisches Patentamt
European Patent Office
Office européen des brevets

(11) EP 2 216 316 A1

(12) **EUROPÄISCHE PATENTANMELDUNG**

(43) Veröffentlichungstag:
**11.08.2010 Patentblatt 2010/32**

(51) Int Cl.:
***C07C 57/32*** *(2006.01)*

(21) Anmeldenummer: **10159644.3**

(22) Anmeldetag: **21.10.2005**

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **04.11.2004 DE 102004053192**

(62) Dokumentnummer(n) der früheren Anmeldung(en) nach Art. 76 EPÜ:
**05795307.7 / 1 809 634**

(71) Anmelder: **Bayer CropScience Aktiengesellschaft**
**42789 Monheim (DE)**

(72) Erfinder:
- **Fischer, Reiner**
  **40789 Monheim (DE)**
- **Lehr, Stefan**
  **65835 Liederbach (DE)**
- **Drewes, Mark**
  **40764 Langenfeld (DE)**
- **Feucht, Dieter**
  **65760 Eschborn (DE)**
- **Malsam, Olga**
  **51503 Rösrath (DE)**
- **Arnold, Christian**
  **40764 Langenfeld (DE)**
- **Auler, Thomas**
  **42799 Leichlingen (DE)**
- **Hills, Martin Jeffrey**
  **65510 Idstein (DE)**
- **Kehne, Heinz**
  **65719 Hofheim (DE)**
- **Rosinger, Christopher Hugh**
  **65719 Hofheim (DE)**
- **Bojack, Guido**
  **65207 Wiesbaden-Naurod (DE)**

Bemerkungen:
Diese Anmeldung ist am 12-04-2010 als Teilanmeldung zu der unter INID-Code 62 erwähnten Anmeldung eingereicht worden.

(54) **Phenylessigsäurederivate**

(57) Verbindungen der Formel (XVIII):

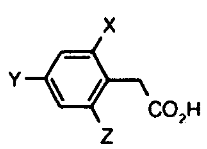

(XVIII)

in welcher
X für Alkoxy steht,
Y für Alkyl steht,
Z für $C_2$-$C_6$-Alkyl steht.

EP 2 216 316 A1

Printed by Jouve, 75001 PARIS (FR)

## Beschreibung

Die Erfindung betrifft neue 2-Alkoxy-6-alkyl-phenylsubstituierte spirocyclische Tetramsäure-Derivate, mehrere Verfahren und Zwischenprodukte zu ihrer Herstellung und ihre Verwendung als Schädlingsbekämpfungsmittel und/oder Herbizide. Die Erfindung betrifft außerdem neue selektiv-herbizide Wirkstoffkombinationen, die 2-Alkoxy-6-alkyl-phenylsubstituierte spirocyclische Tetramsäure-Derivate einerseits und zumindest eine die Kulturpflanzenverträglichkeit verbesserte Verbindung andererseits enthalten und mit besonders gutem Erfolg zur selektiven Unkrautbekämpfung mit verschiedenen Nutzpflanzenkulturen verwendet werden können.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger (Liebigs Ann. Chem. 1985, 1095) synthetisiert. Eine biologische Wirksamkeit dieser Verbindungen wurde nicht beschrieben.

In EP-A-0 262 399 und GB-A-2 266 888 werden ähnlich strukturierte Verbindungen (3-Aryl-pyrrolidin-2,4-dione) offenbart, von denen jedoch keine herbizide, insektizide oder akarizide Wirkung bekannt geworden ist. Bekannt mit herbizider, insektizider oder akarizider Wirkung sind unsubstituierte, bicyclische 3-Aryl-pyrrolidin-2,4-dion-Derivate (EP-A-355 599 und EP-A-415 211) sowie substituierte monocyclische 3-Aryl-pyrrolidin-2,4-dion-Derivate (EP-A-377 893 und EP-A-442 077).

Weiterhin bekannt sind polycyclische 3-Arylpyrrolidin-4-dion-Derivate (EP-A-442 073) sowie 1H-Arylpyrrolidin-dion-Derivate (EP-A-456 063, EP-A-521 334, EP-A-596 298, EP-A-613 884, EP-A-613 885, WO 94/01 997, WO 95/26954, WO 95/20 572, EP-A 0 668 267, WO 96/25 395, WO 96 35 664, WO 97/01 535, WO 97/02 243, WO 97/36 868, WO 97/43275, WO/98/05638, WO 98/06721, WO 98/25928, WO 99/16748, WO 99/24437, WO 99/43649, WO 99/48869, WO 99/55673, WO 01/09092, WO 01/17972, WO 01/23354, WO 01/74770, WO 03/013249, WO 04/007448, WO 04/024688, WO 04/065336, WO 04/080962, WO 04/111042, WO 05/044791, WO 05/044796, WO 05/048710, WO 05/049569 und WO 05/066125.

Die Wirksamkeit und Wirkungsbreite dieser Verbindungen ist jedoch insbesondere bei niedrigen Aufwandmengen und Konzentrationen nicht immer voll zufriedenstellend. Weiterhin ist die Pflanzenverträglichkeit der bekannten Verbindungen nicht immer ausreichend.

Es wurden nun neue Verbindungen der Formel (I)

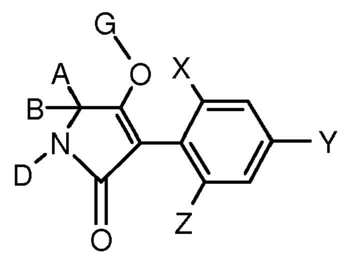

(I)

gefunden,
in welcher

| | |
|---|---|
| X | für Alkoxy steht, |
| Y | für Alkyl steht und |
| Z | für $C_2$-$C_6$-Alkyl steht, |
| A | für Wasserstoff, jeweils gegebenenfalls durch Halogen substituiertes Alkyl, Alkenyl, Alkoxyalkyl, Alkylthioalkyl, gesättigtes oder ungesättigtes, gegebenenfalls substituiertes Cycloalkyl, in welchem gegebenenfalls mindestens ein Ringatom durch ein Heteroatom ersetzt ist, oder jeweils gegebenenfalls durch Halogen, Alkyl, Halogenalkyl, Alkoxy, Halogenalkoxy, Cyano oder Nitro substituiertes Aryl, Arylalkyl oder Hetaryl steht, |
| B | für Wasserstoff, Alkyl oder Alkoxyalkyl steht, oder |
| A und B | gemeinsam mit dem Kohlenstoffatom, an das sie gebunden sind, für einen gesättigten oder ungesät- |

tigten gegebenenfalls mindestens ein Heteroatom enthaltenden $C_4$-$C_8$-Ring, der gegebenenfalls durch Alkyl, Alkoxy oder Halogenalkyl substituiert ist, stehen,

D für Wasserstoff oder einen gegebenenfalls substituierten Rest aus der Reihe Alkyl, Alkenyl, Alkinyl, Alkoxyalkyl, Alkylthioalkyl, gesättigtes oder ungesättigtes Cycloalkyl, in welchem gegebenenfalls eines oder mehrere Ringglieder durch Heteroatome ersetzt sind, Arylalkyl, Aryl, Hetarylalkyl oder Hetaryl steht, oder

A und D gemeinsam mit den Atomen, an die sie gebunden sind für einen gesättigten oder ungesättigten gegebenenfalls durch Heteroatome unterbrochenen unsubstituierten oder substituierten Cyclus stehen,

und

G für Wasserstoff (a) oder für eine der Gruppen

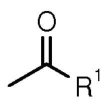 (b), 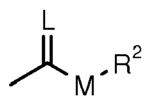 (c), 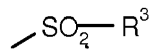 (d),

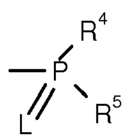 (e), E (f) oder 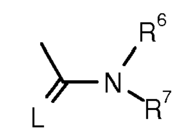 (g),

steht, worin
E für ein Metallionäquivalent oder ein Ammoniumion steht,
L für Sauerstoff oder Schwefel steht,
M für Sauerstoff oder Schwefel steht,

$R^1$ für jeweils gegebenenfalls substituiertes Alkyl, Alkenyl, Alkoxyalkyl, Alkylthioalkyl oder Polyalkoxyalkyl oder für jeweils gegebenenfalls durch Halogen, Alkyl oder Alkoxy substituiertes Cycloalkyl oder Heterocyclyl oder für jeweils gegebenenfalls substituiertes Phenyl, Phenylalkyl, Phenylalkenyl oder Hetaryl steht,

$R^2$ für jeweils gegebenenfalls durch Halogen substituiertes Alkyl, Alkenyl, Alkoxyalkyl oder Polyalkoxyalkyl oder für jeweils gegebenenfalls substituiertes Cycloalkyl, Phenyl oder Benzyl steht,

$R^3$, $R^4$ und $R^5$ unabhängig voneinander für jeweils gegebenenfalls durch Halogen substituiertes Alkyl, Alkoxy, Alkylamino, Dialkylamino, Alkylthio, Alkenylthio oder Cycloalkylthio oder für jeweils gegebenenfalls substituiertes Phenyl, Benzyl, Phenoxy oder Phenylthio stehen,

$R^6$ und $R^7$ unabhängig voneinander für Wasserstoff, für jeweils gegebenenfalls durch Halogen substituiertes Alkyl, Cycloalkyl, Alkenyl, Alkoxy, Alkoxyalkyl, für jeweils gegebenenfalls substituiertes Phenyl oder Benzyl stehen, oder gemeinsam mit dem N-Atom, an das sie gebunden sind, einen gegebenenfalls Sauerstoff oder Schwefel enthaltenden, gegebenenfalls substituierten Cyclus bilden.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.
Unter Einbeziehung der verschiedenen Bedeutungen (a), (b), (c), (d), (e), (f) und (g) der Gruppe G ergeben

sich folgende hauptsächliche Strukturen (I-a) bis (I-g):

**(I-a):**

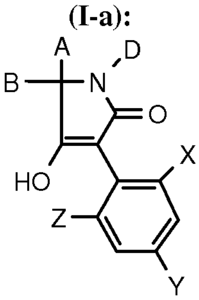

**(I-b):**

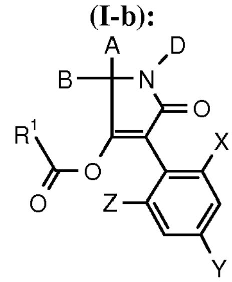

**(I-c):**

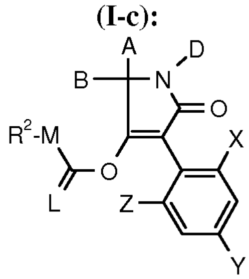

**(I-d):**

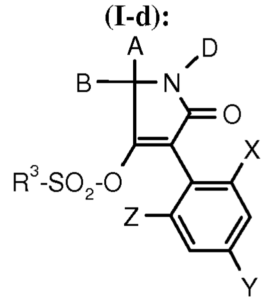

**(I-e):**

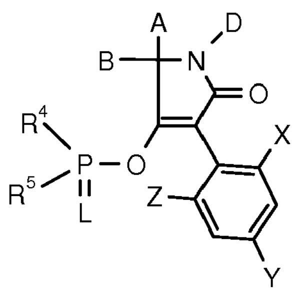

**(I-f):**

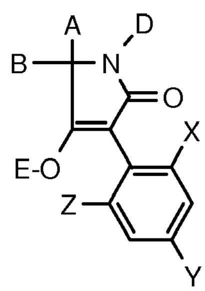

**(I-g):**

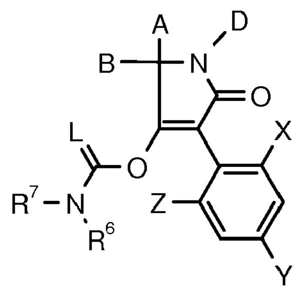

worin

A, B, D, E, L, M, X, Y, Z, $R^1$, $R^2$, $R^3$, $R^4$, $R^5$, $R^6$ und $R^7$ die oben angegebenen Bedeutungen besitzen.

Weiterhin wurde gefunden, dass man die neuen Verbindungen der Formel (I) nach einem der im folgenden beschriebenen Verfahren erhält:

(A) Man erhält Verbindungen der Formel (I-a),

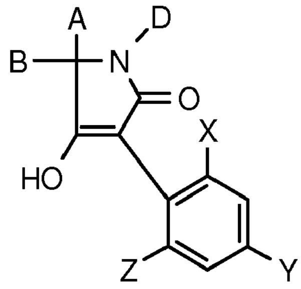

(I-a)

in welcher
A, B, D, X, Y und Z die oben angegebenen Bedeutungen haben,
wenn man
Verbindungen der Formel (II),

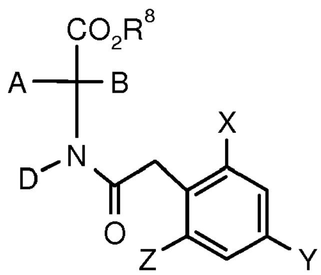

(II)

in welcher
A, B, D, X, Y und Z die oben angegebenen Bedeutungen haben,
und
$R^8$ für Alkyl (bevorzugt $C_1$-$C_6$-Alkyl) steht, in Gegenwart eines Verdünnungsmittels und in Gegenwart einer Base intramolekular kondensiert.

(B) Man erhält Verbindungen der oben gezeigten Formel (I-b), in welcher A, B, D, $R^1$, X, Y und Z die oben angegebenen Bedeutungen haben, wenn man Verbindungen der oben gezeigten Formel (I-a), in welcher A, B, D, X, Y und Z die oben angegebenen Bedeutungen haben,

α) mit Säurehalogeniden der Formel (III),

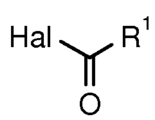

(III)

in welcher
$R^1$ die oben angegebene Bedeutung hat und
Hal für Halogen (insbesondere Chlor oder Brom) steht oder

β) mit Carbonsäureanhydriden der Formel (IV),

$$R^1\text{-CO-O-CO-}R^1 \qquad \text{(IV)}$$

in welcher

$R^1$ die oben angegebene Bedeutung hat, gegebenenfalls in Gegenwart eines Verdünnungsmittels und gegebenenfalls in Gegenwart eines Säurebindemittels umsetzt.

(C) Man erhält Verbindungen der oben gezeigten Formel (I-c), in welcher A, B, D, $R^2$, M, X, Y und Z die oben angegebenen Bedeutungen haben und L für Sauerstoff steht, wenn man Verbindungen der oben gezeigten Formel (I-a), in welcher A, B, D, X, Y und Z die oben angegebenen Bedeutungen haben, jeweils
mit Chlorameisensäureestern oder Chlorameisensäurethioestern der Formel (V),

$R^2$-M-CO-Cl (V)

in welcher
$R^2$ und M die oben angegebenen Bedeutungen haben, gegebenenfalls in Gegenwart eines Verdünnungsmittels und gegebenenfalls in Gegenwart eines Säurebindemittels umsetzt.

(D) Man erhält Verbindungen der oben gezeigten Formel (I-c), in welcher A, B, D, $R^2$, M, X, Y und Z die oben angegebenen Bedeutungen haben und L für Schwefel steht, wenn man Verbindungen der oben gezeigten Formel (I-a), in welcher A, B, D, X, Y und Z die oben angegebenen Bedeutungen haben, jeweils

α) mit Chlormonothioameisensäureestern oder Chlordithioameisensäureestern der Formel (VI),

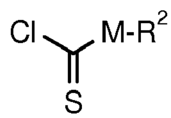

(VI)

in welcher
M und $R^2$ die oben angegebenen Bedeutungen haben, gegebenenfalls in Gegenwart eines Verdünnungsmittels und gegebenenfalls in Gegenwart eines Säurebindemittels umsetzt
oder

β) mit Schwefelkohlenstoff und anschließend mit Verbindungen der Formel (VII),

$R^2$-Hal (VII)

in welcher
$R^2$ die oben angegebene Bedeutung hat und
Hal für Chlor, Brom oder Iod steht, gegebenenfalls in Gegenwart eines Verdünnungsmittels und gegebenenfalls in Gegenwart einer Base umsetzt.

(E) Man erhält Verbindungen der oben gezeigten Formel (I-d), in welcher A, B, D, $R^3$, X, Y und Z die oben angegebenen Bedeutungen haben, wenn man Verbindungen der oben gezeigten Formel (I-a), in welcher A, B, D, X, Y und Z die oben angegebenen Bedeutungen haben, jeweils
mit Sulfonsäurechloriden der Formel (VIII),

$R^3$-$SO_2$-Cl (VIII)

in welcher
$R^3$ die oben angegebene Bedeutung hat, gegebenenfalls in Gegenwart eines Verdünnungsmittels und gegebenenfalls in Gegenwart eines Säurebindemittels umsetzt.

(F) Man erhält Verbindungen der oben gezeigten Formel (I-e), in welcher A, B, D, L, $R^4$, $R^5$, X, Y und Z die oben angegebenen Bedeutungen haben, wenn man Verbindungen der oben gezeigten Formel (I-a), in welcher A, B, D, X, Y und Z die oben angegebenen Bedeutungen haben, jeweils
mit Phosphorverbindungen der Formel (IX),

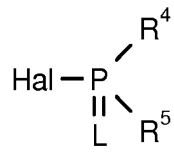

(IX)

in welcher
L, $R^4$ und $R^5$ die oben angegebenen Bedeutungen haben und
Hal für Halogen (insbesondere Chlor oder Brom) steht, gegebenenfalls in Gegenwart eines Verdünnungsmittels und gegebenenfalls in Gegenwart eines Säurebindemittels umsetzt.

(G) Man erhält Verbindungen der oben gezeigten Formel (I-f), in welcher A, B, D, E, X, Y und Z die oben angegebenen Bedeutungen haben, wenn man Verbindungen der Formeln (I-a), in welcher A, B, X, Y und Z die oben angegebenen Bedeutungen haben, jeweils
mit Metallverbindungen oder Aminen der Formeln (X) oder (XI),

$Me(OR^{10})_t$ (X)

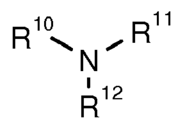

(XI)

in welchen
Me für ein ein- oder zweiwertiges Metall (bevorzugt ein Alkali- oder Erdalkalimetall wie Lithium, Natrium, Kalium, Magnesium oder Calcium),
t für die Zahl 1 oder 2 und
$R^{10}$, $R^{11}$, $R^{12}$ unabhängig voneinander für Wasserstoff oder Alkyl (bevorzugt $C_1$-$C_8$-Alkyl) stehen, gegebenenfalls in Gegenwart eines Verdünnungsmittels umsetzt.

(H) Man erhält Verbindungen der oben gezeigten Formel (I-g), in welcher A, B, D, L, $R^6$, $R^7$, X, Y und Z die oben angegebenen Bedeutungen haben, wenn man Verbindungen der oben gezeigten Formel (I-a), in welcher A, B, D, X, Y und Z die oben angegebenen Bedeutungen haben, jeweils

α) mit Isocyanaten oder Isothiocyanaten der Formel (XII),

$R^6$-N=C=L (XII)

in welcher
$R^6$ und L die oben angegebenen Bedeutungen haben, gegebenenfalls in Gegenwart eines Verdünnungsmittels und gegebenenfalls in Gegenwart eines Katalysators umsetzt oder

β) mit Carbamidsäurechloriden oder Thiocarbamidsäurechloriden der Formel (XIII),

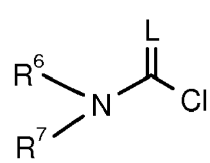

(XIII)

in welcher
L, $R^6$ und $R^7$ die oben angegebenen Bedeutungen haben, gegebenenfalls in Gegenwart eines Verdünnungsmittels und gegebenenfalls in Gegenwart eines Säurebindemittels, umsetzt.

(I) Weiter wurde gefunden, dass man Verbindungen der oben gezeigten Formel (I-a), in welchen A, B, D, X, Y und Z die oben angegebenen Bedeutungen haben, erhält, wenn man Verbindungen der Formel (I-a')

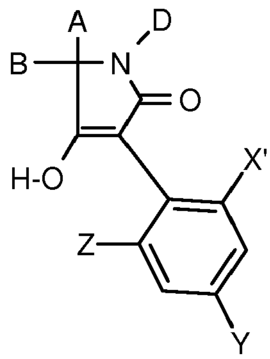

(I-a')

in welchen
A, B, D, Y und Z die oben angegebenen Bedeutungen haben, und
X' für Chlor, Brom, Iod, bevorzugt für Brom steht, mit Alkoholen der Formel (XXV)

Alk- OH (XXV)

in welcher
Alk für Alky steht und in Gegenwart eines Lösungsmittels, einer Base und eines Katalysators umsetzt, wobei als Katalysator insbesondere Kupfer-I-Salze in Frage kommen.

Weiterhin wurde gefunden, dass die neuen Verbindungen der Formel (I) eine sehr gute Wirksamkeit als Schädlingsbekämpfungsmittel, vorzugsweise als Insektizide und/oder Akarizide und/oder als Herbizide aufweisen.

Überraschenderweise wurde nun auch gefunden, dass bestimmte substituierte, cyclische Ketoenole bei gemeinsamer Anwendung mit den im weiteren beschriebenen, die Kulturpflanzen-Verträglichkeit verbessernden Verbindungen (Safenern/Antidots) ausgesprochen gut die Schädigung der Kulturpflanzen verhindern und besonders vorteilhaft als breit wirksame Kombinationspräparate zur selektiven Bekämpfung von unerwünschten Pflanzen in Nutzpflanzenkulturen, wie z.B. in Getreide aber auch Mais, Soja und Reis, verwendet werden können.

Gegenstand der Erfindung sind auch selektiv-herbizide Mittel enthaltend einen wirksamen Gehalt an einer Wirkstoffkombination umfassend als Komponenten

(a') mindestens ein substituiertes Tetramsäure-Derivat der Formel (I), in welcher A, B, D, G, X, Y und Z die oben angegebene Bedeutung haben
und

(b') zumindest eine die Kulturpflanzen-Verträglichkeit verbessernde Verbindung aus der folgenden Gruppe von Verbindungen:

4-Dichloracetyl-1-oxa-4-aza-spiro[4.5]-decan (AD-67, MON-4660), 1-Dichloracetyl-hexahydro-3,3,8a-trimethylpyrrolo [1,2-a]-pyrimidin-6(2H)-on (Dicyclonon, BAS-145138), 4-Dichloracetyl-3,4-dihydro-3-methyl-2H-1,4-benzoxazin (Benoxacor), 5-Chlor-chinolin-8-oxy-essigsäure-(1-methyl-hexylester) (Cloquintocet-mexyl - vgl. auch verwandte Verbindungen in EP-A-86750, EP-A-94349, EP-A-191736, EP-A-492366), 3-(2-Chlor-benzyl)-1-(1-methyl-1-phenylethyl)-harnstoff (Cumyluron), α-(Cyanomethoximino)-phenylacetonitril (Cyometrinil), 2,4-Dichlor-phenoxyessigsäure (2,4-D), 4-(2,4-Dichlor-phenoxy)-buttersäure (2,4-DB), 1-(1-Methyl-1-phenyl-ethyl)-3-(4-methyl-phenyl)-harnstoff (Daimuron, Dymron), 3,6-Dichlor-2-methoxy-benzoesäure (Dicamba), Piperidin-1-thiocarbonsäure-S-1-methyl-1-phenylethylester (Dimepiperate), 2,2-Dichlor-N-(2-oxo-2-(2-propenylamino)-ethyl)-N-(2-propenyl)-acetamid (DKA-24), 2,2-Dichlor-N,N-di-2-propenyl-acetamid (Dichlormid), 4,6-Dichlor-2-phenyl-pyrimidin (Fenclorim), 1-(2,4-Dichlorphenyl)-5-trichlormethyl-1H-1,2,4-triazol-3-carbonsäure-ethylester (Fenchlorazole-ethyl - vgl. auch verwandte Verbindungen in EP-A-174562 und EP-A-346620), 2-Chlor-4-trifluormethyl-thiazol-5-carbonsäure-phenylmethylester (Flurazole), 4-Chlor-N-(1,3-dioxolan-2-yl-methoxy)-α-trifluor-acetophenonoxim (Fluxofenim), 3-Dichloracetyl-5-(2-furanyl)-2,2-dimethyloxazolidin (Furilazole, MON-13900), Ethyl-4,5-dihydro-5,5-diphenyl-3-isoxazolcarboxylat (Isoxadifen-ethyl - vgl. auch verwandte Verbindungen in WO-A-95/07897), 1-(Ethoxycarbonyl)-ethyl-3,6-dichlor-2-methoxybenzoat (Lactidichlor),

(4-Chlor-o-tolyloxy)-essigsäure (MCPA), 2-(4-Chlor-o-tolyloxy)-propionsäure (Mecoprop), Diethyl-1-(2,4-dichlor-phenyl)-4,5-dihydro-5-methyl-1H-pyrazol-3,5-di-carboxylat (Mefenpyr-diethyl - vgl. auch verwandte Verbindungen in WO-A-91/07874) 2-Dichlormethyl-2-methyl-1,3-dioxolan (MG-191), 2-Propenyl-1-oxa-4-azaspiro[4.5]decane-4-carbodithioate (MG-838), 1,8-Naphthalsäureanhydrid, α-(1,3-Dioxolan-2-yl-methoximino)-phenylacetonitril (Oxabetrinil), 2,2-Dichlor-N-(1,3-dioxolan-2-yl-methyl)-N-(2-propenyl)-acetamid (PPG-1292), 3-Dichloracetyl-2,2-dimethyl-oxazolidin (R-28725), 3-Dichloracetyl-2,2,5-trimethyloxazolidin (R-29148), 4-(4-Chlor-o-tolyl)-buttersäure, 4-(4-Chlor-phenoxy)-buttersäure, Diphenylmethoxyessigsäure, Diphenylmethoxyessigsäure-methylester, Diphenylmethoxyessigsäure-ethylester, 1-(2-Chlor-phenyl)-5-phenyl-1H-pyrazol-3-carbonsäure-methylester, 1-(2,4-Dichlor-phenyl)-5-methyl-1H-pyrazol-3-carbonsäure-ethylester, 1-(2,4-Dichlor-phenyl)-5-isopropyl-1H-pyrazol-3-carbonsäure-ethylester, 1-(2,4-Dichlor-phenyl)-5-(1,1-dimethyl-ethyl)-1H-pyrazol-3-carbonsäureethylester, 1-(2,4-Dichlor-phenyl)-5-phenyl-1H-pyrazol-3-carbonsäure-ethylester (vgl. auch verwandte Verbindungen in EP-A-269806 und EP-A-333131), 5-(2,4-Dichlor-benzyl)-2-isoxazolin-3-carbonsäure-ethylester, 5-Phenyl-2-isoxazolin-3-carbonsäure-ethylester, 5-(4-Fluor-phenyl)-5-phenyl-2-isoxazolin-3-carbonsäure-ethylester (vgl. auch verwandte Verbindungen in WO-A-91/08202), 5-Chlor-chinolin-8-oxy-essigsäure-(1,3-dimethyl-but-1-yl)-ester, 5-Chlor-chinolin-8-oxy-essigsäure-4-allyloxy-butylester, 5-Chlor-chinolin-8-oxy-essigsäure-1-allyloxy-prop-2-yl-ester, 5-Chlor-chinoxalin-8-oxy-essigsäure-methylester, 5-Chlor-chinolin-8-oxy-essigsäure-ethylester, 5-Chlor-chinoxalin-8-oxy-essigsäure-allylester, 5-Chlor-chinolin-8-oxy-essigsäure-2-oxo-prop-1-yl-ester, 5-Chlor-chinolin-8-oxy-malonsäure-diethylester, 5-Chlor-chinoxalin-8-oxy-malonsäure-diallylester, 5-Chlor-chinolin-8-oxy-malonsäure-diethylester (vgl. auch verwandte Verbindungen in EP-A-582198), 4-Carboxy-chroman-4-yl-essigsäure (AC-304415, vgl. EP-A-613618), 4-Chlorphenoxy-essigsäure, 3,3'-Dimethyl-4-methoxy-benzophenon, 1-Brom-4-chlormethylsulfonylbenzol, 1-[4-(N-2-Methoxybenzoylsulfamoyl)-phenyl]-3-methyl-harnstoff (alias N-(2-Methoxybenzoyl)-4-[(methylamino-carbonyl)-amino]-benzolsulfonamid), 1-[4-(N-2-Methoxybenzoylsulfamoyl)-phenyl]-3,3-dimethyl-harnstoff, 1-[4-(N-4,5-Dimethylbenzoylsulfamoyl)-phenyl]-3-methyl-harnstoff, 1-[4-(N-Naphthylsulfamoyl)-phenyl]-3,3-dimethyl-harnstoff, N-(2-Methoxy-5-methyl-benzoyl)-4-(cyclopropylaminocarbonyl)-benzolsulfonamid,
und/oder eine der folgenden durch allgemeine Formeln definierten Verbindungen
der allgemeinen Formel (IIa)

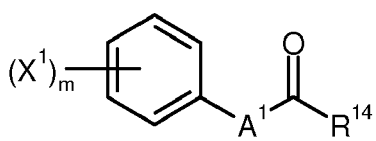

(IIa)

oder der allgemeinen Formel (IIb)

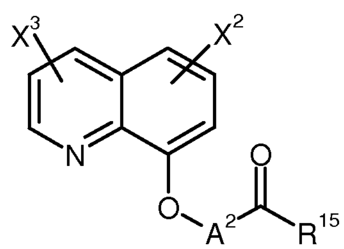

(IIb)

oder der Formel (IIc)

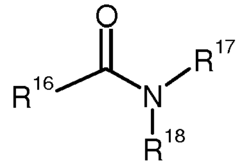

(IIc)

wobei

m für eine Zahl 0, 1, 2, 3, 4 oder 5 steht,

$A^1$ für eine der nachstehend skizzierten divalenten heterocyclischen Gruppierungen steht,

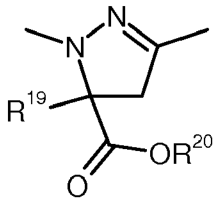
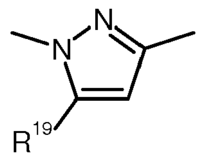
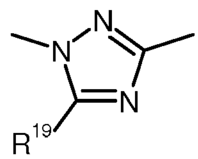
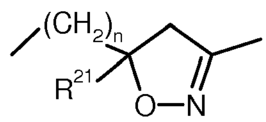

n für eine Zahl 0, 1, 2, 3, 4 oder 5 steht,

$A^2$ für gegebenenfalls durch $C_1$-$C_4$-Alkyl und/oder $C_1$-$C_4$-Alkoxy-carbonyl und/oder $C_1$-$C_4$-Alkenyloxy- carbonyl substituiertes Alkandiyl mit 1 oder 2 Kohlenstoffatomen steht,

$R^{14}$ fürHydroxy,Mercapto,Amino,$C_1$-$C_6$-Alkoxy,$C_1$-$C_6$-Alkylthio,$C_1$-$C_6$-AlkylaminooderDi-($C_1$-$C_4$-alkyl)-amino steht,

$R^{15}$ für Hydroxy, Mercapto, Amino, $C_1$-$C_7$-Alkoxy, $C_1$-$C_6$-Alkenyloxy, $C_1$-$C_6$-Alkenyloxy-$C_1$-$C_6$-alkoxy, $C_1$-$C_6$-Alkylthio, $C_1$-$C_6$-Alkylamino oder Di-($C_1$-$C_4$-alkyl)-amino steht,

$R^{16}$ für jeweils gegebenenfalls durch Fluor, Chlor und/oder Brom substituiertes $C_1$-$C_4$-Alkyl steht,

$R^{17}$ für Wasserstoff, jeweils gegebenenfalls durch Fluor, Chlor und/oder Brom substituiertes $C_1$-$C_6$-Alkyl, $C_2$-$C_6$-Alkenyl oder $C_2$-$C_6$-Alkinyl, $C_1$-$C_4$-Alkoxy-$C_1$-$C_4$-alkyl, Dioxolanyl-$C_1$-$C_4$-alkyl, Furyl, Furyl-$C_1$-$C_4$-alkyl, Thienyl, Thiazolyl, Piperidinyl, oder gegebenenfalls durch Fluor, Chlor und/oder Brom oder $C_1$-$C_4$-Alkyl substituiertes Phenyl steht,

$R^{18}$ für Wasserstoff, jeweils gegebenenfalls durch Fluor, Chlor und/oder Brom substituiertes $C_1$-$C_6$-Alkyl, $C_2$-$C_6$-Alkenyl oder $C_2$-$C_6$-Alkinyl, $C_1$-$C_4$-Alkoxy-$C_1$-$C_4$-alkyl, Dioxolanyl-$C_1$-$C_4$-alkyl, Furyl, Furyl-$C_1$-$C_4$-alkyl, Thienyl, Thiazolyl, Piperidinyl, oder gegebenenfalls durch Fluor, Chlor und/oder Brom oder $C_1$-$C_4$-Alkyl substituiertes Phenyl, steht, oder

$R^{17}$ und $R^{18}$ auch gemeinsam für jeweils gegebenenfalls durch $C_1$-$C_4$-Alkyl, Phenyl, Furyl, einen annellierten Benzolring oder durch zwei Substituenten, die gemeinsam mit dem C-Atom, an das sie gebunden sind, einen 5- oder 6-gliedrigen Carboxyclus bilden, substituiertes $C_3$-$C_6$-Alkandiyl oder $C_2$-$C_5$-Oxaalkandiyl stehen,

$R^{19}$ für Wasserstoff, Cyano, Halogen, oder für jeweils gegebenenfalls durch Fluor, Chlor und/oder Brom substituiertes $C_1$-$C_4$-Alkyl, $C_3$-$C_6$-Cycloalkyl oder Phenyl steht,

$R^{20}$ für Wasserstoff, gegebenenfalls durch Hydroxy, Cyano, Halogen oder $C_1$-$C_4$-Alkoxy substituiertes $C_1$-$C_6$-Alkyl, $C_3$-$C_6$-Cycloalkyl oder Tri-($C_1$-$C_4$-alkyl)-silyl steht,

$R^{21}$ für Wasserstoff, Cyano, Halogen, oder für jeweils gegebenenfalls durch Fluor, Chlor und/oder Brom substituiertes $C_1$-$C_4$-Alkyl, $C_3$-$C_6$-Cycloalkyl oder Phenyl steht,

$X^1$ für Nitro, Cyano, Halogen, $C_1$-$C_4$-Alkyl, $C_1$-$C_4$-Halogenalkyl, $C_1$-$C_4$-Alkoxy oder $C_1$-$C_4$-Halogenalkoxy steht,

$X^2$ für Wasserstoff, Cyano, Nitro, Halogen, $C_1$-$C_4$-Alkyl, $C_1$-$C_4$-Halogenalkyl, $C_1$-$C_4$-Alkoxy oder $C_1$-$C_4$-Halogenalkoxy steht,

$X^3$ für Wasserstoff, Cyano, Nitro, Halogen, $C_1$-$C_4$-Alkyl, $C_1$-$C_4$-Halogenalkyl, $C_1$-$C_4$-Alkoxy oder $C_1$-$C_4$-Halogenalkoxy steht,

und/oder die folgenden durch allgemeine Formeln definierten Verbindungen der allgemeinen Formel (IId)

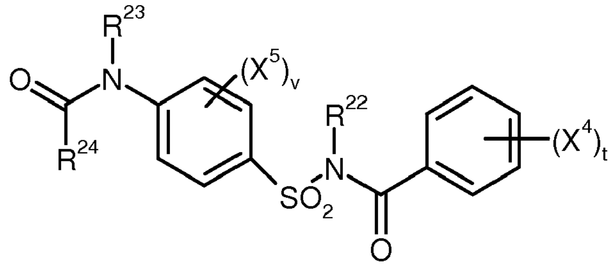

(IId)

oder der allgemeinen Formel (IIe)

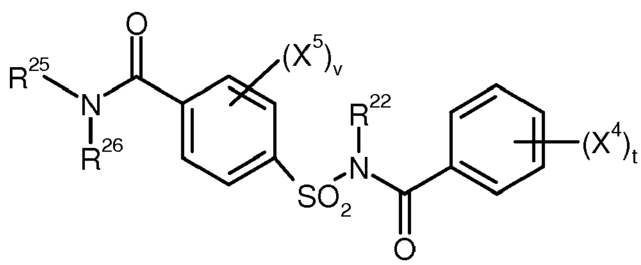

(IIe)

wobei

t für eine Zahl 0, 1, 2, 3, 4 oder 5 steht,

v für eine Zahl 0, 1, 2, 3, 4 oder 5 steht,

$R^{22}$ für Wasserstoff oder $C_1$-$C_4$-Alkyl steht,

$R^{23}$ für Wasserstoff oder $C_1$-$C_4$-Alkyl steht,

$R^{24}$ für Wasserstoff, jeweils gegebenenfalls durch Cyano, Halogen oder $C_1$-$C_4$-Alkoxy substituiertes $C_1$-$C_6$-Alkyl, $C_1$-$C_6$-Alkoxy, $C_1$-$C_6$-Alkylthio, $C_1$-$C_6$-Alkylamino oder Di-($C_1$-$C_4$-alkyl)amino, oder jeweils gegebenenfalls durch Cyano, Halogen oder $C_1$-$C_4$-Alkyl substituiertes $C_3$-$C_6$-Cycloalkyl, $C_3$-$C_6$-Cycloalkyloxy, $C_3$-$C_6$-Cycloalkylthio oder $C_3$-$C_6$-Cycloalkylamino steht,

$R^{25}$ für Wasserstoff, gegebenenfalls durch Cyano, Hydroxy, Halogen oder $C_1$-$C_4$-Alkoxy substituiertes $C_1$-$C_6$-Alkyl, jeweils gegebenenfalls durch Cyano oder Halogen substituiertes $C_3$-$C_6$-Alkenyl oder $C_3$-$C_6$-Alkinyl, oder gegebenenfalls durch Cyano, Halogen oder $C_1$-$C_4$-Alkyl substituiertes $C_3$-$C_6$-Cycloalkyl steht,

$R^{26}$ für Wasserstoff, gegebenenfalls durch Cyano, Hydroxy, Halogen oder $C_1$-$C_4$-Alkoxy substituiertes $C_1$-$C_6$-Alkyl, jeweils gegebenenfalls durch Cyano oder Halogen substituiertes $C_3$-$C_6$-Alkenyl oder $C_3$-$C_6$-Alkinyl, gegebenenfalls durch Cyano, Halogen oder $C_1$-$C_4$-Alkyl substituiertes $C_3$-$C_6$-Cycloalkyl, oder gegebenenfalls durch Nitro, Cyano, Halogen, $C_1$-$C_4$-Alkyl, $C_1$-$C_4$-Halogenalkyl, $C_1$-$C_4$-Alkoxy oder $C_1$-$C_4$-Halogenalkoxy substituiertes Phenyl steht, oder zusammen mit $R^{25}$ für jeweils gegebenenfalls durch $C_1$-$C_4$-Alkyl substituiertes $C_2$-$C_6$-Alkandiyl oder $C_2$-$C_5$-Oxaalkandiyl steht,

$X^4$ für Nitro, Cyano, Carboxy, Carbamoyl, Formyl, Sulfamoyl, Hydroxy, Amino, Halogen, $C_1$-$C_4$-Alkyl, $C_1$-$C_4$-Halogenalkyl, $C_1$-$C_4$-Alkoxy oder $C_1$-$C_4$-Halogenalkoxy steht, und

$X^5$ für Nitro, Cyano, Carboxy, Carbamoyl, Formyl, Sulfamoyl, Hydroxy, Amino, Halogen, $C_1$-$C_4$-Alkyl, $C_1$-$C_4$-Halo-

genalkyl, $C_1$-$C_4$-Alkoxy oder $C_1$-$C_4$-Halogenalkoxy steht.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im folgenden erläutert:

X steht bevorzugt für $C_1$-$C_4$-Alkoxy,

Y steht bevorzugt für $C_1$-$C_3$-Alkyl,

Z steht bevorzugt für Ethyl, n-Propyl oder n-Butyl,

A steht bevorzugt für Wasserstoff oder gegebenenfalls durch Halogen substituiertes $C_1$-$C_{12}$-Alkyl, $C_3$-$C_8$-Alkenyl, $C_1$-$C_{10}$-Alkoxy-$C_1$-$C_8$-alkyl, $C_1$-$C_{10}$-Alkylthio-$C_1$-$C_6$-alkyl, gegebenenfalls durch Halogen, $C_1$-$C_6$-Alkyl oder $C_1$-$C_6$-Alkoxy substituiertes $C_3$-$C_8$-Cycloalkyl, in welchem gegebenenfalls ein oder zwei nicht direkt benachbarte Ringglieder durch Sauerstoff und/oder Schwefel ersetzt sind oder für jeweils gegebenenfalls durch Halogen, $C_1$-$C_6$-Alkyl, $C_1$-$C_6$-Halogenalkyl, $C_1$-$C_6$-Alkoxy, $C_1$-$C_6$-Halogenalkoxy, Cyano oder Nitro substituiertes Phenyl, Naphthyl, Hetaryl mit 5 bis 6 Ringatomen (beispielsweise Furanyl, Pyridyl, Imidazolyl, Triazolyl, Pyrazolyl, Pyrimidyl, Thiazolyl oder Thienyl), Phenyl-$C_1$-$C_6$-alkyl oder Naphthyl-$C_1$-$C_6$-alkyl,

B steht bevorzugt für Wasserstoff, $C_1$-$C_{12}$-Alkyl oder $C_1$-$C_8$Alkoxy-$C_1$-$C_6$-alkyl oder

A, B und das Kohlenstoffatom an das sie gebunden sind, stehen bevorzugt für gesättigtes $C_4$-$C_8$-Cycloalkyl, worin gegebenenfalls eine Methylengruppe durch Sauerstoff oder Schwefel ersetzt ist und welches gegebenenfalls durch $C_1$-$C_6$-Alkyl, $C_1$-$C_4$-Halogenalkyl oder $C_1$-$C_6$-Alkoxy substituiert ist,

D steht bevorzugt für Wasserstoff, für jeweils gegebenenfalls einfach bis dreifach durch Halogen substituiertes $C_1$-$C_8$-Alkyl, $C_1$-$C_8$-Alkenyl, $C_1$-$C_6$-Alkoxy-$C_2$-$C_4$-alkyl oder $C_1$-$C_6$-Alkylthio-$C_2$-$C_4$-alkyl, für gegebenenfalls einfach bis dreifach durch Halogen, $C_1$-$C_4$-Alkyl, $C_1$-$C_4$-Alkoxy oder $C_1$-$C_2$-Halogenalkyl substituiertes $C_3$-$C_8$-Cycloalkyl,

A und D stehen gemeinsam bevorzugt für eine $C_3$-$C_8$-Alkandiyl- oder $C_3$-$C_6$-Alkendiylgruppe, in welchen jeweils gegebenenfalls durch eine Methylengruppe durch Sauerstoff oder Schwefel ersetzt ist und welche jeweils gegebenenfalls einfach bis zweifach substituiert sind durch Halogen, Hydroxy, $C_1$-$C_4$-Alkyl oder $C_1$-$C_4$-Alkoxy, oder durch eine weitere, einen ankondensierten Ring bildende $C_3$-$C_6$-Alkandiyl-, $C_3$-$C_6$-Alkendiyl- oder $C_4$-$C_6$-Alkandiendiylgruppe,

G steht bevorzugt für Wasserstoff (a) oder für eine der Gruppen

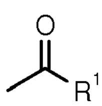 (b), 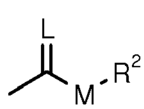 (c), 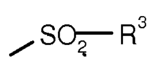 (d),

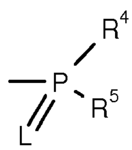 (e), E (f) oder 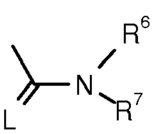 (g) ,

in welchen
E für ein Metallionäquivalent oder ein Ammoniumion steht,
L für Sauerstoff oder Schwefel steht und
M für Sauerstoff oder Schwefel steht,

$R^1$ steht bevorzugt für jeweils gegebenenfalls einfach bis siebenfach durch Halogen, einfach bis zweifach

durch Cyano, einfach durch $COR^{13}$, C=N-$OR^{13}$, $CO_2R^{13}$, oder

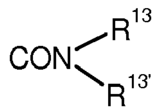

substituiertes $C_1$-$C_{20}$-Alkyl, $C_2$-$C_{20}$-Alkenyl, $C_1$-$C_6$-Alkoxy-$C_1$-$C_6$-alkyl, $C_1$-$C_6$-Alkylthio-$C_1$- $C_6$-alkyl oder Poly-$C_1$-$C_4$-alkoxy-$C_1$-$C_4$-alkyl oder für gegebenenfalls einfach bis dreifach durch Halogen, $C_1$-$C_4$-Alkyl oder $C_1$-$C_4$-Alkoxy substituiertes $C_3$-$C_8$-Cycloalkyl, in welchem gegebenenfalls eine oder zwei nicht direkt benachbarte Methylengruppen durch Sauerstoff und/oder Schwefel ersetzt sind,
für jeweils gegebenenfalls einfach bis dreifach durch Halogen, Cyano, Nitro, $C_1$-$C_6$-Alkyl, $C_1$-$C_6$-Alkoxy, $C_1$-$C_6$-Halogenalkyl, $C_1$-$C_6$-Halogenalkoxy, $C_1$-$C_6$-Alkylthio, $C_1$-$C_6$-Alkylsulfinyl oder $C_1$-$C_6$-Alkylsulfonyl substituiertes Phenyl, Phenyl-$C_1$-$C_2$-alkyl oder Phenyl-$C_1$-$C_2$-alkenyl,
für gegebenenfalls einfach bis zweifach durch Halogen oder $C_1$-$C_6$-Alkyl substituiertes 5- oder 6-gliedriges Hetaryl mit ein oder zwei Heteroatomen aus der Reihe Sauerstoff, Schwefel und Stickstoff,

$R^2$ steht bevorzugt für jeweils gegebenenfalls einfach bis dreifach durch Halogen substituiertes $C_1$-$C_{20}$-Alkyl, $C_2$-$C_{20}$-Alkenyl, $C_1$-$C_6$-Alkoxy-$C_2$-$C_6$-alkyl oder Poly-$C_1$-$C_6$-alkoxy-$C_2$-$C_6$-alkyl,
für gegebenenfalls einfach bis zweifach durch Halogen, $C_1$-$C_6$-Alkyl oder $C_1$-$C_6$-Alkoxy substituiertes $C_3$-$C_8$-Cycloalkyl oder
für jeweils gegebenenfalls einfach bis dreifach durch Halogen, Cyano, Nitro, $C_1$-$C_6$-Alkyl, $C_1$-$C_6$-Alkoxy, $C_1$-$C_6$-Halogenalkyl oder $C_1$-$C_6$-Halogenalkoxy substituiertes Phenyl oder Benzyl,

$R^3$ steht bevorzugt für gegebenenfalls einfach bis mehrfach durch Halogen substituiertes $C_1$-$C_8$-Alkyl oder für jeweils gegebenenfalls einfach bis zweifach durch Halogen, $C_1$-$C_6$-Alkyl, $C_1$-$C_6$-Alkoxy, $C_1$-$C_4$-Halogenalkyl, $C_1$-$C_4$-Halogenalkoxy, Cyano oder Nitro substituiertes Phenyl oder Benzyl,

$R^4$ und $R^5$ stehen unabhängig voneinander bevorzugt für jeweils gegebenenfalls einfach bis dreifach durch Halogen substituiertes $C_1$-$C_8$-Alkyl, $C_1$-$C_8$-Alkoxy, $C_1$-$C_8$-Alkylamino, Di-($C_1$-$C_8$--alkyl)amino, $C_1$-$C_8$-Alkylthio oder $C_2$-$C_8$-Alkenylthio oder für jeweils gegebenenfalls einfach bis dreifach durch Halogen, Nitro, Cyano, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkyl oder $C_1$-$C_4$-Halogenalkyl substituiertes Phenyl, Phenoxy oder Phenylthio,

$R^6$ und $R^7$ stehen unabhängig voneinander bevorzugt für Wasserstoff, für jeweils gegebenenfalls einfach bis dreifach durch Halogen substituiertes $C_1$-$C_8$-Alkyl, $C_3$-$C_8$-Cycloalkyl, $C_1$-$C_8$-Alkoxy, $C_3$-$C_8$-Alkenyl oder $C_1$-$C_8$-Alkoxy-$C_2$-$C_8$-alkyl, für jeweils gegebenenfalls einfach bis dreifach durch Halogen, $C_1$-$C_8$-Alkyl, $C_1$-$C_8$-Halogenalkyl oder $C_1$-$C_8$-Alkoxy substituiertes Phenyl oder Benzyl oder zusammen für einen gegebenenfalls einfach bis zweifach durch $C_1$-$C_4$-Alkyl substituierten $C_3$-$C_6$-Alkylenrest, in welchem gegebenenfalls eine Methylengruppe durch Sauerstoff oder Schwefel ersetzt ist,

$R^{13}$ steht bevorzugt für jeweils gegebenenfalls einfach bis dreifach durch Halogen substituiertes $C_1$-$C_6$-Alkyl, $C_3$-$C_6$-Alkenyl, $C_3$-$C_6$-Alkinyl oder $C_1$-$C_4$-Alkoxy-$C_2$-$C_4$-alkyl oder für gegebenenfalls einfach bis zweifach durch Halogen, $C_1$-$C_2$-Alkyl oder $C_1$-$C_2$-Alkoxy substituiertes $C_3$-$C_6$-Cycloalkyl, in welchem gegebenenfalls eine oder zwei nicht direkt benachbarte Methylengruppen durch Sauerstoff ersetzt sind oder für jeweils gegebenenfalls einfach bis zweifach, durch Halogen, $C_1$-$C_4$-Alkyl, $C_1$-$C_4$-Alkoxy, $C_1$-$C_2$-Halogenalkyl, $C_1$-$C_2$-Halogenalkoxy, Cyano oder Nitro substituiertes Phenyl oder Phenyl-$C_1$-$C_2$-alkyl,

$R^{13'}$ steht bevorzugt für Wasserstoff, $C_1$-$C_6$-Alkyl oder $C_3$-$C_6$-Alkenyl.

In den als bevorzugt genannten Restedefinitionen steht Halogen für Fluor, Chlor, Brom und Iod, insbesondere für Fluor, Chlor und Brom.

X steht besonders bevorzugt für Methoxy, Ethoxy, n-Propoxy,

Y steht besonders bevorzugt für Methyl oder Ethyl,

Z steht besonders bevorzugt für Ethyl oder n-Propyl,

A steht besonders bevorzugt für Wasserstoff, $C_1$-$C_6$-Alkyl, $C_1$-$C_2$-Halogenalkyl, $C_1$-$C_4$-Alkoxy-$C_1$-$C_3$-alkyl oder für gegebenenfalls einfach bis zweifach durch Fluor, Chlor, $C_1$-$C_2$-Alkyl oder $C_1$-$C_2$-Alkoxy substituiertes $C_3$-$C_6$-Cycloalkyl,

B steht besonders bevorzugt für Wasserstoff, $C_1$-$C_2$-Alkyl oder $C_1$-$C_4$-Alkoxy-$C_1$-$C_2$-alkyl,

A, B und das Kohlenstoffatom, an das sie gebunden sind, stehen besonders bevorzugt für gesättigtes $C_3$-$C_7$-Cycloalkyl, worin gegebenenfalls eine Methylenruppe durch Sauerstoff ersetzt ist und welches gegebenenfalls einfach durch $C_1$-$C_4$-Alkyl, $C_1$-$C_2$-Halogenalkyl oder $C_1$-$C_4$-Alkoxy substituiert ist,

D steht besonders bevorzugt für Wasserstoff,

D steht auch besonders bevorzugt für jeweils gegebenenfalls einfach bis dreifach durch Fluor oder Chlor substituiertes $C_1$-$C_6$-Alkyl, $C_3$-$C_6$-Alkenyl, $C_1$-$C_4$-Alkoxy- $C_2$-$C_3$-alkyl oder $C_1$-$C_4$-Alkylthio-$C_2$-$C_3$-alkyl, für gegebenenfalls einfach bis zweifach durch Fluor, Chlor, $C_1$-$C_2$-Alkyl, $C_1$-$C_2$-Alkoxy oder Trifluormethyl substituiertes $C_3$-$C_6$-Cycloalkyl, mit der Maßgabe, dass dann
A nur für Wasserstoff oder $C_1$-$C_3$-Alkyl steht,

A und D stehen gemeinsam besonders bevorzugt für eine $C_3$-$C_5$-Alkandiylgruppe, worin gegebenenfalls eine Methylengruppe durch Sauerstoff oder Schwefel ersetzt ist und welche gegebenenfalls einfach bis zweifach durch $C_1$-$C_2$-Alkyl oder $C_1$-$C_2$-Alkoxy substituiert ist,
oder A und D stehen gemeinsam mit den Atomen, an die sie gebunden sind, für eine der Gruppen AD-1 bis AD-10

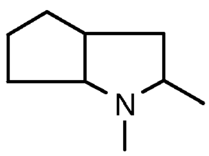
AD-1

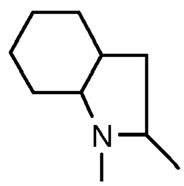
AD-2

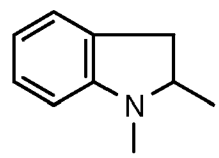
AD-3

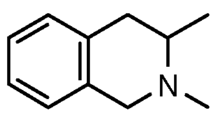
AD-4

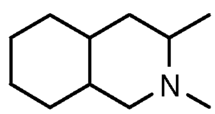
AD-5

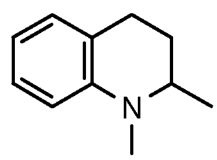
AD-6

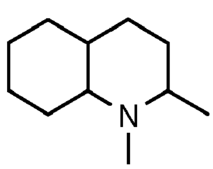
AD-7

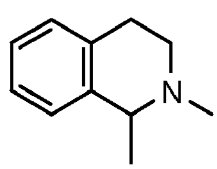
AD-8

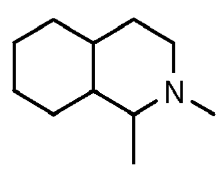
AD-9

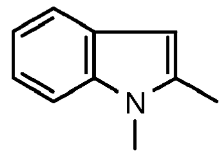

AD-10

G steht <u>besonders bevorzugt</u> für Wasserstoff (a) oder für eine der Gruppen

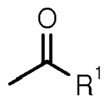 (b), 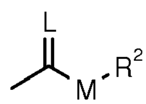 (c), 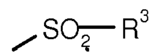 (d),

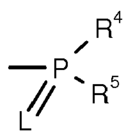 (e), E (f) oder 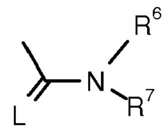 (g)

in welchen
E für ein Metallionäquivalent oder ein Ammoniumion steht,
L für Sauerstoff oder Schwefel steht und
M für Sauerstoff oder Schwefel steht,

$R^1$ steht <u>besonders bevorzugt</u> für jeweils gegebenenfalls einfach bis fünffach durch Fluor oder Chlor, einfach durch Cyano, einfach durch CO-$R^{13}$, C=N-O$R^{13}$ oder $CO_2R^{13}$ substituiertes $C_1$-$C_{10}$-Alkyl, $C_2$-$C_{10}$-Alkenyl, $C_1$-$C_4$-Alkoxy-$C_1$-$C_2$-alkyl, $C_1$-$C_4$-Alkylthio-$C_1$-$C_2$-alkyl oder Poly-$C_1$-$C_3$-alkoxy-$C_1$-$C_2$-alkyl oder für gegebenenfalls einfach bis zweifach durch Fluor, Chlor, $C_1$-$C_2$-Alkyl oder $C_1$-$C_2$-Alkoxy substituiertes $C_3$-$C_6$-Cycloalkyl, in welchem gegebenenfalls eine oder zwei nicht direkt benachbarte Methylengruppen durch Sauerstoff ersetzt sind,
für jeweils gegebenenfalls einfach bis zweifach durch Fluor, Chlor, Brom, Cyano, Nitro, $C_1$-$C_4$-Alkyl, $C_1$-$C_4$-Alkylthio,$C_1$-$C_4$-Alkylsulfinyl,$C_1$-$C_4$-Alkylsulfonyl,$C_1$-$C_4$-Alkoxy,$C_1$-$C_2$-HalogenalkyloderC$_1$-$C_2$-Halogenalkoxy substituiertes Phenyl oder Benzyl,
für jeweils gegebenenfalls einfach bis zweifach durch Fluor, Chlor, Brom oder $C_1$-$C_2$-Alkyl substituiertes Pyrazolyl, Thiazolyl, Pyridyl, Pyrimidyl, Furanyl oder Thienyl,

$R^2$ steht <u>besonders bevorzugt</u> für jeweils gegebenenfalls einfach bis dreifach durch Fluor oder Chlor substituiertes $C_1$-$C_{10}$-Alkyl, $C_2$-$C_{10}$-Alkenyl, $C_1$-$C_4$-Alkoxy-$C_2$-$C_4$-alkyl oder Poly-$C_1$-$C_4$-alkoxy-$C_2$-$C_4$-alkyl,
für gegebenenfalls einfach durch Fluor, Chlor, $C_1$-$C_2$-Alkyl oder $C_1$-$C_2$-Alkoxy substituiertes $C_3$-$C_7$-Cyaoalkyl oder
für jeweils gegebenenfalls einfach bis zweifach durch Fluor, Chlor, Brom, Cyano, Nitro, $C_1$-$C_4$-Alkyl, Methoxy, Trifluormethyl oder Trifluormethoxy substituiertes Phenyl oder Benzyl,

$R^3$ steht <u>besonders bevorzugt</u> für gegebenenfalls einfach bis dreifach durch Fluor oder Chlor substituiertes $C_1$-$C_4$-Alkyl oder für jeweils gegebenenfalls einfach durch Fluor, Chlor, Brom, $C_1$-$C_4$-Alkyl, $C_1$-$C_4$-Alkoxy, Trifluormethyl, Trifluormethoxy, Cyano oder Nitro substituiertes Phenyl oder Benzyl,

$R^4$ und $R^5$ stehen unabhängig voneinander <u>besonders bevorzugt</u> für jeweils gegebenenfalls einfach bis dreifach durch Fluor oder Chlor substituiertes $C_1$-$C_6$-Alkyl, $C_1$-$C_6$-Alkoxy, $C_1$-$C_6$-Alkyl-amino, Di-($C_1$-$C_6$-alkyl)amino, $C_1$-$C_6$-Alkylthio oder $C_3$-$C_4$-Alkenylthio oder für jeweils gegebenenfalls einfach bis zweifach durch Fluor, Chlor, Brom, Nitro, Cyano, $C_1$-$C_3$-Alkoxy, Trifluormethoxy, $C_1$-$C_3$-Alkylthio, $C_1$-$C_3$-Alkyl oder Trifluormethyl substituiertes Phenyl, Phenoxy oder Phenylthio,

$R^6$ und $R^7$ stehen unabhängig voneinander besonders bevorzugt für Wasserstoff, für jeweils gegebenenfalls einfach bis dreifach durch Fluor oder Chlor substituiertes $C_1$-$C_6$-Alkyl, $C_3$-$C_6$-Cycloalkyl, $C_1$-$C_4$-Alkoxy, $C_3$-$C_6$-Alkenyl oder $C_1$-$C_6$-Alkoxy-$C_2$-$C_6$-alkyl, für gegebenenfalls einfach bis zweifach durch Fluor, Chlor, Brom, Trifluormethyl, $C_1$-$C_4$-Alkyl oder $C_1$-$C_4$-Alkoxy substituiertes Phenyl, oder zusammen für einen gegebenenfalls einfach bis zweifach durch Methyl substituierten $C_5$-$C_6$-Alkylenrest, in welchem gegebenenfalls eine Methylengruppe durch Sauerstoff ersetzt ist,

$R^{13}$ steht besonders bevorzugt für $C_1$-$C_4$-Alkyl, $C_3$-$C_4$-Alkenyl, $C_3$-$C_4$-Alkinyl oder $C_1$-$C_4$-Alkoxy-$C_2$-$C_3$-alkyl oder $C_3$-$C_4$-Cycloalkyl, in welchem gegebenenfalls eine Methylengruppe durch Sauerstoff ersetzt ist.

In den als besonders bevorzugt genannten Restedefinitionen steht Halogen für Fluor, Chlor und Brom, insbesondere für Fluor und Chlor.

X steht ganz besonders bevorzugt für Methoxy oder Ethoxy,

Y steht ganz besonders bevorzugt für Methyl,

Z steht ganz besonders bevorzugt für Ethyl,

A steht ganz besonders bevorzugt für Wasserstoff, Methyl, Ethyl, n-Propyl, iso-Propyl, n-Butyl, iso-Butyl, sek.-Butyl, tert.-Butyl, Trifluormethyl, Cyclopropyl, Cyclopentyl oder Cyclohexyl,

B steht ganz besonders bevorzugt für Wasserstoff, Methyl oder Ethyl, oder

A, B und das Kohlenstoffatom an das sie gebunden sind, stehen ganz besonders bevorzugt für gesättigtes $C_6$-Cycloalkyl, worin gegebenenfalls eine Methylengruppe durch Sauerstoff ersetzt ist und welches gegebenenfalls einfach durch Methyl, Trifluormethyl, Methoxy, Ethoxy, n-Propoxy, iso-Propoxy, n-Butoxy oder iso-Butoxy substituiert ist,

D steht ganz besonders bevorzugt für Wasserstoff,

D steht auch ganz besonders bevorzugt für Methyl, Ethyl, n-Propyl, iso-Propyl, n-Butyl, sek.-Butyl, iso-Butyl, Cyclopropyl, Cyclopentyl oder Cyclohexyl, mit der Maßgabe, dass dann

A nur für Wasserstoff, Methyl oder Ethyl steht,

A und D stehen gemeinsam ganz besonders bevorzugt für eine $C_3$-$C_4$-Alkandiylgruppe, worin jeweils gegebenenfalls eine Methylengruppe durch Sauerstoff oder Schwefel ersetzt ist und welche gegebenenfalls einfach bis zweifach durch Methyl oder Methoxy substituiert ist
oder A und D gemeinsam mit den Atomen, an die sie gebunden sind, für die folgende Gruppe:

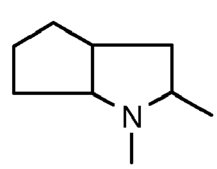

AD-1

G steht ganz besonders bevorzugt für Wasserstoff (a) oder für eine der Gruppen

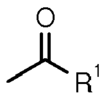
(b),
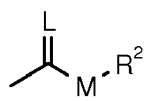
(c),
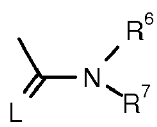
(g)

in welchen
E für ein Metallionäquivalent oder ein Ammoniumion steht,
L für Sauerstoff steht und
M für Sauerstoff oder Schwefel steht,

$R^1$ steht ganz besonders bevorzugt für jeweils gegebenenfalls einfach bis dreifach durch Fluor oder Chlor substituiertes $C_1$-$C_6$-Alkyl, $C_2$-$C_6$-Alkenyl, $C_1$-$C_2$-Alkoxy-$C_1$-$C_2$-alkyl, $C_1$-$C_2$-Alkylthio-$C_1$-$C_2$-alkyl oder Poly-$C_1$-$C_2$-alkoxy-$C_1$-$C_2$-alkyl oder für jeweils gegebenenfalls einfach durch Fluor, Chlor, Methyl, Ethyl oder Methoxy substituiertes Cyclopropyl, Cyclopentyl oder Cyclohexyl,
für gegebenenfalls einfach durch Fluor, Chlor, Brom, Cyano, Nitro, Methyl, Ethyl, n-Propyl, i-Propyl, Methoxy, Ethoxy, Methylthio, Ethylthio, Methylsulfinyl, Ethylsulfinyl, Methylsulfonyl, Ethylsulfonyl, Trifluormethyl oder Trifluormethoxy substituiertes Phenyl,
für jeweils gegebenenfalls einfach durch Chlor, Brom oder Methyl substituiertes Furanyl, Thienyl oder Pyridyl,

$R^2$ steht ganz besonders bevorzugt für $C_1$-$C_8$-Alkyl, $C_2$-$C_6$-Alkenyl oder $C_1$-$C_3$-Alkoxy-$C_2$-$C_3$-alkyl, Cyclopentyl oder Cyclohexyl,
oder für jeweils gegebenenfalls einfach durch Fluor, Chlor, Brom, Cyano, Nitro, Methyl, Methoxy, Trifluormethyl oder Trifluormethoxy substituiertes Phenyl oder Benzyl,

$R^6$ steht ganz besonders bevorzugt für Wasserstoff, für $C_1$-$C_4$-Alkyl, $C_3$-$C_6$-Cycloalkyl oder Allyl, für gegebenenfalls einfach durch Fluor, Chlor, Brom, Methyl, Methoxy oder Trifluormethyl substituiertes Phenyl,

$R^7$ steht ganz besonders bevorzugt für Methyl, Ethyl, n-Propyl, iso-Propyl oder Allyl,

$R^6$ und $R^7$ stehen gemeinsam ganz besonders bevorzugt für einen $C_5$-$C_6$-Alkylenrest, in welchem gegebenenfalls eine Methylengruppe durch Sauerstoff ersetzt ist.

X steht hevorgehoben für Methoxy oder Ethoxy,

Y steht hevorgehoben für Methyl,

Z steht hevorgehoben für Ethyl,

A steht hevorgehoben für Methyl, Ethyl oder Cyclopropyl,

B steht hevorgehoben für Wasserstoff oder Methyl, oder

A, B und das Kohlenstoffatom an das sie gebunden sind, stehen hevorgehoben für gesättigtes $C_6$-Cycloalkyl, welches gegebenenfalls einfach durch Methyl, Methoxy oder n-Butoxy substituiert ist,

D steht hevorgehoben für Wasserstoff,

D steht auch hevorgehoben für Methyl, mit der Maßgabe, dass dann

A nur für Ethyl steht,

A und D stehen gemeinsam hevorgehoben für eine $C_3$-$C_4$-Alkandiylgruppe, welche gegebenenfalls einfach durch Methoxy substituiert ist,

G steht hevorgehoben für Wasserstoff (a) oder für eine der Gruppen

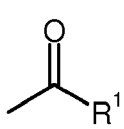 (b) oder 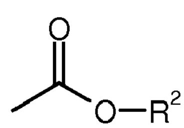 (c)

in welchen

$R^1$ hevorgehoben für $C_1$-$C_6$-Alkyl oder $C_1$-$C_2$-Alkoxy-$C_1$-$C_2$-alkyl steht,

$R^2$ hevorgehoben für $C_1$-$C_8$-Alkyl steht.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Erfindungsgemäß hervorgehoben werden die Verbindungen der Formel (I), in welchen eine Kombination der vorstehend als hervorgehoben aufgeführten Bedeutungen vorliegt.

Gesättigte oder ungesättigte Kohlenwasserstoffreste wie Alkyl, Alkandiyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Im Einzelnen seien außer den bei den Herstellungsbeispielen genannten Verbindungen die folgenden Verbindungen der Formel (I-a) genannt:

**Tabelle 1:**

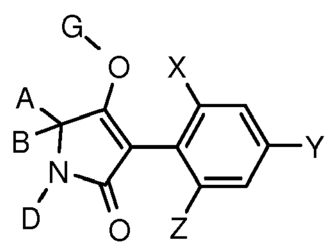

$G = H, X = OCH_3, Y = CH_3, Z = C_2H_5$

| A | B | D |
|---|---|---|
| $CH_3$ | H | H |
| $C_2H_5$ | H | H |
| $C_3H_7$ | H | H |
| i-$C_3H_7$ | H | H |
| $C_4H_9$ | H | H |
| i-$C_4H_9$ | H | H |
| s-$C_4H_9$ | H | H |
| t-$C_4H_9$ | H | H |
| $CH_3$ | $CH_3$ | H |
| $C_2H_5$ | $CH_3$ | H |
| $C_3H_7$ | $CH_3$ | H |
| i-$C_3H_7$ | $CH_3$ | H |

(fortgesetzt)

| A | B | D |
|---|---|---|
| $C_4H_9$ | $CH_3$ | H |
| i-$C_4H_9$ | $CH_3$ | H |
| s-$C_4H_9$ | $CH_3$ | H |
| t-$C_4H_9$ | $CH_3$ | H |
| $C_2H_5$ | $C_2H_5$ | H |
| $C_3H_7$ | $C_3H_7$ | H |
| 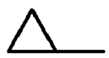 | $CH_3$ | H |
| 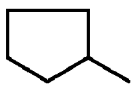 | $CH_3$ | H |
| 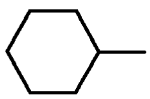 | $CH_3$ | H |
| $-(CH_2)_4-$ | | H |
| $-(CH_2)_5-$ | | H |
| $-(CH_2)_6-$ | | H |
| $-(CH_2)_7-$ | | H |
| $-(CH_2)_2-O-(CH_2)_2-$ | | H |
| $-CH_2-O-(CH_2)_3-$ | | H |
| $-(CH_2)_2-S-(CH_2)_2-$ | | H |
| $-CH_2-CHCH_3-(CH_2)_3-$ | | H |
| $-(CH_2)_2-CHCH_3-(CH_2)_2-$ | | H |
| $-(CH_2)_2-CHC_2H_5-(CH_2)_2-$ | | H |
| $-(CH_2)_2-CHC_3H_7-(CH_2)_2-$ | | H |
| $-(CH_2)_2-CHi-C_3H_7-(CH_2)_2-$ | | H |
| $-(CH_2)_2-CHOCH_3-(CH_2)_2-$ | | H |
| $-(CH_2)_2-CHOC_2H_5-(CH_2)_2-$ | | H |
| $-(CH_2)_2-CHOC_3H_7-(CH_2)_2-$ | | H |
| $-(CH_2)_2-CHO-i-C_3H_7-(CH_2)_2-$ | | H |
| $-(CH_2)_2-C(CH_3)_2-(CH_2)_2-$ | | H |
| $-CH_2-(CHCH_3)_2-(CH_2)_2-$ | | H |
| 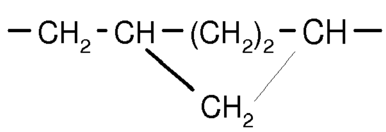 | | H |

(fortgesetzt)

| A | B | D |
|---|---|---|
| 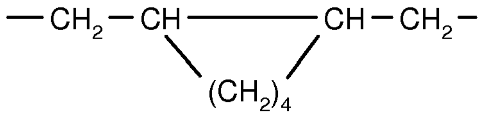 | | H |
| 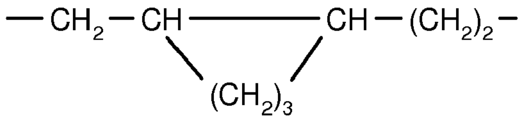 | | H |
| 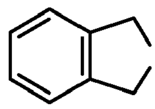 | | H |
| 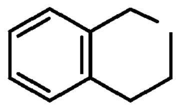 | | H |
| | | |

| A | D | B |
|---|---|---|
| $-(CH_2)_3-$ | | H |
| $-(CH_2)_4-$ | | H |
| $-CH_2-CHCH_3-CH_2-$ | | H |
| $-CH_2-CH_2-CHCH_3-$ | | H |
| $-CH_2-CHCH_3-CHCH_3-$ | | H |
| $-CH_2-CH(OCH_3)-CH_2-$ | | H |
| $-CH_2-CH=CH-CH_2-$ | | H |
| 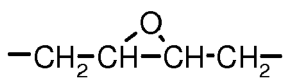 | | H |
| $-CH_2-S-CH_2-$ | | H |
| $-CH_2-S-(CH_2)_2-$ | | H |
| $-(CH_2)_2-S-CH_2-$ | | H |
| 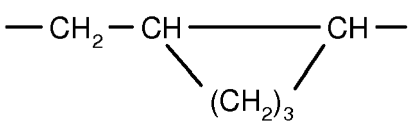 | | H |
| H | $CH_3$ | H |
| H | $C_2H_5$ | H |
| H | $C_3H_7$ | H |
| H | $i-C_3H_7$ | H |

(fortgesetzt)

| A | D | B |
|---|---|---|
| H | 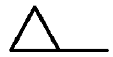 | H |
| H | 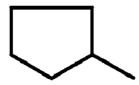 | H |
| H | 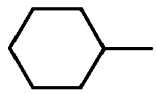 | H |
| $CH_3$ | $CH_3$ | H |
| $CH_3$ | $C_2H_5$ | H |
| $CH_3$ | $C_3H_7$ | H |
| $CH_3$ | $i\text{-}C_3H_7$ | H |
| $CH_3$ | 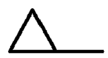 | H |
| $CH_3$ | 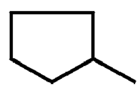 | H |
| $CH_3$ | 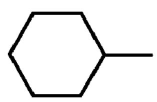 | H |
| 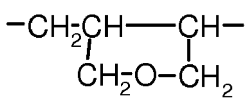 | | H |
| $C_2H_5$ | $CH_3$ | H |
| $C_2H_5$ | $C_2H_5$ | H |

A, B, D, X, Y und Z wie in Tabelle 1 angegeben

| | |
|---|---|
| Tabelle 2 | G = $CH_3$-CO |
| Tabelle 3 | G = $C_2H_5$-CO |
| Tabelle 4 | G = $C_3H_7$-CO |
| Tabelle 5 | G = i-$C_3H_7$-CO |
| Tabelle 6 | G = $C_4H_9$-CO |
| Tabelle 7 | G = i-$C_4H_9$-CO |
| Tabelle 8 | G = s-$C_4H_9$-CO |
| Tabelle 9 | G = t-$C_4H_9$-CO |
| Tabelle 10 | G = 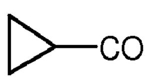 |
| Tabelle 11 | G = $H_3C$-O-$CH_2$-CO |
| Tabelle 12 | G = $H_5C_2$-O-$CH_2$-CO |
| Tabelle 13 | G = $H_3C$-S-$CH_2$-CO |

| | |
|---|---|
| Tabelle 14 | G = $H_5C_2$-S-$CH_2$-CO |
| Tabelle 15 | G = $CH_3$-O-CO |
| Tabelle 16 | G = $C_2H_5$-O-CO |
| Tabelle 17 | G = $C_3H_7$-O-CO |
| Tabelle 18 | G = i-$C_3H_7$-O-CO |
| Tabelle 19 | G = $C_4H_9$-O-CO |
| Tabelle 20 | G = i-$C_4H_9$-O-CO |
| Tabelle 21 | G = s-$C_4H_9$-O-CO |
| Tabelle 22 | G = t-$C_4H_9$-O-CO |
| Tabelle 23 | G = t-$C_4H_9$-$CH_2$-O-CO |
| Tabelle 24 | G = $C_6H_5$-$CH_2$-O-CO |
| Tabelle 25 | G = $C_6H_5$-O-CO |
| Tabelle 26 | G = $CH_3$-S-CO |
| Tabelle 27 | G = $C_2H_5$-S-CO |
| Tabelle 28 | G = $C_3H_7$-S-CO |
| Tabelle 29 | G = i-$C_3H_7$-S-CO |
| Tabelle 30 | G = $C_4H_9$-S-CO |
| Tabelle 31 | G = i-$C_4H_9$-S-CO |
| Tabelle 32 | G = s-$C_4H_9$-S-CO |
| Tabelle 33 | G = t-$C_4H_9$-S-CO |
| Tabelle 34 | G = t-$C_4H_9$-$CH_2$-S-CO |
| Tabelle 35 | G = $C_6H_5$-$CH_2$-S-CO |
| Tabelle 36 | A, B und D wie in Tabelle 1 angegeben und G = H, X = $OC_2H_5$; Y = $CH_3$; Z = $C_2H_5$. |

A, B und D wie in Tabelle 1 und X, Y und Z wie in Tabelle 36 angegeben

| | |
|---|---|
| Tabelle 37 | G = $CH_3$-CO |
| Tabelle 38 | G = $C_2H_5$-CO |
| Tabelle 39 | G = $C_3H_7$-CO |
| Tabelle 40 | G = i-$C_3H_7$-CO |
| Tabelle 41 | G = $C_4H_9$-CO |
| Tabelle 42 | G = i-$C_4H_9$-CO |
| Tabelle 43 | G = s-$C_4H_9$-CO |
| Tabelle 44 | G = t-$C_4H_9$-CO |
| Tabelle 45 | G = 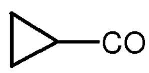 |
| Tabelle 46 | G = $H_3C$-O-$CH_2$-CO |
| Tabelle 47 | G = $H_5C_2$-O-$CH_2$-CO |
| Tabelle 48 | G = $H_3C$-S-$CH_2$-CO |
| Tabelle 49 | G = $H_5C_2$-S-$CH_2$-CO |
| Tabelle 50 | G = $CH_3$-O-CO |
| Tabelle 51 | G = $C_2H_5$-O-CO |
| Tabelle 52 | G = $C_3H_7$-O-CO |
| Tabelle 53 | G = i-$C_3H_7$-O-CO |
| Tabelle 54 | G = $C_4H_9$-O-CO |
| Tabelle 55 | G = i-$C_4H_9$-O-CO |
| Tabelle 56 | G = s-$C_4H_9$-O-CO |
| Tabelle 57 | G = t-$C_4H_9$-O-CO |
| Tabelle 58 | G = t-$C_4H_9$-$CH_2$-O-CO |
| Tabelle 59 | G = $C_6H_5$-$CH_2$-O-CO |
| Tabelle 60 | G = $C_6H_5$-O-CO |
| Tabelle 61 | G = $CH_3$-S-CO |
| Tabelle 62 | G = $C_2H_5$-S-CO |
| Tabelle 63 | G = $C_3H_7$-S-CO |
| Tabelle 64 | G = i-$C_3H_7$-S-CO |

| | |
|---|---|
| Tabelle 65 | G = $C_4H_9$-S-CO |
| Tabelle 66 | G = i-$C_4H_9$-S-CO |
| Tabelle 67 | G = s-$C_4H_9$-S-CO |
| Tabelle 68 | G = t-$C_4H_9$-S-CO |
| Tabelle 69 | G = t-$C_4H_9$-$CH_2$-S-CO |
| Tabelle 70 | G = $C_6H_5$-$CH_2$-S-CO |

Bevorzugte Bedeutungen der oben in Zusammenhang mit den die Kulturpflanzen-Verträglichkeit verbessernden Verbindungen ("Herbizid-Safenern") der Formeln (IIa), (IIb), (IIc), (IId) und (IIe) aufgeführten Gruppen werden im Folgenden definiert.

m steht bevorzugt für die Zahlen 0, 1, 2, 3 oder 4.

$A^1$ steht bevorzugt für eine der nachstehend skizzierten divalenten heterocyclischen Gruppierungen

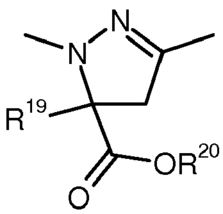 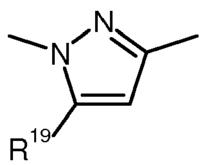 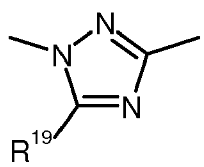 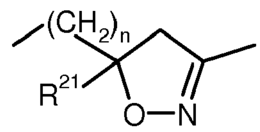 .

n steht bevorzugt für die Zahlen 0, 1, 2, 3 oder 4.

$A^2$ steht bevorzugt für jeweils gegebenenfalls durch Methyl, Ethyl, Methoxycarbonyl, Ethoxy-carbonyl oder Allyloxycarbonyl substituiertes Methylen oder Ethylen.

$R^{14}$ steht bevorzugt für Hydroxy, Mercapto, Amino, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino oder Diethylamino.

$R^{15}$ steht bevorzugt für Hydroxy, Mercapto, Amino, Methoxy, Ethoxy, n- oder i-Propoxy, n-, i-, s- oder t-Butoxy, 1-Methylhexyloxy, Allyloxy, 1-Allyloxymethyl-ethoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino oder Diethylamino.

$R^{16}$ steht bevorzugt für jeweils gegebenenfalls durch Fluor, Chlor und/oder Brom substituiertes Methyl, Ethyl, n- oder i-Propyl.

$R^{17}$ steht bevorzugt für Wasserstoff, jeweils gegebenenfalls durch Fluor und/oder Chlor substituiertes Methyl, Ethyl, n- oder i-Propyl, n-, i-, s- oder t-Butyl, Propenyl, Butenyl, Propinyl oder Butinyl, Methoxymethyl, Ethoxymethyl, Methoxyethyl, Ethoxyethyl, Dioxolanylmethyl, Furyl, Furylmethyl, Thienyl, Thiazolyl, Piperidinyl, oder gegebenenfalls durch Fluor, Chlor, Methyl, Ethyl, n- oder i-Propyl, n-, i-, s- oder t-Butyl substituiertes Phenyl.

$R^{18}$ steht bevorzugt für Wasserstoff, jeweils gegebenenfalls durch Fluor und/oder Chlor substituiertes Methyl, Ethyl, n- oder i-Propyl, n-, i-, s- oder t-Butyl, Propenyl, Butenyl, Propinyl oder Butinyl, Methoxymethyl, Ethoxymethyl, Methoxyethyl, Ethoxyethyl, Dioxolanylmethyl, Furyl, Furylmethyl, Thienyl, Thiazolyl, Piperidinyl, oder gegebenenfalls durch Fluor, Chlor, Methyl, Ethyl, n- oder i-Propyl, n-, i-, s- oder t-Butyl substituiertes Phenyl, oder zusammen mit $R^{17}$ für einen der Reste $-CH_2-O-CH_2-CH_2-$ und $-CH_2-CH_2-O-CH_2-CH_2-$, die gegebenenfalls substituiert sind durch Methyl, Ethyl, Furyl, Phenyl, einen annellierten Benzolring oder durch zwei Substituenten, die gemeinsam mit dem C-Atom, an das sie gebunden sind, einen 5- oder 6-gliedrigen Carbocyclus bilden.

$R^{19}$ steht bevorzugt für Wasserstoff, Cyano, Fluor, Chlor, Brom, oder für jeweils gegebenenfalls durch Fluor, Chlor und/oder Brom substituiertes Methyl, Ethyl, n- oder i-Propyl, Cyclopropyl, Cyclobutyl, Cyclopentyl, Cyclohexyl oder Phenyl.

$R^{20}$ steht bevorzugt für Wasserstoff, gegebenenfalls durch Hydroxy, Cyano, Fluor, Chlor, Methoxy, Ethoxy, n- oder i-Propoxy substituiertes Methyl, Ethyl, n- oder i-Propyl, n-, i-, s- oder t-Butyl.

$R^{21}$ steht bevorzugt für Wasserstoff, Cyano, Fluor, Chlor, Brom, oder für jeweils gegebenenfalls durch Fluor, Chlor und/oder Brom substituiertes Methyl, Ethyl, n- oder i-Propyl, n-, i-, s- oder t-Butyl, Cyclopropyl, Cyclobutyl, Cyclopentyl, Cyclohexyl oder Phenyl.

$X^1$ steht bevorzugt für Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.

$X^2$ steht bevorzugt für Wasserstoff, Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.

$X^3$ steht bevorzugt für Wasserstoff, Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.

t steht bevorzugt für die Zahlen 0, 1, 2, 3 oder 4.

v steht bevorzugt für die Zahlen 0, 1, 2, 3 oder 4.

$R^{22}$ steht bevorzugt für Wasserstoff, Methyl, Ethyl, n- oder i-Propyl.

$R^{23}$ steht bevorzugt für Wasserstoff, Methyl, Ethyl, n- oder i-Propyl.

$R^{24}$ steht bevorzugt für Wasserstoff, jeweils gegebenenfalls durch Cyano, Fluor, Chlor, Methoxy, Ethoxy, n- oder i-Propoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, , Methylamino, Ethylamino, n- oder i-Propylamino, n-, i-, s- oder t-Butylamino, Dimethylamino oder Diethylamino, oder jeweils gegebenenfalls durch Cyano, Fluor, Chlor, Brom, Methyl, Ethyl, n- oder i-Propyl substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino oder Cyclohexylamino.

$R^{25}$ steht bevorzugt für Wasserstoff, jeweils gegebenenfalls durch Cyano, Hydroxy, Fluor, Chlor, Methoxy, Ethoxy, n- oder i-Propoxy substituiertes Methyl, Ethyl, n- oder i-Propyl, n-, i- oder s-Butyl, jeweils gegebenenfalls durch Cyano, Fluor, Chlor oder Brom substituiertes Propenyl, Butenyl, Propinyl oder Butinyl, oder jeweils gegebenenfalls durch Cyano, Fluor, Chlor, Brom, Methyl, Ethyl, n- oder i-Propyl substituiertes Cyclopropyl, Cyclobutyl, Cyclopentyl oder Cyclohexyl.

$R^{26}$ steht bevorzugt für Wasserstoff, jeweils gegebenenfalls durch Cyano, Hydroxy, Fluor, Chlor, Methoxy, Ethoxy, n- oder i-Propoxy substituiertes Methyl, Ethyl, n- oder i-Propyl, n-, i- oder s-Butyl, jeweils gegebenenfalls durch Cyano, Fluor, Chlor oder Brom substituiertes Propenyl, Butenyl, Propinyl oder Butinyl, jeweils gegebenenfalls durch Cyano, Fluor, Chlor, Brom, Methyl, Ethyl, n- oder i-Propyl substituiertes Cyclopropyl, Cyclobutyl, Cyclopentyl oder Cyclohexyl, oder gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, oder zusammen mit $R^{25}$ für jeweils gegebenenfalls durch Methyl oder Ethyl substituiertes Butan-1,4-diyl (Trimethylen), Pentan-1,5-diyl, 1-Oxa-butan-1,4-diyl oder 3-Oxa-pentan-1,5-diyl.

$X^4$ steht bevorzugt für Nitro, Cyano, Carboxy, Carbamoyl, Formyl, Sulfamoyl, Hydroxy, Amino, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy.

$X^5$ steht bevorzugt für Nitro, Cyano, Carboxy, Carbamoyl, Formyl, Sulfamoyl, Hydroxy, Amino, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy.

Beispiele für die als erfindungsgemäße Herbizid-Safener ganz besonders bevorzugten Verbindungen der Formel (IIa) sind in der nachstehenden Tabelle aufgeführt.

**Tabelle** Beispiele für die Verbindungen der Formel (IIa)

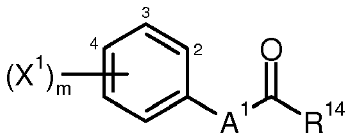 (IIa)

| **Beispiel-Nr.** | **(Positionen) $(X^1)_m$** | **$A^1$** | **$R^{14}$** |
|---|---|---|---|
| IIa-1 | (2) Cl, (4) Cl | 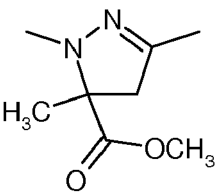 | $OCH_3$ |
| IIa-2 | (2) Cl, (4) Cl | 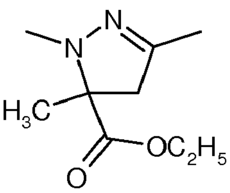 | $OCH_3$ |
| IIa-3 | (2) Cl, (4) Cl | 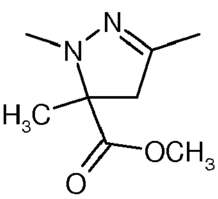 | $OC_2H_5$ |
| IIa-4 | (2) Cl, (4) Cl | 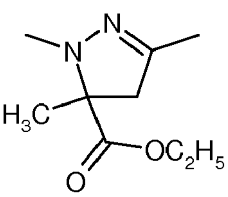 | $OC_2H_5$ |
| IIa-5 | (2) Cl | 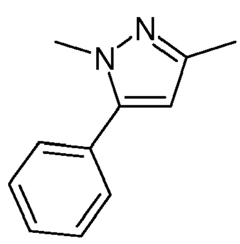 | $OCH_3$ |

(fortgesetzt)

| Beispiel-Nr. | (Positionen) $(X^1)_m$ | $A^1$ | $R^{14}$ |
|---|---|---|---|
| IIa-6 | (2) Cl, (4) Cl | 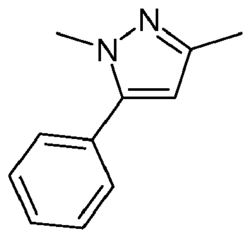 | $OCH_3$ |
| IIa-7 | (2) F | 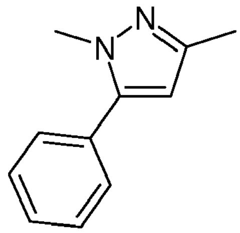 | $OCH_3$ |
| IIa-8 | (2) F | 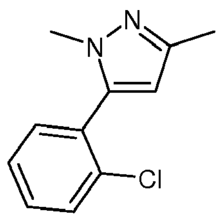 | $OCH_3$ |
| IIa-9 | (2) Cl, (4) Cl | 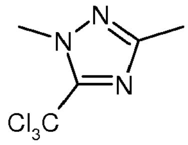 | $OC_2H_5$ |
| IIa-10 | (2) Cl, (4) $CF_3$ | 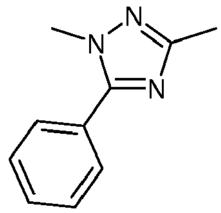 | $OCH_3$ |
| IIa-11 | (2) Cl | 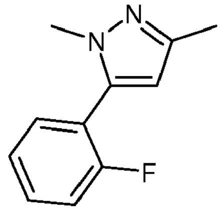 | $OCH_3$ |
| IIa-12 | - | 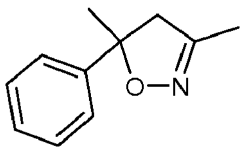 | $OC_2H_5$ |

(fortgesetzt)

| Beispiel-Nr. | (Positionen) $(X^1)_m$ | $A^1$ | $R^{14}$ |
|---|---|---|---|
| IIa-13 | (2) Cl, (4) Cl | 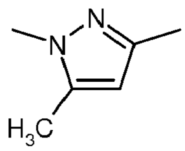 | $OC_2H_5$ |
| IIa-14 | (2) Cl, (4) Cl | 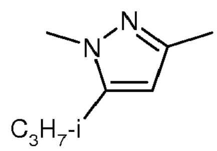 | $OC_2H_5$ |
| IIa-15 | (2) Cl, (4) Cl | 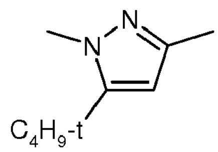 | $OC_2H_5$ |
| IIa-16 | (2) Cl, (4) Cl | 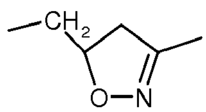 | $OC_2H_5$ |
| IIa-17 | (2) Cl, (4) Cl | 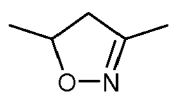 | $OC_2H_5$ |
| IIa-18 | - | 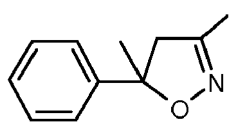 | OH |

Beispiele für die als erfindungsgemäße Herbizid-Safener ganz besonders bevorzugten Verbindungen der Formel (IIb) sind in der nachstehenden Tabelle aufgeführt.

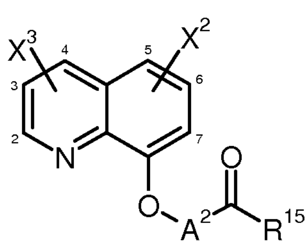

(IIb)

Tabelle Beispiele für die Verbindungen der Formel (IIb)

| Beispiel-Nr. | (Position) $X^2$ | (Position) $X^3$ | $A^2$ | $R^{15}$ |
|---|---|---|---|---|
| IIb-1 | (5) Cl | - | $CH_2$ | OH |

(fortgesetzt)

| Beispiel-Nr. | (Position) $X^2$ | (Position) $X^3$ | $A^2$ | $R^{15}$ |
|---|---|---|---|---|
| IIb-2 | (5) Cl | - | $CH_2$ | $OCH_3$ |
| IIb-3 | (5) Cl | - | $CH_2$ | $OC_2H_5$ |
| IIb-4 | (5) Cl | - | $CH_2$ | $OC_3H_7$-n |
| IIb-5 | (5) Cl | - | $CH_2$ | $OC_3H_7$-i |
| IIb-6 | (5) Cl | - | $CH_2$ | $OC_4H_9$-n |
| IIb-7 | (5) Cl | - | $CH_2$ | $OCH(CH_3)C_5H_{11}$-n |
| IIb-8 | (5) Cl | (2) F | $CH_2$ | OH |
| IIb-9 | (5) Cl | (2) Cl | $CH_2$ | OH |
| IIb-10 | (5) Cl | - | $CH_2$ | $OCH_2CH=CH_2$ |
| IIb-11 | (5) Cl | - | $CH_2$ | $OC_4H_9$-i |
| IIb-12 | (5) Cl | - | $CH_2$ | 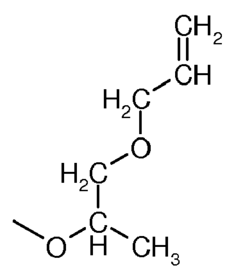 |
| IIb-13 | (5) Cl | - | 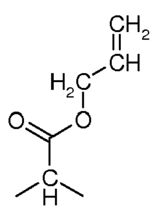 | $OCH_2CH=CH_2$ |
| IIb-14 | (5) Cl | - | 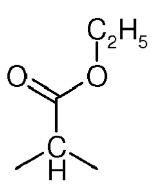 | $OC_2H_5$ |

(fortgesetzt)

| **Beispiel-Nr.** | **(Position) $X^2$** | **(Position) $X^3$** | **$A^2$** | **$R^{15}$** |
|---|---|---|---|---|
| IIb-15 | (5) Cl | - | 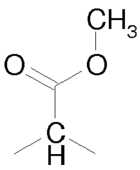 | $OCH_3$ |

Beispiele für die als erfindungsgemäße Herbizid-Safener ganz besonders bevorzugten Verbindungen der Formel (IIc) sind in der nachstehenden Tabelle aufgeführt.

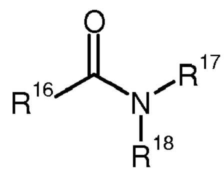

(IIc)

__Tabelle__ Beispiele für die Verbindungen der Formel (IIc)

| **Beispiel-Nr.** | **$R^{16}$** | **$N(R^{17},R^{18})$** |
|---|---|---|
| IIc-1 | $CHCl_2$ | $N(CH_2CH=CH_2)_2$ |
| IIc-2 | $CHCl_2$ | 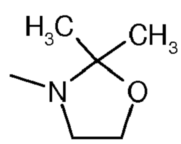 |
| IIc-3 | $CHCl_2$ | 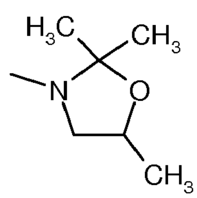 |
| IIc-4 | $CHCl_2$ | 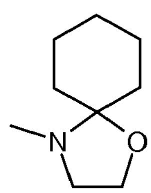 |
| IIc-5 | $CHCl_2$ | 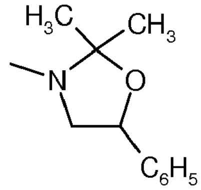 |

(fortgesetzt)

| Beispiel-Nr. | $R^{16}$ | $N(R^{17},R^{18})$ |
|---|---|---|
| IIc-6 | $CHCl_2$ | 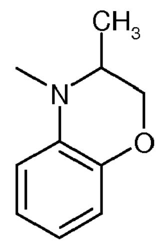 |
| IIc-7 | $CHCl_2$ | 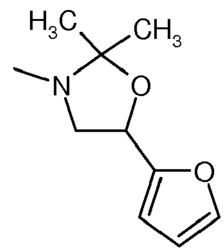 |

Beispiele für die als erfindungsgemäße Herbizid-Safener ganz besonders bevorzugten Verbindungen der Formel (IId) sind in der nachstehenden Tabelle aufgeführt.

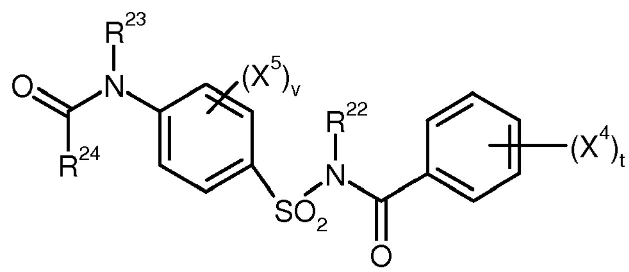

(IId)

**Tabelle** Beispiele für die Verbindungen der Formel (IId)

| Beispiel-Nr. | $R^{22}$ | $R^{23}$ | $R^{24}$ | (Positionen) $(X^4)t$ | (Positionen) $(X^5)v$ |
|---|---|---|---|---|---|
| IId-1 | H | H | $CH_3$ | (2) $OCH_3$ | - |
| IId-2 | H | H | $C_2H_5$ | (2) $OCH_3$ | - |
| IId-3 | H | H | $C_3H_7$-n | (2) $OCH_3$ | - |
| IId-4 | H | H | $C_3H_7$-i | (2) $OCH_3$ | - |
| IId-5 | H | H | 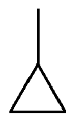 | (2) $OCH_3$ | - |
| IId-6 | H | H | $CH_3$ | (2) $OCH_3$<br>(5) $CH_3$ | - |
| IId-7 | H | H | $C_2H_5$ | (2) $OCH_3$<br>(5) $CH_3$ | - |

(fortgesetzt)

| Beispiel-Nr. | $R^{22}$ | $R^{23}$ | $R^{24}$ | (Positionen) $(X^4)t$ | (Positionen) $(X^5)v$ |
|---|---|---|---|---|---|
| IId-8 | H | H | $C_3H_7$-n | (2) $OCH_3$<br>(5) $CH_3$ | - |
| IId-9 | H | H | $C_3H_7$-i | (2) $OCH_3$<br>(5) $CH_3$ | - |
| IId-10 | H | H | 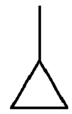 | (2) $OCH_3$<br>(5) $CH_3$ | - |
| IId-11 | H | H | $OCH_3$ | (2) $OCH_3$<br>(5) $CH_3$ | - |
| IId-12 | H | H | $OC_2H_5$ | (2) $OCH_3$<br>(5) $CH_3$ | - |
| IId-13 | H | H | $OC_3H_7$-i | (2) $OCH_3$<br>(5) $CH_3$ | - |
| IId-14 | H | H | $SCH_3$ | (2) $OCH_3$<br>(5) $CH_3$ | - |
| IId-15 | H | H | $SC_2H_5$ | (2) $OCH_3$<br>(5) $CH_3$ | - |
| IId-16 | H | H | $SC_3H_7$-i | (2) $OCH_3$<br>(5) $CH_3$ | - |
| IId-17 | H | H | $NHCH_3$ | (2) $OCH_3$<br>(5) $CH_3$ | - |
| IId-18 | H | H | $NHC_2H_5$ | (2) $OCH_3$<br>(5) $CH_3$ | - |
| IId-19 | H | H | $NHC_3H_7$-i | (2) $OCH_3$<br>(5) $CH_3$ | - |
| IId-20 | H | H | 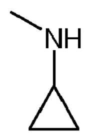 | (2) $OCH_3$<br>(5) $CH_3$ | - |
| IId-21 | H | H | $NHCH_3$ | (2) $OCH_3$ | - |
| IId-22 | H | H | $NHC_3H_7$-i | (2) $OCH_3$ | - |
| IId-23 | H | H | $N(CH_3)_2$ | (2) $OCH_3$ | - |
| IId-24 | H | H | $N(CH_3)_2$ | (3) $CH_3$<br>(4) $CH_3$ | - |
| IId-25 | H | H | $CH_2$-O-$CH_3$ | (2) $OCH_3$ | - |

Beispiele für die als erfindungsgemäße Herbizid-Safener ganz besonders bevorzugten Verbindungen der Formel (IIe) sind in der nachstehenden Tabelle aufgeführt.

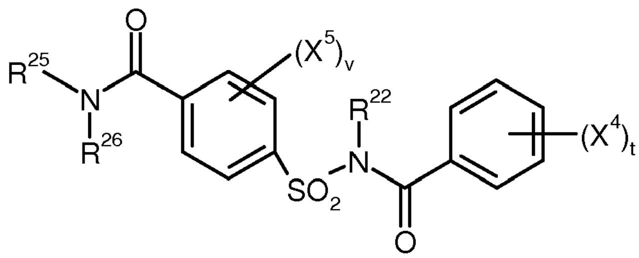

(IIe)

**Tabelle** Beispiele für die Verbindungen der Formel (IIe)

| **Beispiel-Nr.** | **$R^{22}$** | **$R^{25}$** | **$R^{26}$** | **(Positionen) $(X^4)_t$** | **(Positionen) $(X^5)_v$** |
|---|---|---|---|---|---|
| IIe-1 | H | H | $CH_3$ | (2) $OCH_3$ | - |
| IIe-2 | H | H | $C_2H_5$ | (2) $OCH_3$ | - |
| IIe-3 | H | H | $C_3H_7$-n | (2) $OCH_3$ | - |
| IIe-4 | H | H | $C_3H_7$-i | (2) $OCH_3$ | - |
| IIe-5 | H | H | 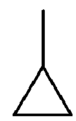 | (2) $OCH_3$ | - |
| IIe-6 | H | $CH_3$ | $CH_3$ | (2) $OCH_3$ | - |
| IIe-7 | H | H | $CH_3$ | (2) $OCH_3$<br>(5) $CH_3$ | - |
| IIe-8 | H | H | $C_2H_5$ | (2) $OCH_3$<br>(5) $CH_3$ | - |
| IIe-9 | H | H | $C_3H_7$-n | (2) $OCH_3$<br>(5) $CH_3$ | - |
| IIe-10 | H | H | $C_3H_7$-i | (2) $OCH_3$<br>(5) $CH_3$ | - |
| IIe-11 | H | H | 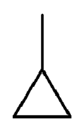 | (2) $OCH_3$<br>(5) $CH_3$ | - |
| IIe-12 | H | $CH_3$ | $CH_3$ | (2) $OCH_3$<br>(5) $CH_3$ | - |

Als die die Kulturpflanzen-Verträglichkeit verbessernde Verbindung [Komponente (b')] sind Cloquintocet-mexyl, Fenchlorazol-ethyl, Isoxadifen-ethyl, Mefenpyr-diethyl, Furilazole, Fenclorim, Cumyluron, Dymron, Dimepiperate und die Verbindungen IIe-5 und IIe-11 am meisten bevorzugt, wobei Cloquintocet-mexyl und Mefenpyr-diethyl besonders hervorgehoben seien.

Die als Safener erfindungsgemäß zu verwendenden Verbindungen der allgemeinen Formel (IIa) sind bekannt und/oder können nach an sich bekannten Verfahren hergestellt werden (vgl. WO-A-91/07874, WO-A-95/07897).

Die als Safener erfindungsgemäß zu verwendenden Verbindungen der allgemeinen Formel (IIb) sind bekannt und/oder können nach an sich bekannten Verfahren hergestellt werden (vgl. EP-A-191736).

Die als Safener erfindungsgemäß zu verwendenden Verbindungen der allgemeinen Formel (IIc) sind bekannt und/oder können nach an sich bekannten Verfahren hergestellt werden (vgl. DE-A-2218097, DE-A-2350547).

Die als Safener erfindungsgemäß zu verwendenden Verbindungen der allgemeinen Formel (IId) sind bekannt

und/oder können nach an sich bekannten Verfahren hergestellt werden (vgl. DE-A-19621522/US-A-6235680).

Die als Safener erfindungsgemäß zu verwendenden Verbindungen der allgemeinen Formel (IIe) sind bekannt und können nach an sich bekannten Verfahren hergestellt werden (vgl. WO-A-99/66795/US-A-6251827).

Beispiele für die erfindungsgemäßen selektiv herbiziden Kombinationen aus jeweils einem Wirkstoff der Formel (I) und jeweils einem der oben definierten Safener sind in der nachstehenden Tabelle aufgeführt.

**Tabelle** Beispiele für die erfindungsgemäßen Kombinationen

| Wirkstoffe der Formel (I) | Safener |
|---|---|
| I-a | Cloquintocet-mexyl |
| I-a | Fenchlorazole-ethyl |
| I-a | Isoxadifen-ethyl |
| I-a | Mefenpyr-diethyl |
| I-a | Furilazole |
| I-a | Fenclorim |
| I-a | Cumyluron |
| I-a | Daimuron /Dymron |
| I-a | Dimepiperate |
| I-a | IIe-11 |
| I-a | IIe-5 |
| I-b | Cloquintocet-mexyl |
| I-b | Fenchlorazole-ethyl |
| I-b | Isoxadifen-ethyl |
| I-b | Mefenpyr-diethyl |
| I-b | Furilazole |
| I-b | Fenclorim |
| I-b | Cumyluron |
| I-b | Daimuron /Dymron |
| I-b | Dimepiperate |
| I-b | IIe-11 |
| I-b | IIe-5 |
| I-c | Cloquintocet-mexyl |
| I-c | Fenchlorazole-ethyl |
| I-c | Isoxadifen-ethyl |
| I-c | Mefenpyr-diethyl |
| I-c | Furilazole |
| I-c | Fenclorim |
| I-c | Cumyluron |
| I-c | Daimuron /Dymron |
| I-c | Dimepiperate |
| I-c | IIe-5 |
| I-c | IIe-11 |
| I-d | Cloquintocet-mexyl |

(fortgesetzt)

| Wirkstoffe der Formel (I) | Safener |
|---|---|
| I-d | Fenchlorazole-ethyl |
| I-d | Isoxadifen-ethyl |
| I-d | Mefenpyr-diethyl |
| I-d | Furilazole |
| I-d | Fenclorim |
| I-d | Cumyluron |
| I-d | Daimuron /Dymron |
| I-d | Dimepiperate |
| I-d | IIe-11 |
| I-d | IIe-5 |
| I-e | Cloquintocet-mexyl |
| I-e | Fenchlorazole-ethyl |
| I-e | Isoxadifen-ethyl |
| I-e | Mefenpyr-diethyl |
| I-e | Furilazole |
| I-e | Fenclorim |
| I-e | Cumyluron |
| I-e | Daimuron /Dymron |
| I-e | Dimepiperate |
| I-e | IIe-5 |
| I-e | IIe-11 |
| I-f | Cloquintocet-mexyl |
| I-f | Fenchlorazole-ethyl |
| I-f | Isoxadifen-ethyl |
| I-f | Mefenpyr-diethyl |
| I-f | Furilazole |
| I-f | Fenclorim |
| I-f | Cumyluron |
| I-f | Daimuron /Dymron |
| I-f | Dimepiperate |
| I-f | IIe-5 |
| I-f | IIe-11 |
| I-g | Cloquintocet-mexyl |
| I-g | Fenchlorazole-ethyl |
| I-g | Isoxadifen-ethyl |
| I-g | Mefenpyr-diethyl |
| I-g | Furilazole |
| I-g | Fenclorim |

(fortgesetzt)

| Wirkstoffe der Formel (I) | Safener |
|---|---|
| I-g | Cumyluron |
| I-g | Daimuron /Dymron |
| I-g | Dimepiperate |
| I-g | IIe-5 |
| I-g | IIe-11 |

Es wurde nun überraschend gefunden, dass die oben definierten Wirkstoffkombinationen aus substituierten Tetramsäurederivaten der allgemeinen Formel (I) und Safenern (Antidots) aus der oben aufgeführten Gruppe (b') bei sehr guter Nutzpflanzen-Verträglichkeit eine besonders hohe herbizide Wirksamkeit aufweisen und in verschiedenen Kulturen, insbesondere in Getreide (vor allem Weizen), aber auch in Soja, Kartoffeln, Mais und Reis zur selektiven Unkrautbekämpfung verwendet werden können.

Dabei ist es als überraschend anzusehen, dass aus einer Vielzahl von bekannten Safenern oder Antidots, die befähigt sind, die schädigende Wirkung eines Herbizids auf die Kulturpflanzen zu antagonisieren, gerade die oben aufgeführten Verbindungen der Gruppe (b') geeignet sind, die schädigende Wirkung von substituierten Tetramsäurederivaten auf die Kulturpflanzen annähernd vollständig aufzuheben, ohne dabei die herbizide Wirksamkeit gegenüber den Unkräutern maßgeblich zu beeinträchtigen.

Hervorgehoben sei hierbei die besonders vorteilhafte Wirkung der besonders und am meisten bevorzugten Kombinationspartner aus der Gruppe (b'), insbesondere hinsichtlich der Schonung von Getreidepflanzen, wie z.B. Weizen, Gerste und Roggen, aber auch Mais und Reis, als Kulturpflanzen.

Verwendet man beispielsweise gemäß Verfahren (A) N-[(2-Methoxy-4-methyl-6-ethyl)-phenylacetyl]-1-amino-4-ethoxy-cyclohexan-carbonsäureethylester als Ausgangsstoff, so kann der Verlauf des erfindungsgemäßen Verfahrens durch folgendes Reaktionsschema wiedergegeben werden:

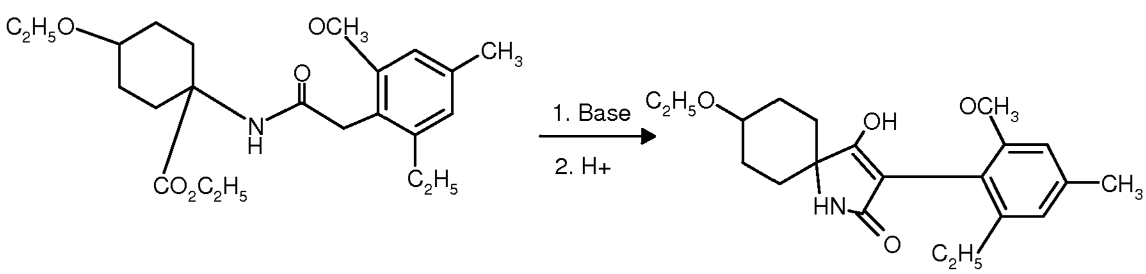


Verwendet man beispielsweise gemäß Verfahren (Bα) 3-[(2-Methoxy-4-methyl-6-ethyl)-phenyl]-1-azaspiro [4,5]dec-an-2,4-dion und Pivaloylchlorid als Ausgangsstoffe, so kann der Verlauf des erfindungsgemäßen Verfahrens durch folgendes Reaktionsschema wiedergegeben werden:

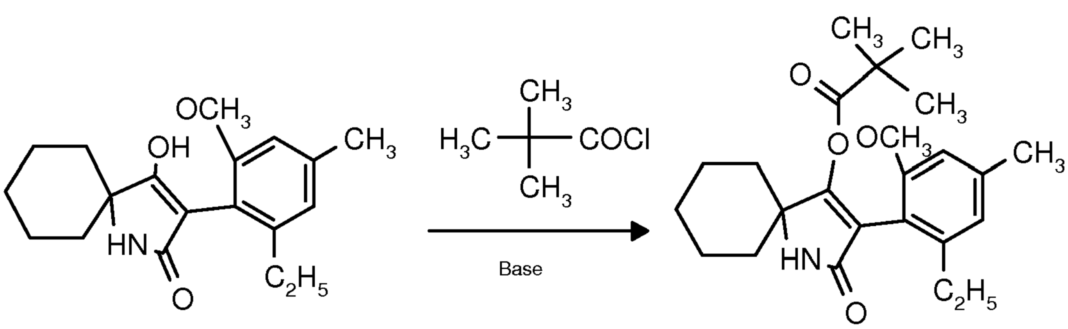


Verwendet man beispielsweise gemäß Verfahren (B) (Variante β) 8-Methoxy-3-[(2-methoxy-4-methyl-6-ethyl)-phenyl]-1-azasprio[4,5]dec-an-2,4-dion und Acetanhydrid als Ausgangsverbindungen, so kann der Verlauf des erfindungsgemäßen Verfahrens durch folgendes Reaktionsschema wiedergegeben werden:

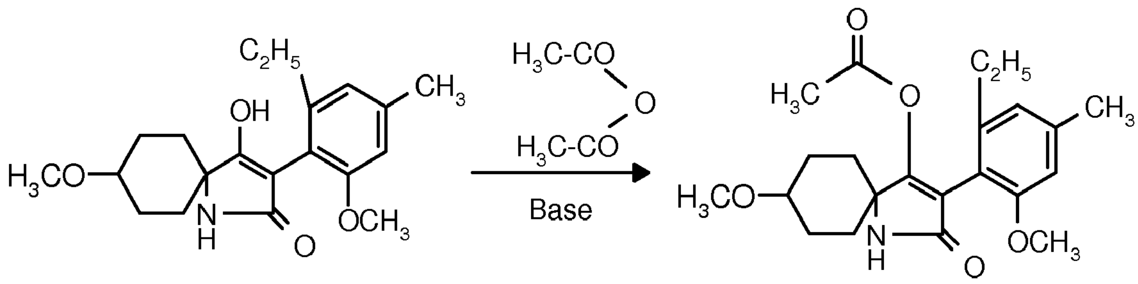


Verwendet man beispielsweise gemäß Verfahren (C) 3-[2-Methoxy-4-methyl-6-ethyl)-phenyl]-1-azaspiro[4,5]dec-3-an-2,4-dion und Chlorameisensäureethylester als Ausgangsverbindungen, so kann der Verlauf des erfindungsgemäßen Verfahrens durch folgendes Reaktionsschema wiedergegeben werden:

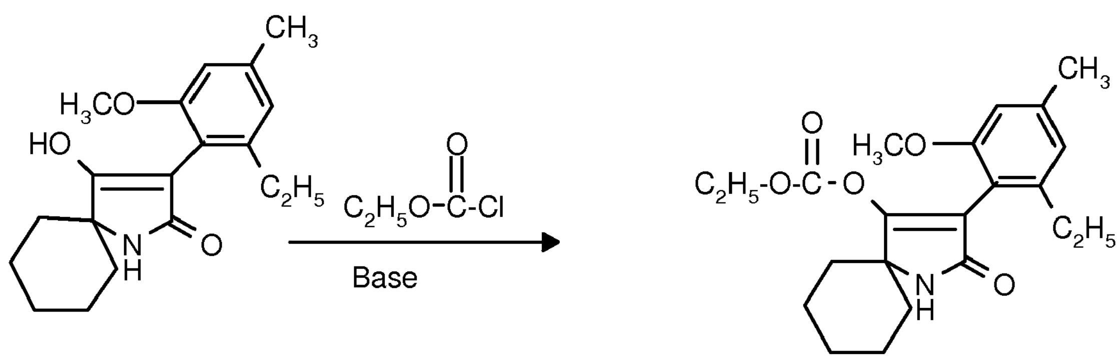


Verwendet man beispielsweise gemäß Verfahren (D), Variante α 3-[(2-Methoxy-4-methyl-6-ethyl)-phenyl]-1-azaspiro[4,5]dec-an-2,4-dion und Chlormonothioameisensäuremethylester als Ausgangsprodukte, so kann der Reaktionsverlauf folgendermaßen wiedergegeben werden:

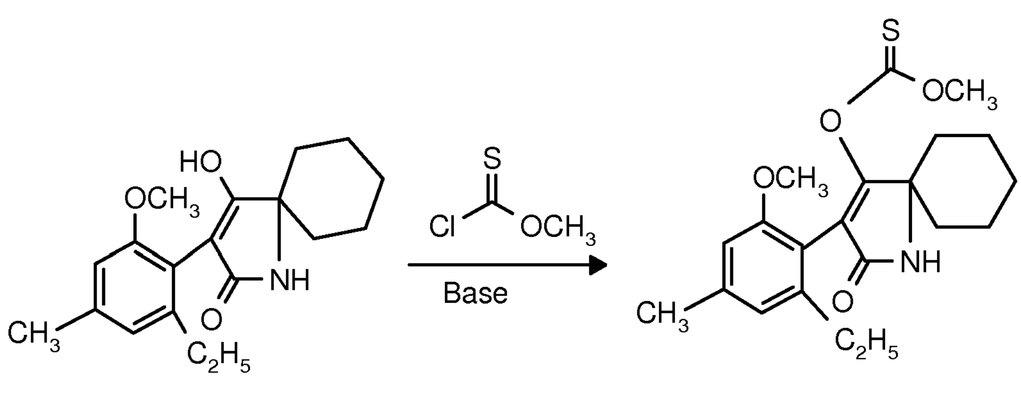


Verwendet man beispielsweise gemäß Verfahren (D), Variante β 8-Methoxy-3-[(2-methoxy-4-methyl-6-ethyl)-phenyl]-1-azaspiro[4,5]dec-an-2,4-dion, Schwefelkohlenstoff und Methyliodid als Ausgangskomponenten, so kann der Reaktionsverlauf wie folgt wiedergegeben werden:

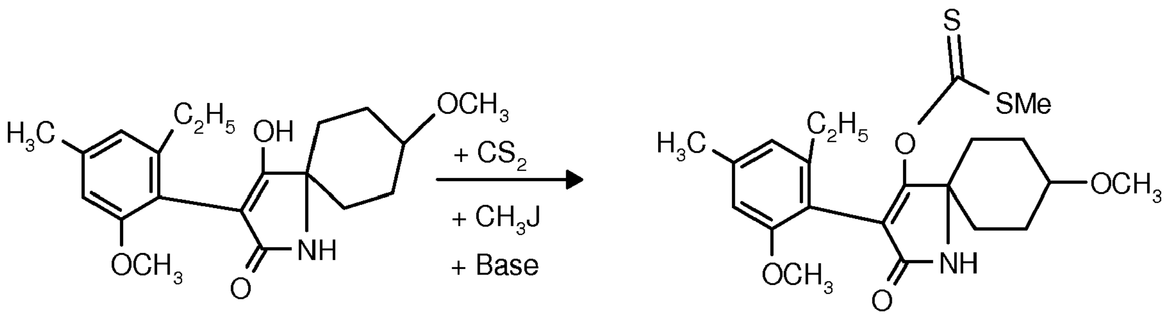


Verwendet man beispielsweise gemäß Verfahren (E) 3-[(2-Methoxy-4-methyl-6-ethyl)-phenyl]-1-azaspiro[4,5] dec-an-2,4-dion und Methansulfonsäurechlorid als Ausgangsprodukt, so kann der Reaktionsverlauf durch folgendes Reaktionsschema wiedergegeben werden:

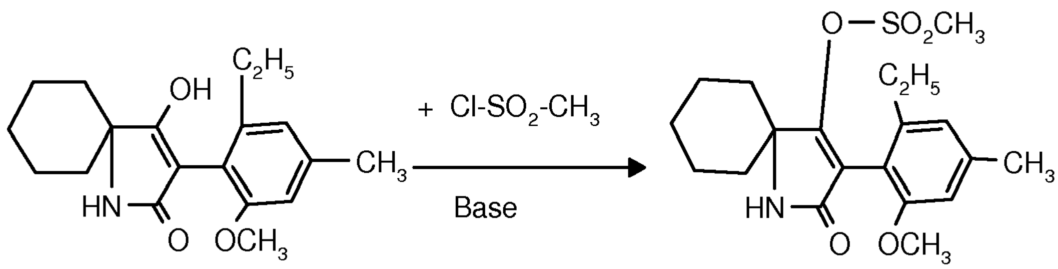


Verwendet man beispielsweise gemäß Verfahren (F) 3-[(2-Methoxy-4-methyl-6-ethyl)-phenyl]-1-azaspiro[4,5] dec-an-2,4-dion und Methanthio-phosphonsäurechlorid-(2,2,2-trifluorethylester) als Ausgangsprodukte, so kann der Reaktionsverlauf durch folgendes Reaktionsschema wiedergegeben werden:

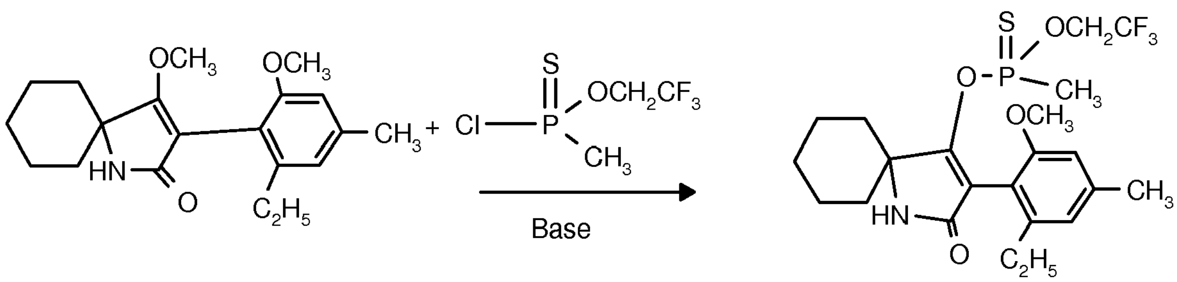


Verwendet man beispielsweise gemäß Verfahren (G) 8-Methoxy-3-[(2-methoxy-4-methyl-6-ethyl)-phenyl]-1-azaspiro[4,5]dec-an-2,4-dion und NaOH als Komponenten, so kann der Verlauf des erfindungsgemäßen Verfahrens durch folgendes Reaktionsschema wiedergegeben werden:

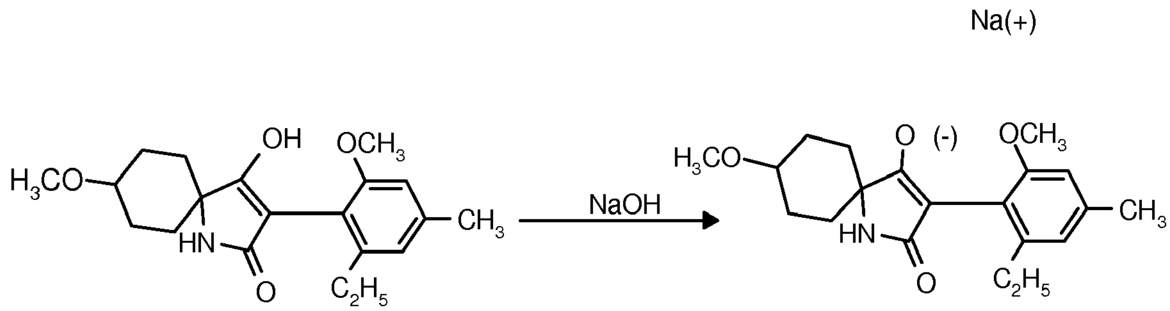


Verwendet man beispielsweise gemäß Verfahren (H) Variante α 3-[(2-Methoxy-4-methyl-6-ethyl)-phenyl]-1-azaspiro[4,5]dec-an-2,4-dion und Ethylisocyanat als Ausgangsprodukte, so kann der Reaktionsverlauf durch folgendes Reaktionsschema wiedergegeben werden:

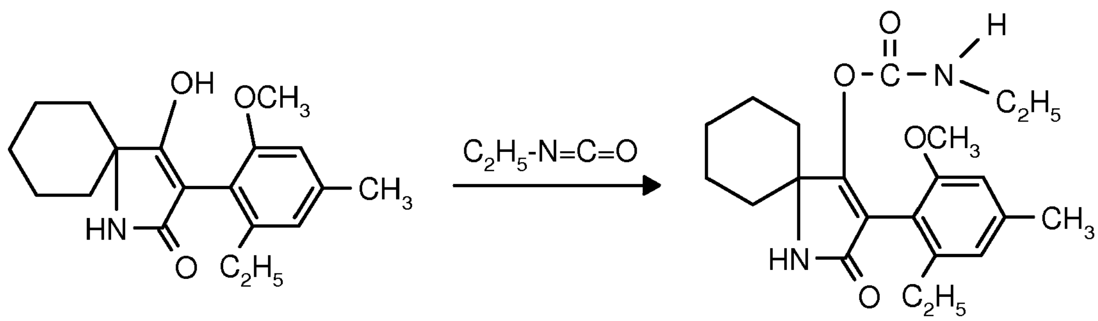


Verwendet man beispielsweise gemäß Verfahren (H) Variante β 8-Methoxy-3-[(2-methoxy-4-methyl-6-ethyl)-phenyl]-1-azaspiro[4,5]dec-an-2,4-dion und Dimethylcarbamidsäurechlorid als Ausgangsprodukte, so kann der Reaktionsverlauf durch folgendes Schema wiedergegeben werden:

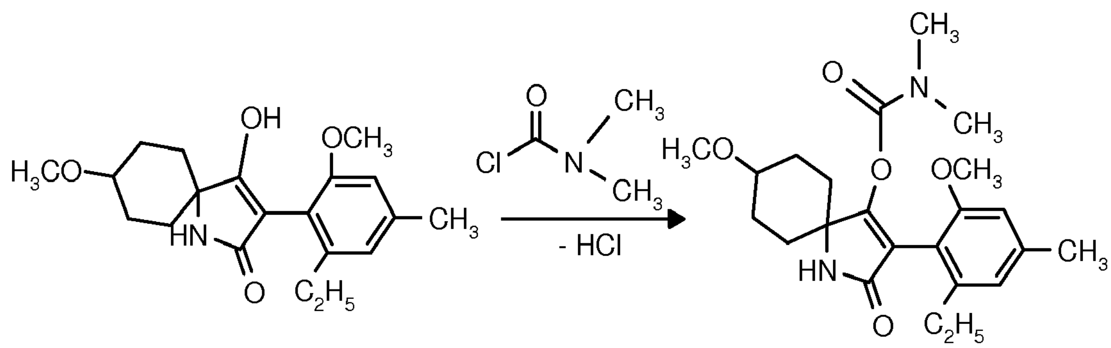


Die beim erfindungsgemäßen Verfahren (A) als Ausgangsstoffe benötigten Verbindungen der Formel (II),

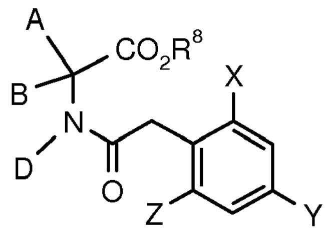

(II)

in welcher
A, B, D, X, Y, Z und $R^8$ die oben angegebene Bedeutungen haben,
sind neu.

Man erhält die Acylaminosäureester der Formel (II) beispielsweise, wenn man Aminosäurederivate der Formel (XIV),

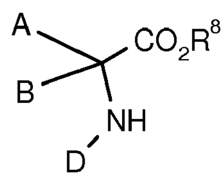

(XIV)

in welcher
A, B, D und $R^8$ die oben angegebenen Bedeutungen haben,
mit substituierten Phenylessigsäurehalogeniden der Formel (XV),

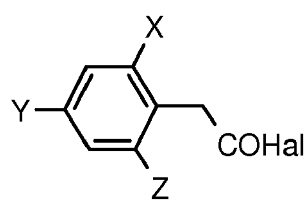

(XV)

in welcher

X, Y und Z die oben angegebenen Bedeutungen haben und

Hal für Chlor oder Brom steht,

acyliert (Chem. Reviews 52, 237-416 (1953); Bhattacharya, Indian J. Chem. 6, 341-5, 1968, eingangs zitierte Patentliteratur, z.B. WO 97/02243)
oder wenn man Acylaminosäuren der Formel (XVI),

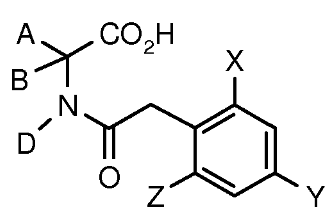

(XVI)

in welcher

A, B, D, X, Y und Z die oben angegebenen Bedeutungen haben,

verestert (Chem. Ind. (London) 1568 (1968)).

Die Verbindungen der Formel (XVI),

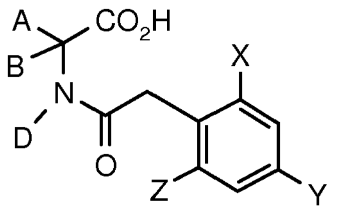

(XVI)

in welcher

A, B, D, X, Y und Z die oben angegebenen Bedeutungen haben,

sind ebenfalls neu.

Man erhält die Verbindungen der Formel (XVI), wenn man Aminosäuren der Formel (XVII),

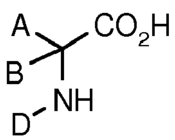

(XVII)

in welcher

A, B und D die oben angegebenen Bedeutungen haben,

mit substituierten Phenylessigsäurehalogeniden der Formel (XV),

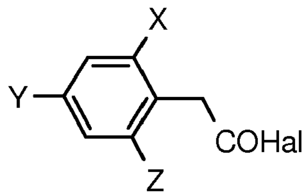

(XV)

in welcher

X, Y und Z die oben angegebenen Bedeutungen haben und

Hal für Chlor oder Brom steht,

nach Schotten-Baumann acyliert (Organikum, VEB Deutscher Verlag der Wissenschaften, Berlin 1977, S. 505).

Die Verbindungen der Formel (XV) sind teilweise neu und lassen sich nach im Prinzip bekannten Verfahren herstellen (WO 97/02243).

Man erhält die Verbindungen der Formel (XV) beispielsweise, indem man substituierte Phenylessigsäuren der Formel (XVIII),

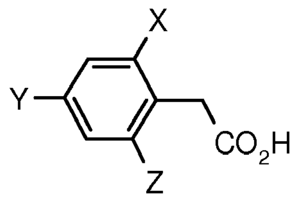

(XVIII)

in welcher

X, Y und Z die oben angegebene Bedeutung haben,

mit Halogenierungsmitteln (z.B. Thionylchlorid, Thionylbromid, Oxalylchlorid, Phosgen, Phosphortrichlorid, Phosphortribromid oder Phosphorpentachlorid) gegebenenfalls in Gegenwart eines Verdünnungsmittels (z.B. gegebenenfalls chlorierten aliphatischen oder aromatischen Kohlenwasserstoffen wie Toluol oder Methylenchlorid) bei Temperaturen von -20˚C bis 150˚C, bevorzugt von -10˚C bis 100˚C, umsetzt.

Die Verbindungen der Formel (XVIII) sind neu.

Man erhält die Verbindungen der Formel (XVIII) beispielsweise, indem man substituierte Phenylessigsäureester der Formel (XIX),

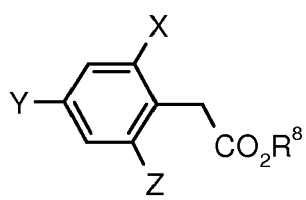

(XIX)

in welcher

X, Y, Z und $R^8$ die oben angegebene Bedeutung haben,

in Gegenwart einer Säure (z.B. einer anorganischen Säure wie Chlorwasserstoffsäure) oder einer Base (z.B. eines Alkalihydroxids wie Natrium- oder Kaliumhydroxid) und gegebenenfalls eines Verdünnungsmittels (z.B. eines wässrigen Alkohols wie Methanol oder Ethanol) bei Temperaturen zwischen 0˚C und 150˚C, bevorzugt zwischen 20˚C und 100˚C, hydrolysiert.

Die Verbindungen der Formel (XIX) sind ebenfalls neu und lassen sich nach im Prinzip bekannten Verfahren herstellen (WO 04/080962).

Die Verbindungen der Formel (XIX)

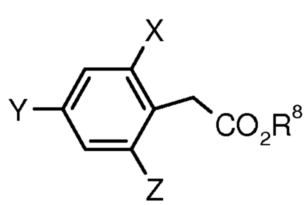

(XIX)

in welcher
X, Y, Z und $R^8$ die oben angegebene Bedeutung haben,
erhält man beispielsweise, wenn man Phenylessigsäureester der Formel (XIX-a)

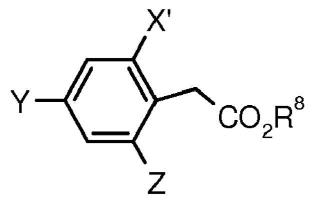

(XIX-a)

in welcher

| | |
|---|---|
| $R^8$, Y und Z | die oben angegebene Bedeutung haben, und |
| X' | für Halogen (insbesondere für Brom) steht, |

in Gegenwart eines Alkohols, in Gegenwart einer Base und gegebenenfalls in Gegenwart eines Katalysators (bevorzugt Kupfersalze wie z.B. Kupfer(I)bromid) umsetzt.

Die Verbindungen der Formel (XIX-a) sind teilweise bekannt und lassen sich nach im Prinzip bekannten Verfahren herstellen (WO 05/044796).

Die Verbindungen der Formel (XIV) und (XVII) sind teilweise bekannt und/oder lassen sich nach bekannten Verfahren darstellen (siehe z.B. Compagnon, Miocque Ann. Chim. (Paris) [14] 5, S. 11-22, 23-27 (1970)).

Die substituierten cyclischen Aminocarbonsäuren der Formel (XVII), in der A und B einen Ring bilden, sind im Allgemeinen nach der Bucherer-Bergs-Synthese oder nach der Strecker-Synthese erhältlich und fallen dabei jeweils in unterschiedlichen Isomerenformen an. So erhält man nach den Bedingungen der Bucherer-Bergs-Synthese vorwiegend die Isomeren (im folgenden der Einfachheit halber als β bezeichnet), in welchen die Reste R und die Carboxylgruppe äquatorial stehen, während nach den Bedingungen der Strecker-Synthese vorwiegend die Isomeren (im folgenden der Einfachheit halber als α bezeichnet) anfallen, bei denen die Aminogruppe und die Reste R äquatorial stehen.

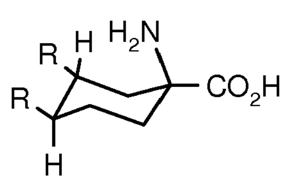

Bucherer-Bergs-Synthese

(β-Isomeres)

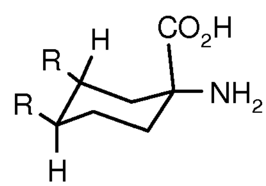

Strecker-Synthese

(α-Isomeres)

Die 3-Alkoxy-1-amino-cyclohexan-carbonsäuren (XVII) in der A und B einen Ring bilden sind bekannt aus DE-A-04 030 753 und sind im Allgemeinen nach der Bucherer-Bergs-Synthese oder nach der Strecker-Synthese erhältlich und fallen dabei jeweils in unterschiedlichen Isomerenformen an. Im Folgenden werden der Einfachheit halber die Isomeren als β bezeichnet, in welchem der 3-Substituent (R) und die Aminogruppe äquatorial/axial oder axial/äquatorial stehen. Im Folgenden wurden der Einfachheit halber die Isomeren als α bezeichnet, in welchen die Aminogruppe und der 3-Substituent (R) äquatorial/äquatorial oder axial/axial stehen.

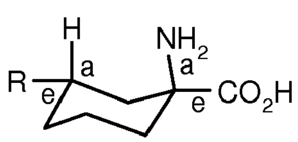

Beispiel: ß-Isomeres

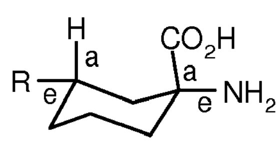

Beispiel: α-Isomeres

(L. Munday, J. Chem. Soc. 4372 (1961); J.T. Eward, C. Jitrangeri, Can. J. Chem. 53, 3339 (1975).

Weiterhin lassen sich die bei dem obigen Verfahren (A) verwendeten Ausgangsstoffe der Formel (II),

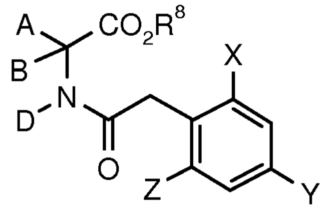

(II)

in welcher

A, B, D, X, Y, Z und $R^8$ die oben angegebenen Bedeutungen haben,

herstellen, wenn man Aminonitrile der Formel (XXIII),

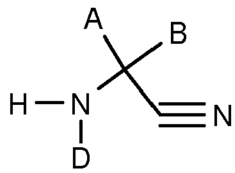

(XXIII)

in welcher

A, B und D die oben angegebenen Bedeutungen haben,

mit substituierten Phenylessigsäurehalogeniden der Formel (XV),

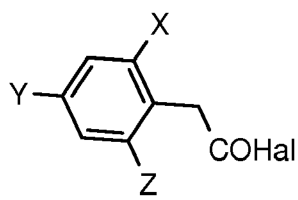

(XV)

in welcher

X, Y, Z und Hal die oben angegebenen Bedeutungen haben,

zu Verbindungen der Formel (XXIV),

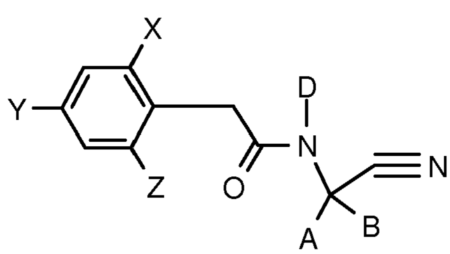

(XXIV)

in welcher

A, B, D, X, Y und Z die oben angegebenen Bedeutungen haben,

umsetzt,
und diese anschließend einer sauren Alkoholyse unterwirft.

Die Verbindungen der Formel (XXIV) sind ebenfalls neu.

Die zur Durchführung der erfindungsgemäßen Verfahren (B), (C), (D), (E), (F), (G) und (H) außerdem als Ausgangsstoffe benötigten Säurehalogenide der Formel (III), Carbonsäureanhydride der Formel (IV), Chlorameisensäureester oder Chlorameisensäurethioester der Formel (V), Chlormonothioameisensäureester oder Chlordithioameisensäureester der Formel (VI), Alkylhalogenide der Formel (VII), Sulfonsäurechloride der Formel (VIII), Phosphorverbindungen der Formel (IX) und Metallhydroxide, Metallalkoxide oder Amine der Formel (X) und (XI) und Isocyanate der Formel (XII) und Carbamidsäurechloride der Formel (XIII) sind allgemein bekannte Verbindungen der organischen bzw. anorganischen Chemie.

Die Verbindungen der Formeln (XIV) und (XVII) sind darüber hinaus aus den eingangs zitierten Patentanmeldungen bekannt und/oder lassen sich nach den dort angegebenen Methoden herstellen.

Das Verfahren (A) ist **dadurch gekennzeichnet, dass** man Verbindungen der Formel (II), in welcher A, B, D, X, Y, Z und $R^8$ die oben angegebenen Bedeutungen haben, in Gegenwart eines Verdünnungsmittels und in Gegenwart einer Base einer intramolekularen Kondensation unterwirft.

Als Verdünnungsmittel können bei dem erfindungsgemäßen Verfahren (A) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl($C_8$-$C_{10}$)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natriumethylat und Kalium-tert.-butylat einsetzbar.

Die Reaktionstemperatur kann bei der Durchführung des erfindungsgemäßen Verfahrens (A) innerhalb eines größeren Bereiches variiert werden. Im Allgemeinen arbeitet man bei Temperaturen zwischen 0˚C und 250˚C, vorzugsweise zwischen 50˚C und 150˚C.

Das erfindungsgemäße Verfahren (A) wird im Allgemeinen unter Normaldruck durchgeführt.

Bei der Durchführung des erfindungsgemäßen Verfahrens (A) setzt man die Reaktionskomponente der Formel (II) und die deprotonierende Base im Allgemeinen in äquimolaren bis etwa doppeltäquimolaren Mengen ein. Es ist jedoch auch möglich, die eine oder andere Komponente in einem größeren Überschuss (bis zu 3 Mol) zu verwenden.

Das Verfahren (B$\alpha$) ist **dadurch gekennzeichnet, dass** man Verbindungen der Formel (I-a) jeweils mit Carbonsäurehalogeniden der Formel (III) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (B$\alpha$) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan. Wenn die Hydrolysestabilität des Säurehalogenids es zulässt, kann die Umsetzung auch in Gegenwart von Wasser durchgeführt werden.

Als Säurebindemittel kommen bei der Umsetzung nach dem erfindungsgemäßen Verfahren (B$\alpha$) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperatur kann bei dem erfindungsgemäßen Verfahren (B$\alpha$) innerhalb eines größeren Bereiches variiert werden. Im Allgemeinen arbeitet man bei Temperaturen zwischen -20˚C und +150˚C, vorzugsweise zwischen 0˚C und 100˚C.

Bei der Durchführung des erfindungsgemäßen Verfahrens (Bα) werden die Ausgangsstoffe der Formel (I-a) und das Carbonsäurehalogenid der Formel (III) im Allgemeinen jeweils in angenähert äquivalenten Mengen verwendet. Es ist jedoch auch möglich, das Carbonsäurehalogenid in einem größeren Überschuss (bis zu 5 Mol) einzusetzen. Die Aufarbeitung erfolgt nach üblichen Methoden.

Das Verfahren (Bβ) ist **dadurch gekennzeichnet, dass** man Verbindungen der Formel (I-a) jeweils mit Carbonsäureanhydriden der Formel (IV) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (Bβ) vorzugsweise diejenigen Verdünnungsmittel verwendet werden, die auch bei der Verwendung von Säurehalogeniden vorzugsweise in Betracht kommen. Im übrigen kann auch ein im Überschuss eingesetztes Carbonsäureanhydrid gleichzeitig als Verdünnungsmittel fungieren.

Als gegebenenfalls zugesetzte Säurebindemittel kommen beim Verfahren (Bβ) vorzugsweise diejenigen Säurebindemittel in Frage, die auch bei der Verwendung von Säurehalogeniden vorzugsweise in Betracht kommen.

Die Reaktionstemperatur kann bei dem erfindungsgemäßen Verfahren (Bβ) innerhalb eines größeren Bereiches variiert werden. Im Allgemeinen arbeitet man bei Temperaturen zwischen -20°C und +150°C, vorzugsweise zwischen 0°C und 100°C.

Bei der Durchführung des erfindungsgemäßen Verfahrens (Bβ) werden die Ausgangsstoffe der Formel (I-a) und das Carbonsäureanhydrid der Formel (IV) im Allgemeinen in jeweils angenähert äquivalenten Mengen verwendet. Es ist jedoch auch möglich, das Carbonsäureanhydrid in einem größeren Überschuss (bis zu 5 Mol) einzusetzen. Die Aufarbeitung erfolgt nach üblichen Methoden.

Im Allgemeinen geht man so vor, dass man Verdünnungsmittel und im Überschuss vorhandenes Carbonsäureanhydrid sowie die entstehende Carbonsäure durch Destillation oder durch Waschen mit einem organischen Lösungsmittel oder mit Wasser entfernt.

Das Verfahren (C) ist **dadurch gekennzeichnet, dass** man Verbindungen der Formel (I-a) jeweils mit Chlorameisensäureestern oder Chlorameisensäurethiolestern der Formel (V) gegebenenfalls in Gegenwart eines Verdünnungsmittels und gegebenenfalls in Gegenwart eines Säurebindemittels umsetzt.

Als Säurebindemittel kommen bei dem erfindungsgemäßen Verfahren (C) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (C) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan.

Die Reaktionstemperatur kann bei der Durchführung des erfindungsgemäßen Verfahrens (C) innerhalb eines größeren Bereiches variiert werden. Die Reaktionstemperatur liegt im Allgemeinen zwischen -20°C und +100°C, vorzugsweise zwischen 0°C und 50°C.

Das erfindungsgemäße Verfahren (C) wird im Allgemeinen unter Normaldruck durchgeführt.

Bei der Durchführung des erfindungsgemäßen Verfahrens (C) werden die Ausgangsstoffe der Formel (I-a) und der entsprechende Chlorameisensäureester bzw. Chlorameisensäurethiolester der Formel (V) im Allgemeinen jeweils in angenähert äquivalenten Mengen verwendet. Es ist jedoch auch möglich, die eine oder andere Komponente in einem größeren Überschuss (bis zu 2 Mol) einzusetzen. Die Aufarbeitung erfolgt nach üblichen Methoden. Im Allgemeinen geht man so vor, dass man ausgefallene Salze entfernt und das verbleibende Reaktionsgemisch durch Abziehen des Verdünnungsmittels einengt.

Das erfindungsgemäße Verfahren (D) ist **dadurch gekennzeichnet, dass** man Verbindungen der Formel (I-a) jeweils mit (Dα) Verbindungen der Formel (VI) in Gegenwart eines Verdünnungsmittels und gegebenenfalls in Gegenwart eines Säurebindemittels oder (Dβ) Schwefelkohlenstoff und anschließend mit Alkylhalogeniden der Formel (VII) gegebenenfalls in Gegenwart eines Verdünnungsmittels und gegebenenfalls in Gegenwart einer Base umsetzt.

Beim Herstellungsverfahren (Dα) setzt man pro Mol Ausgangsverbindung der Formel (I-a) ca. 1 Mol Chlormonothioameisensäureester bzw. Chlordithioameisensäureester der Formel (VI) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Ester, Amide, Sulfone, Sulfoxide, aber auch Halogenalkane.

Vorzugsweise werden Dimethylsulfoxid, Ethylacetat, Tetrahydrofuran, Dimethylformamid oder Methylenchlorid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindung (I-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin und Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren (Dβ) setzt man pro Mol Ausgangsverbindungen der Formel (I-a) jeweils die äquimolare Menge bzw. einen Überschuss Schwefelkohlenstoff zu. Man arbeitet hierbei vorzugsweise bei Temperaturen von 0 bis 50˚C und insbesondere bei 20 bis 30˚C.

Oft ist es zweckmäßig zunächst aus den Verbindungen der Formel (I-a) durch Zusatz einer Base (wie z.B. Kaliumtertiärbutylat oder Natriumhydrid) das entsprechende Salz herzustellen. Man setzt die Verbindung (I-a) jeweils so lange mit Schwefelkohlenstoff um, bis die Bildung der Zwischenverbindung abgeschlossen ist, z.B. nach mehrstündigem Rühren bei Raumtemperatur.

Als Basen können beim Verfahren (Dβ) alle üblichen Protonenakzeptoren eingesetzt werden. Vorzugsweise verwendbar sind Alkalimetallhydride, Alkalimetallalkoholate, Alkali- oder Erdalkalimetallcarbonate oder -hydrogencarbonate oder Stickstoffbasen. Genannt seien beispielsweise Natriumhydrid, Natriummethanolat, Natriumhydroxid, Calciumhydroxid, Kaliumcarbonat, Natriumhydrogencarbonat, Triethylamin, Dibenzylamin, Diisopropylethylamin, Pyridin, Chinolin, Diazabicyclooctan (DABCO), Diazabicyclononen (DBN) und Diazabicycloundecen (DBU).

Als Verdünnungsmittel können bei diesem Verfahren alle üblichen Lösungsmittel verwendet werden.

Vorzugsweise sind verwendbar aromatische Kohlenwasserstoffe wie Benzol oder Toluol, Alkohole wie Methanol, Ethanol, Isopropanol oder Ethylenglykol, Nitrile wie Acetonitril, Ether wie Tetrahydrofuran oder Dioxan, Amide wie Dimethylformamid oder andere polare Lösungsmittel wie Dimethylsulfoxid oder Sulfolan.

Die weitere Umsetzung mit dem Alkylhalogenid der Formel (VII) erfolgt vorzugsweise bei 0 bis 70˚C und insbesondere bei 20 bis 50˚C. Hierbei wird mindestens die äquimolare Menge Alkylhalogenid eingesetzt.

Man arbeitet bei Normaldruck oder unter erhöhtem Druck, vorzugsweise bei Normaldruck.

Die Aufarbeitung erfolgt wiederum nach üblichen Methoden.

Das erfindungsgemäße Verfahren (E) ist **dadurch gekennzeichnet, dass** man Verbindungen der Formel (I-a) jeweils mit Sulfonsäurechloriden der Formel (VIII) gegebenenfalls in Gegenwart eines Verdünnungsmittels und gegebenenfalls in Gegenwart eines Säurebindemittels umsetzt.

Beim Herstellungsverfahren (E) setzt man pro Mol Ausgangsverbindung der Formel (I-a) ca. 1 Mol Sulfonsäurechlorid der Formel (VIII) bei -20 bis 150˚C, vorzugsweise bei 20 bis 70˚C um.

Das Verfahren (E) wird vorzugsweise in Gegenwart eines Verdünnungsmittels durchgeführt.

Als Verdünnungsmittel kommen alle inerten polaren organischen Lösungsmittel in Frage wie Ether, Ester, Amide, Nitrile, Sulfone, Sulfoxide oder halogenierte Kohlenwasserstoffe wie Methylenchlorid.

Vorzugsweise werden Dimethylsulfoxid, Tetrahydrofuran, Ethylacetat, Dimethylformamid, Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung (I-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin und Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (F) ist **dadurch gekennzeichnet, dass** man Verbindungen der Formel (I-a) jeweils mit Phosphorverbindungen der Formel (IX) gegebenenfalls in Gegenwart eines Verdünnungsmittels und gegebenenfalls in Gegenwart eines Säurebindemittels umsetzt.

Beim Herstellungsverfahren (F) setzt man zum Erhalt von Verbindungen der Formel (I-e) auf 1 Mol der Verbindung (I-a), 1 bis 2, vorzugsweise 1 bis 1,3 Mol der Phosphorverbindung der Formel (IX) bei Temperaturen zwischen -40˚C und 150˚C, vorzugsweise zwischen -10 und 110˚C um.

Das Verfahren (F) wird vorzugsweise in Gegenwart eines Verdünnungsmittels durchgeführt.

Als Verdünnungsmittel kommen alle inerten, polaren organischen Lösungsmittel in Frage wie Ether, Ester, Amide, Nitrile, Sulfide, Sulfone, Sulfoxide etc.

Vorzugsweise werden Acetonitril, Ethylacetat, Dimethylsulfoxid, Tetrahydrofuran, Dimethylformamid, Methylenchlorid eingesetzt.

Als gegebenenfalls zugesetzte Säurebindemittel kommen übliche anorganische oder organische Basen in Frage wie Hydroxide, Carbonate oder Amine. Beispielhaft seien Natriumhydroxid, Natriumcarbonat, Kaliumcarbonat, Pyridin und Triethylamin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der Organischen Chemie. Die Endprodukte werden vorzugsweise durch Kristallisation, chromatographische Reinigung oder durch sogenanntes "Andestillieren", d.h. Entfernung der flüchtigen Bestandteile im Vakuum gereinigt.

Das Verfahren (G) ist **dadurch gekennzeichnet, dass** man Verbindungen der Formel (I-a) jeweils mit Metallhydroxiden bzw. Metallalkoxiden der Formel (X) oder Aminen der Formel (XI), gegebenenfalls in Gegenwart eines Verdünnungsmittels, umsetzt.

Als Verdünnungsmittel können bei dem erfindungsgemäßen Verfahren (G) vorzugsweise Ether wie Tetrahydrofuran, Dioxan, Diethylether oder aber Alkohole wie Methanol, Ethanol, Isopropanol, aber auch Wasser eingesetzt werden. Das erfindungsgemäße Verfahren (G) wird im Allgemeinen unter Normaldruck durchgeführt. Die Reaktionstemperatur liegt im Allgemeinen zwischen -20°C und 100°C, vorzugsweise zwischen 0°C und 50°C.

Das erfindungsgemäße Verfahren (H) ist **dadurch gekennzeichnet, dass** man Verbindungen der Formel (I-a) jeweils mit (Hα) Verbindungen der Formel (XII) gegebenenfalls in Gegenwart eines Verdünnungsmittels und gegebenenfalls in Gegenwart eines Katalysators oder (Hβ) mit Verbindungen der Formel (XIII) gegebenenfalls in Gegenwart eines Verdünnungsmittels und gegebenenfalls in Gegenwart eines Säurebindemittels umsetzt.

Bei Herstellungsverfahren (Hα) setzt man pro Mol Ausgangsverbindung der Formel (I-a) ca. 1 Mol Isocyanat der Formel (XII) bei 0 bis 100°C, vorzugsweise bei 20 bis 50°C um.

Das Verfahren (Hα) wird vorzugsweise in Gegenwart eines Verdünnungsmittels durchgeführt.

Als Verdünnungsmittel kommen alle inerten organischen Lösungsmittel in Frage, wie Ether, Ester, Amide, Nitrile, Sulfone oder Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden.

Es wird vorzugsweise bei Normaldruck gearbeitet.

Beim Herstellungsverfahren (Hβ) setzt man pro Mol Ausgangsverbindung der Formel (I-a) ca. 1 Mol Carbamidsäurechlorid der Formel (XIII) bei 0 bis 150°C, vorzugsweise bei 20 bis 70°C um.

Als gegebenenfalls zugesetzte Verdünnungsmittel kommen alle inerten polaren organischen Lösungsmittel in Frage wie Ether, Ester, Amide, Sulfone, Sulfoxide oder halogenierte Kohlenwasserstoffe.

Vorzugsweise werden Dimethylsulfoxid, Ethylacetat, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung (I-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin oder Pyridin genannt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das Verfahren (I) ist **dadurch gekennzeichnet, dass** man Verbindungen der Formel (I-a'), in welchen A, B, D, Y, Z, $R^1$, $R^2$, $R^3$, $R^4$, $R^5$, $R^6$ und $R^7$ die oben genannten Bedeutungen haben und X' für Halogen besonders bevorzugt für Brom steht, mit Alkoholen der Formel (XXV), in welcher X die oben angegebene Bedeutung hat, in Gegenwart eines Kupfersalzes und in Gegenwart einer Base einer Austauschreaktion unterwirft.

Als Lösungsmittel kommen bei dem erfindungsgemäßen Verfahren (I) beispielsweise gegebenenfalls halogenierte aliphatische, alicyclische oder aromatische Kohlenwasserstoffe, wie beispielsweise Benzin, Benzol, Toluol, Xylol, Petrolether, Hexan, Cyclohexan, Chlorbenzol, Dichlorbenzol, Ether, wie Diethylether, Diisopropylether, Dioxan, Tetrahydrofuran oder Ethylenglykoldimethyl- oder -diethylether; Amide wie beispielsweise N,N-Dimethylformamid, N,N-Dimethylacetamid, N-Methylformanilid, N-Methylpyrrolidon oder Hexamethylphosphorsäuretriamid; Ester, wie Essigsäuremethylester oder Essigsäureethylester oder Mischungen solcher Lösungsmittel in Frage. Bevorzugt wird N,N-Dimethylformamid eingesetzt.

Als Basen können bei den erfindungsgemäßen Verfahren (I) Alkali- und/oder Erdalkalimetallcarbonate, -alkoholate, und/oder -hydroxide eingesetzt werden, wobei insbesondere Natriummethanolat, Natriumethylat, Natriumpropylat, Natriumbuytlat, Kalium-tert.-butylat, Kaliumamylat bevorzugt sind.

Als Kupfersalze werden bei dem erfindungsgemäßen Verfahren (I) Kupfer-I-salze eingesetzt wie z.B. CuBr, CuJ.

Die Reaktionstemperaturen können bei der Durchführung des Verfahrens (I) innerhalb eines größeren Bereiches variiert werden. Im Allgemeinen arbeitet man bei Temperaturen von 0 bis 250°C, bevorzugt bei 30 bis 200°C; ganz besonders bevorzugt bei 50 bis 150°C.

Das erfindungsgemäße Verfahren (I) wird im Allgemeinen unter Normaldruck durchgeführt.

Bei der Durchführung des erfindungsgemäßen Verfahrens (I) setzt man die Reaktionskomponenten der Formeln (I-1'-a') und (XXV) im Allgemeinen in äquimolaren Mengen ein. Es ist jedoch auch möglich, die Alkohole der Formel (XXV) in einem größeren Überschuss oder als Lösungsmittel zu verwenden. Die Basen werden im Allgemeinen im

Molverhältnis 1:1 bis 30:1 bevorzugt 2:1 bis 10:1 eingesetzt. Die Kupfersalze werden im Allgemeinen im Molverhältnis von 0,01:1 bis 1:1, bevorzugt 0,05:1 bis 0,5:1 eingesetzt.

Die Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren und Nematoden, die in der Landwirtschaft, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spp.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Acheta domesticus, Gryllotalpa spp., Locusta migratoria migratorioides, Melanoplus spp., Schistocerca gregaria.

Aus der Ordnung der Blattaria z.B. Blatta orientalis, Periplaneta americana, Leucophaea maderae, Blattella germanica.

Aus der Ordnung der Dermaptera z.B. Forficula auricularia.

Aus der Ordnung der Isoptera z.B. Reticulitermes spp.

Aus der Ordnung der Phthiraptera z.B. Pediculus humanus corporis, Haematopinus spp., Linognathus spp., Trichodectes spp., Damalinia spp.

Aus der Ordnung der Thysanoptera z.B. Hercinothrips femoralis, Thrips tabaci, Thrips palmi, Frankliniella accidentalis.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella xylostella, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Mamestra brassicae, Panolis flammea, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana, Cnaphalocerus spp., Oulema oryzae.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp.,

Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica, Lissorhoptrus oryzophilus.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemyia spp., Liriomyza spp.

Aus der Ordnung der Siphonaptera z.B. Xenopsylla cheopis, Ceratophyllus spp.

Aus der Klasse der Arachnida z.B. Scorpio maurus, Latrodectus mactans, Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp., Brevipalpus spp.

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp., Bursaphelenchus spp.

Die erfindungsgemäßen Verbindungen können gegebenenfalls in bestimmten Konzentrationen bzw. Aufwandmengen auch als Herbizide und Mikrobizide, beispielsweise als Fungizide, Antimykotika und Bakterizide verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injezieren und bei Vermehrungsmaterial, insbesondere bei Samen, weiterhin durch ein- oder mehrschichtiges Umhüllen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit,

Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

**Fungizide:**

2-Phenylphenol; 8-Hydroxyquinoline sulfate; Acibenzolar-S-methyl; Aldimorph; Amidoflumet; Ampropylfos; Ampropylfos-potassium; Andoprim; Anilazine; Azaconazole; Azoxystrobin; Benalaxyl; Benodanil; Benomyl; Benthiavalicarb-isopropyl; Benzamacril; Benzamacril-isobutyl; Bilanafos; Binapacryl; Biphenyl; Bitertanol; Blasticidin-S; Bromuconazole; Bupirimate; Buthiobate; Butylamine; Calcium polysulfide; Capsimycin; Captafol; Captan; Carbendazim; Carboxin; Carpropamid; Carvone; Chinomethionat; Chlobenthiazone; Chlorfenazole; Chloroneb; Chlorothalonil; Chlozolinate; Clozylacon; Cyazofamid; Cyflufenamid; Cymoxanil; Cyproconazole; Cyprodinil; Cyprofuram; Dagger G; Debacarb; Dichlofluanid; Dichlone; Dichlorophen; Diclocymet; Diclomezine; Dicloran; Diethofencarb; Difenoconazole; Diflumetorim; Dimethirimol; Dimethomorph; Dimoxystrobin; Diniconazole; Diniconazole-M; Dinocap; Diphenylamine; Dipyrithione; Ditalimfos; Dithianon; Dodine; Drazoxolon; Edifenphos; Epoxiconazole; Ethaboxam; Ethirimol; Etridiazole; Famoxadone; Fenamidone; Fenapanil; Fenarimol; Fenbuconazole; Fenfuram; Fenhexamid; Fenitropan; Fenoxanil; Fenpiclonil; Fenpropidin; Fenpropimorph; Ferbam; Fluazinam; Flubenzimine; Fludioxonil; Flumetover; Flumorph; Fluoromide; Fluoxastrobin; Fluquinconazole; Flurprimidol; Flusilazole; Flusulfamide; Flutolanil; Flutriafol; Folpet; Fosetyl-Al; Fosetyl-sodium; Fuberidazole; Furalaxyl; Furametpyr; Furcarbanil; Furmecyclox; Guazatine; Hexachlorobenzene; Hexaconazole; Hymexazol; Imazalil; Imibenconazole; Iminoctadine triacetate; Iminoctadine tris(albesilate); Iodocarb; Ipconazole; Iprobenfos; Iprodione; Iprovalicarb; Irumamycin; Isoprothiolane; Isovaledione; Kasugamycin; Kresoxim-methyl; Mancozeb; Maneb; Meferimzone; Mepanipyrim; Mepronil; Metalaxyl; Metalaxyl-M; Metconazole; Methasulfocarb; Methfuroxam; Metiram; Metominostrobin; Metsulfovax; Mildiomycin; Myclobutanil; Myclozolin; Natamycin; Nicobifen; Nitrothal-isopropyl; Noviflumuron; Nuarimol; Ofurace; Orysastrobin; Oxadixyl; Oxolinic acid; Oxpoconazole; Oxycarboxin; Oxyfenthiin; Paclobutrazol; Pefurazoate; Penconazole; Pencycuron; Phosdiphen; Phthalide; Picoxystrobin; Piperalin; Polyoxins; Polyoxorim; Probenazole; Prochloraz; Procymidone; Propamocarb; Propanosine-sodium; Propiconazole; Propineb; Proquinazid; Prothioconazole; Pyraclostrobin; Pyrazophos; Pyrifenox; Pyrimethanil; Pyroquilon; Pyroxyfur; Pyrrolnitrine; Quinconazole; Quinoxyfen; Quintozene; Simeconazole; Spiroxamine; Sulfur; Tebuconazole; Tecloftalam; Tecnazene; Tetcyclacis; Tetraconazole; Thiabendazole; Thicyofen; Thifluzamide; Thiophanate-methyl; Thiram; Tioxymid; Tolclofos-methyl; Tolylfluanid; Triadimefon; Triadimenol; Triazbutil; Triazoxide; Tricyclamide; Tricyclazole; Tridemorph; Trifloxystrobin; Triflumizole; Triforine; Triticonazole; Uniconazole; Validamycin A; Vinclozolin; Zineb; Ziram; Zoxamide; (2S)-N-[2-[4-[[3-(4-chlorophenyl)-2-propynyl]oxy]-3-methoxyphenyl]-ethyl]-3-methyl- 2-[(methylsulfonyl)amino]-butanamide; 1-(1-naphthalenyl)-1H-pyrrole-2,5-dione; 2,3,5,6-tetrachloro-4-(methylsulfonyl)-pyridine; 2-amino-4-methyl-N-phenyl-5-thiazolecarboxamide; 2-chloro-N-(2,3-dihydro-1,1,3-trimethyl-1H-inden-4-yl)-3-pyridincarboxamide; 3,4,5-trichloro-2,6-pyridinedicarbonitrile; Actinovate; cis-1-(4-chlorophenyl)-2-(1H-1,2,4-triazole-1-yl)-cycloheptanol; methyl 1-(2,3-dihydro-2,2-dimethyl-1H-inden-1-yl)-1H-imidazole-5-carboxylate; monopotassium carbonate; N-(6-methoxy-3-pyridinyl)-cyclopropanecarboxamide; N-butyl-8-(1,1-dimethylethyl)-1-oxaspiro[4.5]decan-3-amine; Sodium

tetrathiocarbonate;
sowie Kupfersalze und -zubereitungen, wie Bordeaux mixture; Copper hydroxide; Copper naphthenate; Copper oxychloride; Copper sulfate; Cufraneb; Cuprous oxide; Mancopper; Oxinecopper.

**Bakterizide:**

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

**Insektizide / Akarizide / Nematizide**

1. Acetylcholinesterase (AChE) Inhibitoren

1.1 Carbamate, zum Beispiel
Alanycarb, Aldicarb, Aldoxycarb, Allyxycarb, Aminocarb, Bendiocarb, Benfuracarb, Bufencarb, Butacarb, Butocarboxim, Butoxycarboxim, Carbaryl, Carbofuran, Carbosulfan, Cloethocarb, Dimetilan, Ethiofencarb, Fenobucarb, Fenothiocarb, Formetanate, Furathiocarb, Isoprocarb, Metam-sodium, Methiocarb, Methomyl, Metolcarb, Oxamyl, Pirimicarb, Promecarb, Propoxur, Thiodicarb, Thiofanox, Trimethacarb, XMC, Xylylcarb
Triazamate

1.2 Organophosphate, zum Beispiel
Acephate, Azamethiphos, Azinphos (-methyl, -ethyl), Bromophos-ethyl, Bromfenvinfos (-methyl), Butathiofos, Cadusafos, Carbophenothion, Chlorethoxyfos, Chlorfenvinphos, Chlormephos, Chlorpyrifos (-methyl/-ethyl), Coumaphos, Cyanofenphos, Cyanophos, Chlorfenvinphos, Demeton-S-methyl, Demeton-S-methylsulphon, Dialifos, Diazinon, Dichlofenthion, Dichlorvos/DDVP, Dicrotophos, Dimethoate, Dimethylvinphos, Dioxabenzofos, Disulfoton, EPN, Ethion, Ethoprophos, Etrimfos, Famphur, Fenamiphos, Fenitrothion, Fensulfothion, Fenthion, Flupyrazofos, Fonofos, Formothion, Fosmethilan, Fosthiazate, Heptenophos, Iodofenphos, Iprobenfos, Isazofos, Isofenphos, Isopropyl O-salicylate, Isoxathion, Malathion, Mecarbam, Methacrifos, Methamidophos, Methidathion, Mevinphos, Monocrotophos, Naled, Omethoate, Oxydemeton-methyl, Parathion (-methyl/-ethyl), Phenthoate, Phorate, Phosalone, Phosmet, Phosphamidon, Phosphocarb, Phoxim, Pirimiphos (-methyl/-ethyl), Profenofos, Propaphos, Propetamphos, Prothiofos, Prothoate, Pyraclofos, Pyridaphenthion, Pyridathion, Quinalphos, Sebufos, Sulfotep, Sulprofos, Tebupirimfos, Temephos, Terbufos, Tetrachlorvinphos, Thiometon, Triazophos, Triclorfon, Vamidothion

2. Natrium-Kanal-Modulatoren / Spannungsabhängige Natrium-Kanal-Blocker

2.1 Pyrethroide, zum Beispiel
Acrinathrin, Allethrin (d-cis-trans, d-trans), Beta-Cyfluthrin, Bifenthrin, Bioallethrin, Bioallethrin-S-cyclopentylisomer, Bioethanomethrin, Biopermethrin, Bioresmethrin, Chlovaporthrin, Cis-Cypermethrin, Cis-Resmethrin, Cis-Permethrin, Clocythrin, Cycloprothrin, Cyfluthrin, Cyhalothrin, Cypermethrin (alpha-, beta-, theta-, zeta-), Cyphenothrin, Deltamethrin, Empenthrin (1R-isomer), Esfenvalerate, Etofenprox, Fenfluthrin, Fenpropathrin, Fenpyrithrin, Fenvalerate, Flubrocythrinate, Flucythrinate, Flufenprox, Flumethrin, Fluvalinate, Fubfenprox, Gamma-Cyhalothrin, Imiprothrin, Kadethrin, Lambda-Cyhalothrin, Metofluthrin, Permethrin (cis-, trans-), Phenothrin (1R-trans isomer), Prallethrin, Profluthrin, Protrifenbute, Pyresmethrin, Resmethrin, RU 15525, Silafluofen, Tau-Fluvalinate, Tefluthrin, Terallethrin, Tetramethrin (-1R- isomer), Tralomethrin, Transfluthrin, ZXI 8901, Pyrethrins (pyrethrum)
DDT

2.2 Oxadiazine, zum Beispiel Indoxacarb

3. Acetylcholin-Rezeptor-Agonisten/-Antagonisten

3.1 Chloronicotinyle, zum Beispiel
Acetamiprid, Clothianidin, Dinotefuran, Imidacloprid, Nitenpyram, Nithiazine, Thiacloprid, Thiamethoxam

3.2 Nicotine, Bensultap, Cartap

4. Acetylcholin-Rezeptor-Modulatoren

4.1 Spinosyne, zum Beispiel Spinosad

5. GABA-gesteuerte Chlorid-Kanal-Antagonisten

5.1 Cyclodiene Organochlorine, zum Beispiel
Camphechlor, Chlordane, Endosulfan, Gamma-HCH, HCH, Heptachlor, Lindane, Methoxychlor

5.2 Fiprole, zum Beispiel
Acetoprole, Ethiprole, Fipronil, Vaniliprole

6. Chlorid-Kanal-Aktivatoren

6.1 Mectine, zum Beispiel
Avermectin, Emamectin, Emamectin-benzoate, Ivermectin, Milbemycin

7. Juvenilhormon-Mimetika, zum Beispiel
Diofenolan, Epofenonane, Fenoxycarb, Hydroprene, Kinoprene, Methoprene, Pyriproxifen, Triprene

8. Ecdysonagonisten/disruptoren

8.1 Diacylhydrazine, zum Beispiel
Chromafenozide, Halofenozide, Methoxyfenozide, Tebufenozide

9. Inhibitoren der Chitinbiosynthese

9.1 Benzoylharnstoffe, zum Beispiel
Bistrifluron, Chlofluazuron, Diflubenzuron, Fluazuron, Flucycloxuron, Flufenoxuron, Hexaflumuron, Lufenuron, Novaluron, Noviflumuron, Penfluron, Teflubenzuron, Triflumuron

9.2 Buprofezin

9.3 Cyromazine

10. Inhibitoren der oxidativen Phosphorylierung, ATP-Disruptoren

10.1 Diafenthiuron

10.2 Organotine, zum Beispiel Azocyclotin, Cyhexatin, Fenbutatin-oxide

11. Entkoppler der oxidativen Phoshorylierung durch Unterbrechung des H-Protongradienten

11.1 Pyrrole, zum Beispiel Chlorfenapyr

11.2 Dinitrophenole, zum Beispiel Binapacyrl, Dinobuton, Dinocap, DNOC

12. Seite-I-Elektronentransportinhibitoren

12.1 METI's, zum Beispiel Fenazaquin, Fenpyroximate, Pyrimidifen, Pyridaben, Tebufenpyrad, Tolfenpyrad

12.2 Hydramethylnon

12.3 Dicofol

13. Seite-II-Elektronentransportinhibitoren
Rotenone

14. Seite-III-Elektronentransportinhibitoren
Acequinocyl, Fluacrypyrim

15. Mikrobielle Disruptoren der Insektendarmmembran
Bacillus thuringiensis-Stämme

16. Inhibitoren der Fettsynthese
Tetronsäuren, zum Beispiel
Spirodiclofen, Spiromesifen
Tetramsäuren, zum Beispiel
3-(2,5-Dimethylphenyl)-8-methoxy-2-oxo-1-azaspiro[4.5]dec-3-en-4-yl ethyl carbonate (alias: Carbonic acid, 3-(2,5-dimethylphenyl)-8-methoxy-2-oxo-1-azaspiro[4.5]dec-3-en-4-yl ethyl ester, CAS-Reg.-No.: 382608-10-8) and Carbonic acid, cis-3-(2,5-dimethylphenyl)-8-methoxy-2-oxo-1-azaspiro[4.5]dec-3-en-4-yl ethyl ester (CAS-Reg.-No.: 203313-25-1)

17. Carboxamide, zum Beispiel Flonicamid

18. Oktopaminerge Agonisten, zum Beispiel Amitraz

19. Inhibitoren der Magnesium-stimulierten ATPase, zum Beispiel Propargite

20. BDCAs, zum Beispiel N2-[1,1-Dimethyl-2-(methylsulfonyl)ethyl]-3-iodo-N1-[2-methyl-4-[1,2,2,2-tetrafluoro-1-(trifluoromethyl)ethyl]phenyl]-1,2-benzenedicarboxamide (CAS-Reg.-No.: 272451-65-7)

21. Nereistoxin-Analoge, zum Beispiel Thiocyclam hydrogen oxalate, Thiosultap-sodium

22. Biologika, Hormone oder Pheromone, zum Beispiel
Azadirachtin, Bacillus spec., Beauveria spec., Codlemone, Metarrhizium spec., Paecilomyces spec., Thuringiensin, Verticillium spec.

23. Wirkstoffe mit unbekannten oder nicht spezifischen Wirkmechanismen

23.1 Begasungsmittel, zum Beispiel
Aluminium phosphide, Methyl bromide, Sulfuryl fluoride

23.2 Selektive Fraßhemmer, zum Beispiel
Cryolite, Flonicamid, Pymetrozine

23.3 Milbenwachstumsinhibitoren, zum Beispiel
Clofentezine, Etoxazole, Hexythiazox

23.4 Amidoflumet, Benclothiaz, Benzoximate, Bifenazate, Bromopropylate, Buprofezin, Chinomethionat, Chlordimeform, Chlorobenzilate, Chloropicrin, Clothiazoben, Cycloprene, Dicyclanil, Fenoxacrim, Fentrifanil, Flubenzimine, Flufenerim, Flutenzin, Gossyplure, Hydramethylnone, Japonilure, Metoxadiazone, Petroleum, Piperonyl butoxide, Potassium oleate, Pyridalyl, Sulfluramid, Tetradifon, Tetrasul, Triarathene,Verbutin,

sowie Präparate, welche insektizid wirksame Pflanzenextrakte, Nematoden, Pilze oder Viren enthalten.

Auch eine Mischung mit anderen bekannten Wirkstoffen, wie Herbiziden oder mit Düngemitteln und Wachstumsregulatoren Safenern bzw. Semichemicals ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten

Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepassten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetic Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Tabak, Raps sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden.

Die bei den Wirkstoffen bzw. Mischungen oben angegebenen Vorzugsbereiche gelten auch für die Behandlung

dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp.

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp.

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp.

Aus der Ordnung der Siphonapterida z.B. Pulex spp., Ctenocephalides spp., Xenopsylla spp., Ceratophyllus spp.

Aus der Ordnung der Heteropterida z.B. Cimex spp., Triatoma spp., Rhodnius spp., Panstrongylus spp.

Aus der Ordnung der Blattarida z.B. Blatta orientalis, Periplaneta americana, Blattela germanica, Supella spp.

Aus der Unterklasse der Acari, (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp.

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, dass die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:

Käfer wie

Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.

Hautflügler wie

Sirex juvencus, Urocerus gigas, Urocerus gigas taignus, Urocerus augur.

Termiten wie

Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.
Borstenschwänze wie Lepisma saccharina.
Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.
Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen:

Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.
Die Wirkstoffe können als solche, in Form von Konzentraten oder allgemein üblichen Formulierungen wie Pulver, Granulate, Lösungen, Suspensionen, Emulsionen oder Pasten angewendet werden.
Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.
Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.
Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.
Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.
Als organisch-chemische Lösungsmittel werden vorzugsweise ölige oder ölartige Lösungsmittel mit einer Verdunstungszahl über 35 und einem Flammpunkt oberhalb 30˚C, vorzugsweise oberhalb 45˚C, eingesetzt. Als derartige schwerflüchtige, wasserunlösliche, ölige und ölartige Lösungsmittel werden entsprechende Mineralöle oder deren Aromatenfraktionen oder mineral-ölhaltige Lösungsmittelgemische, vorzugsweise Testbenzin, Petroleum und/oder Alkylbenzol verwendet.
Vorteilhaft gelangen Mineralöle mit einem Siedebereich von 170 bis 220˚C, Testbenzin mit einem Siedebereich von 170 bis 220˚C, Spindelöl mit einem Siedebereich von 250 bis 350˚C, Petroleum bzw. Aromaten vom Siedebereich von 160 bis 280˚C, Terpentinöl und dgl. zum Einsatz.
In einer bevorzugten Ausführungsform werden flüssige aliphatische Kohlenwasserstoffe mit einem Siedebereich von 180 bis 210˚C oder hochsiedende Gemische von aromatischen und aliphatischen Kohlenwasserstoffen mit einem Siedebereich von 180 bis 220˚C und/oder Spindeöl und/oder Monochlornaphthalin, vorzugsweise $\alpha$-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, dass das Lösungsmittelgemisch ebenfalls eine Verdunstungszahl über 35 und einen Flammpunkt oberhalb 30°C, vorzugsweise oberhalb 45°C, aufweist und dass das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher (gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron, Transfluthrin, Thiacloprid, Methoxyphenoxid, Triflumuron, Chlothianidin, Spinosad, Tefluthrin,
sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, sein.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln), wie verschiedene Lepas- und Scalpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflusskrebse) zusammengefasst werden, besondere Bedeutung zu.

Es wurde nun überraschenderweise gefunden, dass die erfindungsgemäßen Verbindungen allein oder in Kombination mit anderen Wirkstoffen, eine hervorragende Antifouling (Antibewuchs)-Wirkung aufweisen.

Durch Einsatz von erfindungsgemäßen Verbindungen allein oder in Kombination mit anderen Wirkstoffen, kann auf den Einsatz von Schwermetallen wie z.B. in Bis(trialkylzinn)-sulfiden, Tri-n-butylzinnlaurat, Tri-n-butylzinnchlorid, Kupfer(I)-oxid, Triethylzinnchlorid, Tri-n-butyl(2-phenyl-4-chlorphenoxy)-zinn, Tributylzinnoxid, Molybdändisulfid, Antimonoxid, polymerem Butyltitanat, Phenyl-(bispyridin)-wismutchlorid, Tri-n-butylzinnfluorid, Manganethylenbisthiocarbamat, Zinkdimethyldithiocarbamat, Zinkethylenbisthiocarbamat, Zink- und Kupfersalze von 2-Pyridinthiol-1-oxid, Bisdimethyldithiocarbamoylzinkethylenbisthiocarbamat, Zinkoxid, Kupfer(I)-ethylen-bisdithiocarbamat, Kupferthiocyanat, Kupfernaphthenat und Tributylzinnhalogeniden verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling-Mittel eignen sich vorzugsweise:

Algizide wie

2-*tert*.-Butylamino-4-cyclopropylamino-6-methylthio-1,3,5-triazin, Dichlorophen, Diuron, Endothal, Fentinacetat, Isoproturon, Methabenzthiazuron, Oxyfluorfen, Quinoclamine und Terbutryn;

Fungizide wie

Benzo[*b*]thiophencarbonsäurecyclohexylamid-S,S-dioxid, Dichlofluanid, Fluorfolpet, 3-Iod-2-propinyl-butylcarbamat, Tolylfluanid und Azole wie

Azaconazole, Cyproconazole, Epoxyconazole, Hexaconazole, Metconazole, Propiconazole und Tebuconazole;

Molluskizide wie

Fentinacetat, Metaldehyd, Methiocarb, Niclosamid, Thiodicarb und Trimethacarb, Fe-chelate;

oder herkömmliche Antifouling-Wirkstoffe wie

4,5-Dichlor-2-octyl-4-isothiazolin-3-on, Diiodmethylparatrylsulfon, 2-(N,N-Dimethylthiocarbamoylthio)-5-nitrothiazyl, Kalium-, Kupfer-, Natrium- und Zinksalze von 2-Pyridinthiol-1-oxid, Pyridin-triphenylboran, Tetrabutyldistannoxan, 2,3,5,6-Tetrachlor-4-(methylsulfonyl)-pyridin, 2,4,5,6-Tetrachloroisophthalonitril, Tetramethylthiuramdisulfid und 2,4,6-Trichlorphenylmaleinimid.

Die verwendeten Antifouling-Mittel enthalten die erfindungsgemäßen Wirkstoff der erfindungsgemäßen Verbindungen in einer Konzentration von 0,001 bis 50 Gew.-%, insbesondere von 0,01 bis 20 Gew.-%.

Die erfindungsgemäßen Antifouling-Mittel enthalten desweiteren die üblichen Bestandteile wie z.B. in Ungerer, Chem. Ind. 1985, 37, 730-732 und Williams, Antifouling Marine Coatings, Noyes, Park Ridge, 1973 beschrieben.

Antifouling-Anstrichmittel enthalten neben den algiziden, fungiziden, molluskiziden und erfindungsgemäßen insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wässrigen System, Vinylchlorid/Vinylacetat-Copolymersysteme in Form wässriger Dispersionen oder in Form von organischen Lösungsmittelsystemen, Butadien/- Styrol/Acrylnitril-Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self-Polishing-Antifouling-Systemen können die erfindungsgemäßen Verbindungen oder die oben genannten Mischungen eingearbeitet werden.

Die Wirkstoffe eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:

Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coloptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die erfindungsgemäßen Wirkstoffe können auch als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive

Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.

Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe ist jedoch keineswegs auf diese Gattungen beschränkt, sondern erstreckt sich in gleicher Weise auch auf andere Pflanzen.

Die erfindungsgemäßen Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuss-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen der Formel (I) zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die erfindungsgemäßen Wirkstoffe können in bestimmten Konzentrationen bzw. Aufwandmengen auch zur Bekämpfung von tierischen Schädlingen und pilzlichen oder bakteriellen Pflanzenkrankheiten verwendet werden. Sie lassen sich gegebenenfalls auch als Zwischen- oder Vorprodukte für die Synthese weiterer Wirkstoffe einsetzen.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden und/oder mit Stoffen, welche die Kulturpflanzen-Verträglichkeit verbessern ("Safenern") zur Unkrautbekämpfung verwendet werden, wobei Fertigformulierungen oder Tankmischungen möglich sind. Es sind also auch Mischungen mit Unkrautbekämpfungsmitteln möglich, welche ein oder mehrere bekannte Herbizide und einen Safener enthalten.

Für die Mischungen kommen bekannte Herbizide infrage, beispielsweise

Acetochlor, Acifluorfen (-sodium), Aclonifen, Alachlor, Alloxydim (-sodium), Ametryne, Amicarbazone, Amidochlor, Amidosulfuron, Aminopyralid, Anilofos, Asulam, Atrazine, Azafenidin, Azimsulfuron, Beflubutamid, Benazolin (-ethyl), Bencarbazone, Benfuresate, Bensulfuron (-methyl), Bentazon, Benzfendizone, Benzobicyclon, Benzofenap, Benzoylprop (-ethyl), Bialaphos, Bifenox, Bispyribac (-sodium), Bromobutide, Bromofenoxim, Bromoxynil, Butachlor, Butafenacil (-allyl), Butroxydim, Butylate, Cafenstrole, Caloxydim, Carbetamide, Carfentrazone (-ethyl), Chlomethoxyfen, Chloramben, Chloridazon, Chlorimuron (-ethyl), Chlornitrofen, Chlorsulfuron, Chlortoluron, Cinidon (-ethyl), Cinmethylin, Cinosulfuron, Clefoxydim, Clethodim, Clodinafop (-propargyl), Clomazone, Clomeprop, Clopyralid, Clopyrasulfuron (-methyl), Cloransulam (-methyl), Cumyluron, Cyanazine, Cybutryne, Cycloate, Cyclosulfamuron, Cycloxydim, Cyhalofop (-butyl), 2,4-D, 2,4-DB, Desmedipham, Diallate, Dicamba, Dichlorprop (-P), Diclofop (-methyl), Diclosulam, Diethatyl (-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron (-methyl), Ethofumesate, Ethoxyfen, Ethoxysulfuron, Etobenzanid, Fenoxaprop (-P-ethyl), Fentrazamide, Flamprop (-isopropyl, -isopropyl-L, -methyl), Flazasulfuron, Florasulam, Fluazifop (-P-butyl), Fluazolate, Flucarbazone (-sodium), Flucetosulfuron, Flufenacet, Flumetsulam, Flumiclorac (-pentyl), Flumioxazin, Flumipropyn, Flumetsulam, Fluometuron, Fluorochloridone, Fluoroglycofen (-ethyl), Flupoxam, Flupropacil, Flurpyrsulfuron (-methyl, -sodium), Flurenol (-butyl), Fluridone, Fluroxypyr (-butoxypropyl, -meptyl), Flurprimidol, Flurtamone, Fluthiacet (-methyl), Fluthiamide, Fomesafen, Foramsulfuron, Glufosinate (-ammonium), Glyphosate (-isopropylammonium), Halosafen, Haloxyfop (-ethoxyethyl, -P-methyl), Hexazinone, HOK-201, Imazamethabenz (-methyl), Imazamethapyr, Imazamox, Imazapic, Imazapyr, Imazaquin, Imazethapyr, Imazosulfuron, Iodosulfuron (-methyl, -sodium), Ioxynil, Isopropalin, Isoproturon, Isouron, Isoxaben, Isoxachlortole, Isoxaflutole, Isoxapyrifop, Lactofen, Lenacil, Linuron, MCPA, Mecoprop, Mefenacet, Mesosulfurone, Mesotrione, Metamifop, Metamitron, Metazachlor, Methabenzthiazuron, Metobenzuron, Metobromuron, (alpha-) Metolachlor, Metosulam, Metoxuron, Metribuzin, Metsulfuron (-methyl), Molinate, Monolinuron, Naproanilide, Napropamide, Neburon, Nicosulfuron, Norflurazon, Orbencarb, Orthosulfamuron, Oryzalin, Oxadiargyl, Oxadiazon, Oxasulfuron, Oxaziclomefone, Oxyfluorfen, Paraquat, Pelargonsäure, Pendimethalin, Pendralin, Penoxsulam, Pentoxazone, Phenmedipham, Picolinafen, Pinoxaden, Piperophos, Pretilachlor, Primisulfuron (-methyl), Profluazol, Prometryn, Propachlor, Propanil, Propaquizafop, Propisochlor, Propoxycarbazone (-sodium), Propyzamide, Prosulfocarb, Prosulfuron, Pyraflufen (-ethyl), Pyrasulfotole, Pyrazogyl, Pyrazolate, Pyrazosulfuron (-ethyl), Pyrazoxyfen, Pyribenzoxim, Pyributicarb, Pyridate, Pyridatol, Pyriftalid, Pyriminobac (-methyl), Pyrithiobac (-sodium), Pyrimisulfan, Quinchlorac, Quinmerac, Quinoclamine, Quizalofop (-P-ethyl, -P-tefuryl), Rimsulfuron, Sethoxydim, Simazine, Simetryn, Sulcotrione, Sulfentrazone, Sulfometuron (-methyl), Sulfosate, Sulfosulfuron, Tebutam, Tebuthiuron, Tembotrione, Tepraloxydim, Terbuthylazine, Terbutryn, Thenylchlor, Thiafluamide, Thiazopyr, Thidiazimin, Thiencarbazone-methyl, Thifensulfuron (-methyl), Thiobencarb, Tiocarbazil, Topramezone, Tralkoxydim, Triallate, Triasulfuron, Tribenuron (-methyl), Triclopyr, Tridiphane, Trifluralin, Trifloxysulfuron, Triflusulfuron (-methyl), Tritosulfuron, Triflosulam,

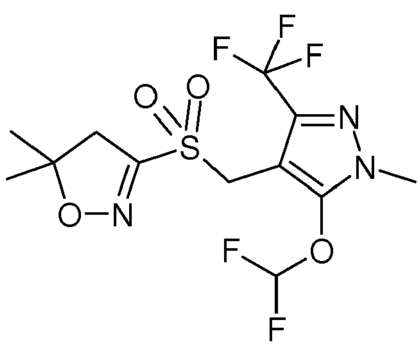

KIH 485

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe bzw. Wirkstoffkombinationen können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe bzw. Wirkstoffkombinationen können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im Allgemeinen liegen die Aufwandmengen zwischen 1 g und 10 kg Wirkstoff pro Hektar Bodenfläche, vorzugsweise zwischen 5 g und 5 kg pro ha.

Der vorteilhafte Effekt der Kulturpflanzen-Verträglichkeit der erfindungsgemäßen Wirkstoffkombinationen ist bei bestimmten Konzentrationsverhältnissen besonders stark ausgeprägt. Jedoch können die Gewichtsverhältnisse der Wirkstoffe in den Wirkstoffkombinationen in relativ großen Bereichen variiert werden. Im allgemeinen entfallen auf 1 Gewichtsteil Wirkstoff der Formel (I)

Salzen 0,001 bis 1000 Gewichtsteile, vorzugsweise 0,01 bis 100 Gewichtsteile, besonders bevorzugt 0,05 bis 20 Gewichtsteile einer der oben unter (b') genannten, die Kulturpflanzen Verträglichkeit verbessernden Verbindungen (Antidots/Safener).

Die erfindungsgemäßen Wirkstoffkombinationen werden im allgemeinen in Form von Fertigformulierungen zur Anwendung gebracht. Die in den Wirkstoffkombinationen enthaltenen Wirkstoffe können aber auch in Einzelformulierungen bei der Anwendung gemischt, d.h. in Form von Tankmischungen zur Anwendung gebracht werden.

Für bestimmte Anwendungszwecke, insbesondere im Nachauflauf-Verfahren, kann es ferner vorteilhaft sein, in die Formulierungen als weitere Zusatzstoffe pflanzenverträgliche mineralische oder vegetabilische Öle (z.B. das Handelspräparat "Rako Binol") oder Ammoniumsalze wie z.B. Ammoniumsulfat oder Ammoniumrhodanid aufzunehmen.

Die neuen Wirkstoffkombinationen können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Stäuben oder Streuen.

Die Aufwandmengen der erfindungsgemäßen Wirkstoffkombinationen können in einem gewissen Bereich variiert werden; sie hängen u.a. vom Wetter und von den Bodenfaktoren ab. Im allgemeinen liegen die Aufwandmengen zwischen 0,001 und 5 kg pro ha, vorzugsweise zwischen 0,005 und 2 kg pro ha, besonders bevorzugt zwischen 0,01 und 0,5 kg pro ha.

Die erfindungsgemäßen Wirkstoffkombinationen können vor und nach dem Auflaufen der Pflanzen appliziert werden, also im Vorauflauf und Nachauflauf-Verfahren.

Die erfindungsgemäß einzusetzenden Safener können je nach ihren Eigenschaften zur Vorbehandlung des Saatgutes der Kulturpflanze (Beizung der Samen) verwendet werden oder vor der Saat in die Saatfurchen eingebracht oder vor dem Herbizid separat angewendet werden oder zusammen mit dem Herbizid vor oder nach dem Ablaufen der Pflanzen angewendet werden.

Als Beispiele der Pflanzen werden die wichtigen Kulturpflanzen, wie Getreide (Weizen, Gerste, Reis), Mais, Soja, Kartoffel, Baumwolle, Raps, Rüben, Zuckerrohr sowie Obstpflanzen (mit den Früchten Äpfel, Birnen, Zitrusfrüchten und Weintrauben) erwähnt, wobei Getreide, Mais, Soja, Kartoffel, Baumwolle und Raps besonders hervorgehoben werden.

Die Bezeichnung "Wirkstoffe" schließt immer auch die hier genannten Wirkstoffkombinationen mit ein.

Die Herstellung und die Verwendung der erfindungsgemäßen Wirkstoffe geht aus den nachfolgenden Beispielen hervor.

## Herstellungsbeispiele

Beispiel I-a-1

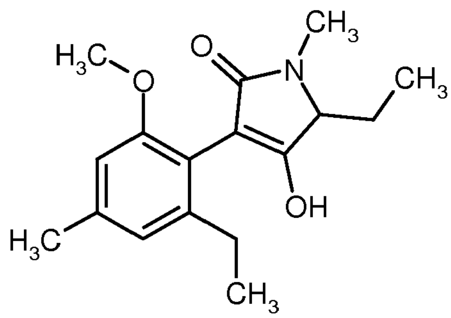

Verfahren (I)

1 g der Verbindung gemäß Beispiel I-a-6 aus WO 05/044791 und 0,085 g Kupfer-I-bromid und 0,89 ml Essigsäuremethylester in 2,77 ml Natriummethylatlösung (30 %) geben. Es wird 4 Stunden unter Rückfluss gerührt. Die Reaktionslösung wird bis zur Trockne einrotiert und der Rückstand in Wasser aufgenommen. Nach Filtration wird mit 1 N HCl-Lösung auf pH 1 gestellt und 10 min bei Raumtemperatur gerührt.

Der Niederschlag wird über eine Fritte abgesaugt.
Ausbeute: 0,6 g (70 % der Theorie). Fp. 85-87°C

Beispiel I-a-4

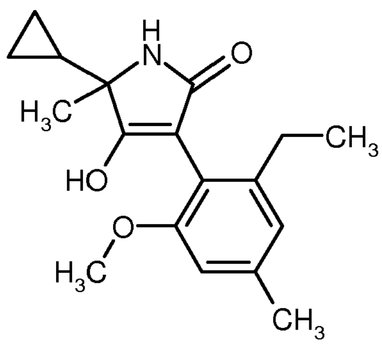

Verfahren (A)

1,63 g Kalium-tert.-butylat in 20 ml Dimethylformamid (DMF) vorlegen und 2,2 g der Verbindung gemäß Beispiel II-1 in 20 ml DMF langsam zutropfen. Bei Raumtemperatur rühren. Nach Abschluss der Reaktion (dünnschichtchromatographische Kontrolle) das Lösungsmittel abdestillieren, den Rückstand in 200 ml Wasser aufnehmen und mit 1 N HCl auf pH 2 stellen. Es wird mit Essigsäureethylester extrahiert, über Natriumsulfat getrocknet und das Lösungsmittel abdestilliert.
Ausbeute: 1,71 g (86 % der Theorie), Fp. 180-188°C.

In Analogie zu Beispiel (I-a-1) und (I-a-4) und gemäß den allgemeinen Angaben zur Herstellung werden folgende Verbindungen der Formel (I-a) erhalten:

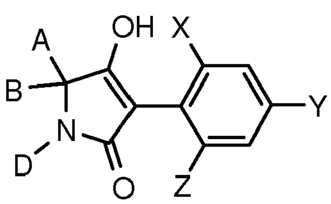

(I-a)

| Bsp.-Nr. | X | Y | Z | D | A | B | Fp.°C | Isomer |
|---|---|---|---|---|---|---|---|---|
| I-a-2 | $OCH_3$ | $CH_3$ | $C_2H_5$ | $-(CH_2)_3-$ | | H | 120-121 | - |
| I-a-3 | $OCH_3$ | $CH_3$ | $C_2H_5$ | H | $CH_3$ | $CH_3$ | 147-148 | - |
| I-a-4 | $OCH_3$ | $CH_3$ | $C_2H_5$ | H | 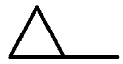 | $CH_3$ | 180-182 | - |
| I-a-5 | $OCH_3$ | $CH_3$ | $C_2H_5$ | H | $-(CH_2)_2-CHCH_3-(CH_2)_2-$ | | 229-232 | B |
| I-a-6 | $OCH_3$ | $CH_3$ | $C_2H_5$ | H | $-(CH_2)_2-CHOCH_3-(CH_2)_2-$ | | 112 | B |
| I-a-7 | $OCH_3$ | $CH_3$ | $C_2H_5$ | H | $-CH_2-CHOC_4H_9-(CH_2)_3-$ | | *2.34 (m, 2H, Ar-$CH_2$) 3.42 (t, 2H, O-$CH_2$) | B |
| I-a-8 | $OC_2H_5$ | $CH_3$ | $C_2H_5$ | H | $-(CH_2)_2-CHOCH_3-(CH_2)_2-$ | | *3.26 (s, 3H, O$\underline{CH_3}$) 3.85 (m, 2H, Ar-O-$\underline{CH_2}$) | β |
| I-a-9 | $OCH_3$ | $CH_3$ | $C_2H_5$ | H | $-CH_2-CHOCH_3-(CH_2)_3-$ | | *3.26 (s, 3H, O$\underline{CH_3}$) 3.66 (s, 3H, Ar- O$\underline{CH_3}$ | B |

$^1$H-NMR (400 MHz, $d_6$-DMSO): Verschiebung δ in ppm

<u>Beispiel I-b-1</u>

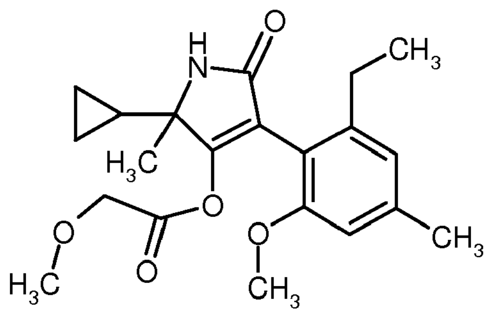

0,18 g der Verbindung gemäß Beispiel I-a-4 wird in 10 ml Dichlormethan gelöst und 0,09 ml Triethylamin hinzugegeben. Anschließend gibt man 0,06 ml Methoxyessigsäurechlorid in 2 ml Dichlormethan hinzu und lässt bei Raumtemperatur über Nacht rühren. Der Ansatz wird auf 10 ml gesättigte Natriumchloridlösung gegeben, die organische Phase abgetrennt und die wässrige Phase mit Dichlormethan gewaschen. Die vereinigten organischen Phasen werden mit Natriumsulfat getrocknet, eingeengt und säulenchromatographisch mit n-Heptan/Essigsäureethylester (Gradient 4: 1 nach 0:100) gereinigt.
Ausbeute: 0,22 g (98 % der Theorie)
$^1$H-NMR (300 MHz, CDCl3): δ = 4.01 (s, 2H, O-$CH_2$-CO), 1.42 (d, 3H, $CH_3$) ppm.

In Analogie zu Beispiel (I-b-1) und gemäß den allgemeinen Angaben zur Herstellung werden folgende Verbindungen der Formel (I-b) erhalten:

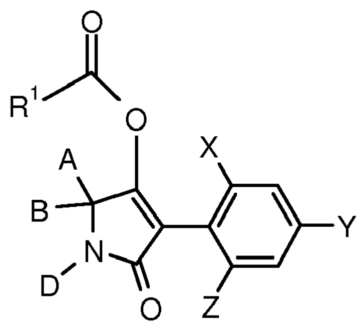

(I-b)

| Bsp.-Nr. | X | Y | Z | D | A | B | $R^1$ | Fp.˚C | Isomer |
|---|---|---|---|---|---|---|---|---|---|
| I-b-2 | $OCH_3$ | $CH_3$ | $C_2H_5$ | H | 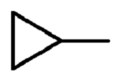 | $CH_3$ | $i\text{-}C_3H_7$ | 125-133 | - |
| I-b-3 | $OCH_3$ | $CH_3$ | $C_2H_5$ | H | $CH_3$ | $CH_3$ | $i\text{-}C_3H_7$ | 142 | - |
| I-b-4 | $OCH_3$ | $CH_3$ | $C_2H_5$ | H | $-CH_2\text{-}CHOC_4H_9\text{-}(CH_2)_3-$ | | $i\text{-}C_3H_7$ | 161-163 | B |
| I-b-5 | $OCH_3$ | $CH_3$ | $C_2H_5$ | $-CH_2\text{-}CHOCH_3\text{-}CH_2-$ | | H | $H_3C\text{-}O\text{-}CH_2$ | *2.32 (s, 3H,Ar-$CH_3$) 4.08 (s, 2H,O-$CH_2$-CO) | - |

*$^1$H-NMR (300 MHz, $CDCl_3$): Verschiebung δ in ppm

Beispiel I-c-1

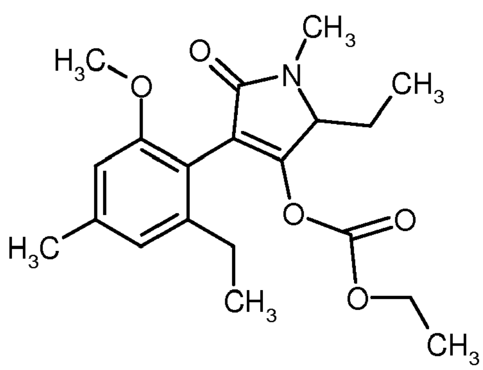

0,2 g der Verbindung gemäß Beispiel I-a-1 wird in 10 ml Dichlormethan gelöst, und 0,11 ml Triethylamin zugegeben. Anschließend dosiert man 0,07 ml Chlorameisensäureethylester portionsweise zu. Man rührt 20 h bei Raumtemperatur und versetzt anschließend mit 8 ml 5 %iger Natriumcarbonatlösung. Die organische Phase wird abgetrennt und chromatographisch mit Essigsäureethylester/n-Heptan (Gradient von 1:4 nach 2:1) an Kieselgel gereinigt.
Ausbeute: 0,135 g (54 % der Theorie), Öl
$^1$H-NMR (300 MHz, $CDCl_3$): δ = 3.72 (d, 3H, O$CH_3$), 4.66 (dq, 2H, N-CH) ppm.

In Analogie zu Beispiel (I-c-1) und gemäß den allgemeinen Angaben zur Herstellung erhält man folgende Verbindungen der Formel (I-c)

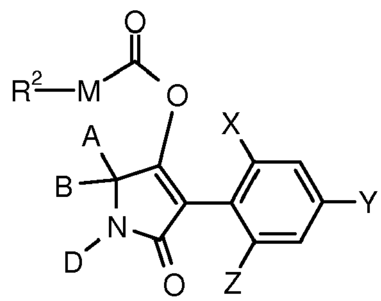

(I-c)

| Bsp.-Nr. | X | Y | Z | D | A | B | M | $R^2$ | Fp.˚C | Isomer |
|---|---|---|---|---|---|---|---|---|---|---|
| I-c-2 | $OCH_3$ | $CH_3$ | $C_2H_5$ | $-(CH_2)_3-$ | | H | O | $C_2H_5$ | Öl* | - |
| I-c-3 | $OCH_3$ | $CH_3$ | $C_2H_5$ | H | $CH_3$ | $CH_3$ | O | $C_2H_5$ | *1.42(d,6H,2x $\underline{CH_3}$) 4.02(q,2H,O $CH_3$) | - |
| I-c-4 | $OCH_3$ | $CH_3$ | $C_2H_5$ | H | $-(CH_2)_2-CHOCH_3-(CH_2)_2-$ | | O | $C_2H_5$ | 144-148 | β |
| I-c-5 | $OCH_3$ | $CH_3$ | $C_2H_5$ | H | 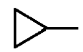 | $CH_3$ | O | $C_2H_5$ | *1.51(d,3H,$CH_3$) 4.01 (q,2H,O $\underline{CH_2}$) | - |
| I-c-6 | $OCH_3$ | $CH_3$ | $C_2H_5$ | H | $-(CH_2)_2-CHCH_3-(CH_2)_2-$ | | O | $C_2H_5$ | *0,95(d,3H, $CH_3$) 4.02(q,2H,O$\underline{CH_2}$) | β |
| I-c-7 | $OCH_3$ | $CH_3$ | $C_2H_5$ | H | $-CH_2-CHOC_4H_9-(CH_2)_3-$ | | O | $C_2H_5$ | 178-181 | B |
| I-c-8 | $OCH_3$ | $CH_3$ | $C_2H_5$ | $-CH_2-CHOCH_3-CH_2-$ | | H | O | $C_2H_5$ | *3.36(s,3H,O $CH_3$) 4.19(q,2H,O $CH_2$) | Gemisch |

*$^1$H-NMR (300 MHz, $CDCl_3$): Verschiebung δ in ppm

Beispiel II-1

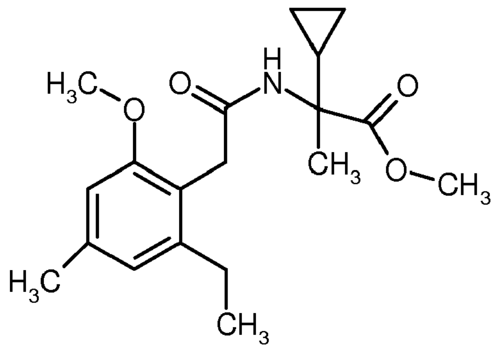

3.88 ml konz. Schwefelsäure vorlegen, 3,77 g der Verbindung gemäß Beispiel XXIV-1 in 20 ml $CH_2Cl_2$ zugeben. 3 h bei 40˚C rühren. 20 ml Methanol zugeben, 4 h bei 60˚C rühren. Über Nacht bei Raumtemperatur rühren. Nach Reaktionsende auf 200 ml $H_2O$ geben, mit 300 ml Essigsäureethylester extrahieren, über Natriumsulfat trocknen, einrotieren. In Essigsäureethylester aufnehmen, mit n-Heptan ausfällen.
Ausbeute: 2,2 g (53 % der Theorie)
$^1$H-NMR (300 MHz, CDCl3): δ = 3.53 (s, 2H, Ar-$CH_2$-CO), 0.26 (m, 2H, Cyclopropyl) ppm.

Beispiel II-2

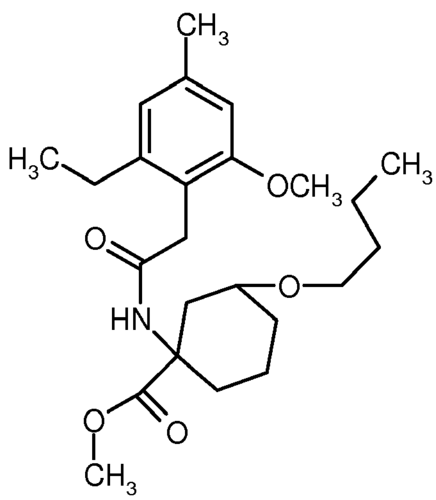

1.52 g trans-3-Butoxy-1-amino-cyclohexancarbonsäure-methylester Hydrochlorid und 3.11 g Kaliumcarbonat in 20 ml Dichlormethan vorlegen. Bei Raumtemperatur 10 min. rühren. 1,5 g der Verbindung gemäß Beispiel XV-1 in 10 ml Dichlormethan bei T >0˚C langsam zutropfen. Bei Raumtemperatur bis zum Reaktionsende rühren (dünnschichtchromatographische Kontrolle). Reaktionsmischung auf 200 ml Wasser geben, 10 min. bei Raumtemperatur rühren, mit Essigsäureethylester extrahieren, organische Phase über Natriumsulfat trocknen und einrotieren.
Ausbeute: 1,7 g (65 % d. Theorie)

Ohne Aufreinigung wird die Verbindung weiter umgesetzt.

In Analogie zu Beispielen (II-1) und (II-2) und gemäß den allgemeinen Angaben zur Herstellung erhält man folgende Verbindungen der Formel (II)

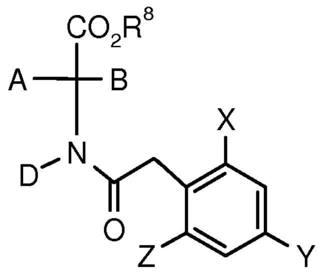

(II)

| Bsp.-Nr. | X | Y | Z | D | A | B | $R^8$ | Fp.˚C | Isomer |
|---|---|---|---|---|---|---|---|---|---|
| II-3 | $OCH_3$ | $CH_3$ | $C_2H_5$ | H | $CH_3$ | $CH_3$ | $CH_3$ | *1.45 (s, 6H, 2 x $\underline{CH_3}$) 3.57 (s, 2H, Ar-$\underline{CH_2}$-CO) | - |
| II-4 | $OCH_3$ | $CH_3$ | $C_2H_5$ | $-CH_2-CHOCH_3-CH_2-$ | | H | $CH_3$ | *3.34 (s, 2H, Ar-$CH_2$-CO) 3.76 (d, 3H, $CO_2CH_3$) | Gemisch |

*$^1$H-NMR (300 MHz, $CDCl_3$): Verschiebung δ in ppm

<u>Beispiel XXIV-1</u>

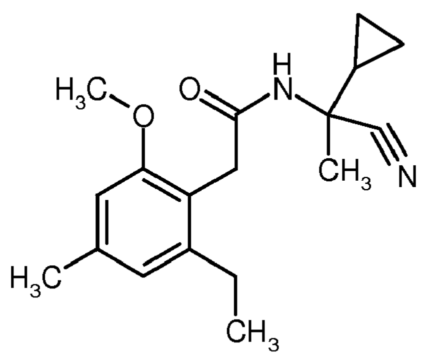

1,46 g 2-Amino-2-cyclopropyl-propionitril und 6,22 g Kaliumcarbonat in 20 ml $CH_2Cl_2$ vorlegen, 10 min. bei Raumtemperatur rühren, 3 g 2-Methoxy-6-ethyl-4-methyl-phenylessigsäurechlorid (XV-1) in 10 ml $CH_2Cl_2$ bei ca. 0˚C langsam zutropfen. Über Nacht bei Raumtemperatur rühren. Nach Reaktionsende (dünnschichtchromatographische Kontrolle) auf 200 ml Wasser geben, 10 min. bei Raumtemperatur rühren, mit Essigsäureethylester extrahieren, organische Phase über Natriumsulfat trocknen, einrotieren.
Ausbeute: 3,77 g (95 % d. Theorie).
Die Verbindung wird ohne Aufreinigung weiter umgesetzt.
$^1$H-NMR (300 MHz, CDCl3): δ = 3.59 (s, 2H, Ar-$CH_2$-CO), 1.71 (s, 3H, NH-C($\underline{CH_3}$) ppm.

<u>Beispiel XV-1</u>

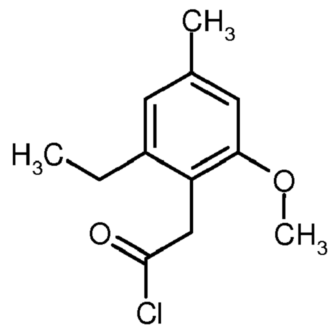

10 g der Verbindung gemäß Beispiel XVIII-1 in 100 ml Dichlormethan vorlegen, 7.923 g (5,45 ml) Oxalylchlorid zugeben und unter Rückfluss rühren bis die Gasentwicklung zu Ende ist. Das Lösungsmittel abrotieren und den Rückstand in 50 ml Dichlormethan aufnehmen und dann einrotieren.

Die Verbindung wird ohne Aufarbeitung weiter umgesetzt.

Beispiel XVIII-1

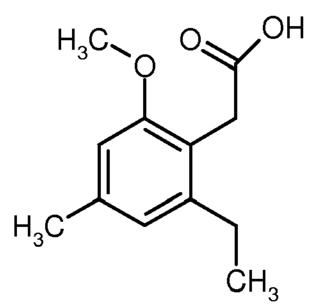

23,49 g der Verbindung gemäß Beispiel XIX-1 in 200 ml Tetrahydrofuran vorlegen, 2,784 g Lithiumhydroxid in 100 ml Wasser zugeben. Über Nacht bei Raumtemperatur rühren, einrotieren. Rückstand mit 500 ml 1N HCl versetzen, 10 min. bei Raumtemperatur rühren, und über eine Fritte absaugen.
Ausbeute: 20 g (91 % d. Theorie), Fp: 112˚C

In Analogie zu Beispiel (XVIII-1) erhält man die Verbindung (XVIII-2)

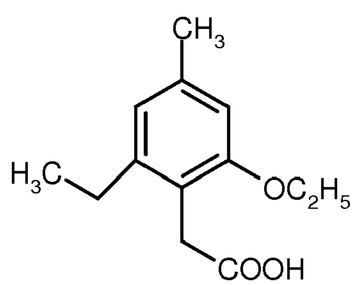

$^1$H-NMR (300 MHz, CDCl3): δ = 1.18 (t, 3H, Ar-$CH_2$-<u>$CH_3$</u>), 4.01 (q, 2H, Ar-O-<u>$CH_2$</u>-$CH_3$) ppm.

Beispiel XIX-1

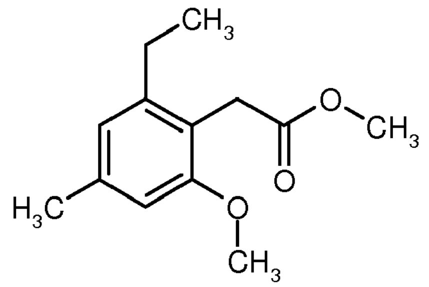

30 g 2-Brom-4-methyl-6-ethyl-phenylessigsäure-methylester bekannt z.B. aus WO 05/044796, WO 05/044791 und 103,77 g Natriummetylat-Lösung 30 %ig, 3,174 g Kupfer-1-bromid und 33,33 ml Essigsäuremethylester werden 7 h bei Rückfluss gerührt, bis das Edukt vollständig umgesetzt ist. Bis zur Trockne einrotieren, Rückstand in Wasser aufnehmen, abfiltrieren. Wasser-Phase mit HCl auf pH 1 stellen, mit Essigsäureethylester extrahieren, organische Phase über Natriumsulfat trocknen, einrotieren.
Ausbeute: 23,49 g (96 % d. Theorie)

$^1$H-NMR (300 MHz, CDCl3): δ = 1.18 (t, 3H, $CH_2$-$\underline{CH_3}$)
2.32 (s, 2H, Ar-$\underline{CH_3}$)
2.57 (q, 2H, $\underline{CH_2}$-$CH_3$)
3.65 (4s, 5H, Ar-$\underline{CH_2}$-CO, Ar-$OCH_3$)
3.78 (s, CO-$OCH_3$)
6.55 (s, 1H, Ar-$H_3$)
6.65 (s, 1H, Ar-$H_5$) ppm.

**Beispiel A**

**Myzus-Test** (Spritzbehandlung)

| | | |
|---|---|---|
| Lösungsmittel: | 78 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.
Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.
Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.
Bei diesem Test zeigen z.B. die folgenden Verbindungen der Herstellungsbeispiele gute Wirksamkeit:
Bei einer Wirkstoffkonzentration von 500 g/ha erreichten folgende Verbindungen einen Abtötungsgrad in % nach 5d von ≥ 90 %: Bsp. I-a-5, I-c-4

**Beispiel B**

**Phaedon-Test** (Spritzbehandlung)

| | | |
|---|---|---|
| Lösungsmittel: | 78 | Gewichtsteile Aceton |

(fortgesetzt)

| | | |
|---|---|---|
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis*) werden mit einer Wirkstoffzubereitung der gewünschten Konzentration gespritzt und nach dem Abtrocknen mit Larven des Meerrettichblattkäfers *(Phaedon cochleariae)* besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z.B. die folgenden Verbindungen der Herstellungsbeispiele gute Wirksamkeit:

Bei einer Wirkstoffkonzentration von 500 g/ha erreichten folgende Verbindungen einen Abtötungsgrad in % nach 7d von ≥ 80 %: Bsp. I-a-6, I-c-4

## Beispiel C

**Tetranychus-Test** (OP-resistent/Spritzbehandlung)

| | | |
|---|---|---|
| Lösungsmittel: | 78 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z.B. die folgenden Verbindungen der Herstellungsbeispiele gute Wirksamkeit:

Bei einer Wirkstoffkonzentration von 500 g/ha erreichten folgende Verbindungen einen Abtötungsgrad in % nach 5d von ≥ 70 %: Bsp. I-a-4, I-b-1, I-c-2, 1-c-8

Bei einer Wirkstoffkonzentration von 100 g/ha erreichten folgende Verbindungen einen Abtötungsgrad in % nach 5d von ≥ 70 %: Bsp. I-b-3, I-b-4, I-c-3

## Beispiel D

1. Herbizide Wirkung im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) formulierten Testverbindungen werden dann als wässrige Suspension mit einer Wasseraufwandmenge von umgerechnet 600 l/ha unter Zusatz von 0,2% Netzmittel in unterschiedlichen Dosierungen auf die Oberfläche der Abdeckerde appliziert.

Nach der Behandlung werden die Töpfe im Gewächshaus aufgestellt und unter guten Wachstumsbedingungen für die Testpflanzen gehalten. Die visuelle Bonitur der Auflaufschäden an den Versuchspflanzen erfolgt nach einer Versuchszeit von 3 Wochen im Vergleich zu unbehandelten Kontrollen (herbizide Wirkung in Prozent (%): 100% Wirkung

= Pflanzen sind abgestorben, 0 % Wirkung = wie Kontrollpflanzen).

Folgende Verbindungen zeigten im Vorauflauf mit 320 g a.i./ha gegen Avena sativa, Lolium multiflorum und Setaria viridis eine Wirkung von ≥ 80 %: I-a-2, I-a-3, I-a-5, I-a-6, I-c-2, I-c-4

2. Herbizide Wirkung im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2-3 Wochen nach der Aussaat werden die Versuchspflanzen im Einblattstadium behandelt. Die als Spritzpulver (WP) formulierten Testverbindungen werden in verschiedenen Dosierungen mit einer Wasseraufwandmenge von umgerechnet 600 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen).

| | Gewächshaus | Dosage (g a.i./ha) | Avena | Lolium | Setaria |
|---|---|---|---|---|---|
| Bsp. I-a-6 | pre-emergence | 320 | 100 | 100 | 100 |
| | Gewächshaus | Dosage (g a.i./ha) | Echinochloa | Lolium | Setaria |
| Bsp. I-a-6 | post-emergence | 320 | 100 | 100 | 90 |
| Bsp. I-a-2 | post-emergence | 320 | 100 | 100 | 100 |

Folgende Verbindungen zeigten im Nachauflauf mit 320 g a.i./ha gegen Avena sativa, Echinocloa crus-galli, Lolium multiflorum und Setaria viridis eine Wirkung von ≥ 90 %: I-a-1, I-a-2, I-a-3, I-a-4, I-a-5, I-a-6, I-b-1, I-c-2, I-c-3, I-c-4, I-c-5

Herbizide Wirkung im Nachauflauf

Samen von mono- bzw. dikotylen Unkraut- bzw. Kulturpflanzen werden in Holzfaserföpfen oder in Plastiktöpfen in sandigem Lehmboden ausgelegt, mit Erde abgedeckt und im Gewächshaus, während der Vegetationsperiode auch im Freien außerhalb des Gewächshauses, unter guten Wachstumsbedingungen angezogen. 2 bis 3 Wochen nach der Aussaat werden die Versuchspflanzen im Ein- bis Drei-Blattstadium behandelt. Die als Spritzpulver (WP) oder Flüssigkeit (EC) formulierten Testverbindungen werden in verschiedenen Dosierungen mit einer Wasseraufwandmenge von umgerechnet 300 l/ha unter Zusatz von Netzmitteln (0,2 bis 0.3 %) auf die Pflanzen und die Bodenoberfläche gespritzt. 3 bis 4 Wochen nach Behandlung der Versuchspflanzen wird die Wirkung der Präparate visuell im Vergleich zu unbehandelten Kontrollen bonitiert (herbizide Wirkung in Prozent (%):

100 % Wirkung = Pflanzen sind abgestorben, 0 % Wirkung = wie Kontrollpflanzen).

Verwendung von Safenern

Soll zusätzlich getestet werden, ob Safener die Pflanzenverträglichkeit von Testsubstanzen bei den Kulturpflanzen verbessern können, werden folgende Möglichkeiten für die Anwendung des Safeners verwendet:

- Samen der Kulturpflanzen werden vor der Aussaat mit der Safenersubstanz gebeizt (Angabe der Safenermenge in Prozent bezogen auf das Samengewicht)
- Kulturpflanzen werden vor Anwendung der Testsubstanzen mit dem Safener mit einer bestimmten Hektaraufwandmenge gespritzt (üblicherweise 1 Tag vor Anwendung der Prüfsubstanzen)
- der Safener wird zusammen mit der Testsubstanz als Tankmischung appliziert (Angabe der Safenermenge in g/ha oder als Verhältnis zum Herbizid).

Durch Vergleich der Wirkung von Testsubstanzen auf Kulturpflanzen, welche ohne und mit Safener behandelt wurden, kann die Wirkung der Safenersubstanz beurteilt werden.

**Gefäßversuche mit Getreide im Gewächshaus**

Mefenpyr 1 Tag vor Herbizidapplikation

| | | 28 Tage nach Applikation | |
|---|---|---|---|
| | Aufwandmenge g a.i./ha | Sommergerste beobachtet (%) | Sommerweizen beobachtet (%) |
| Beispiel I-a-2 | 50<br>25<br>12,5 | 90 | 95<br>60<br>40 |
| Beispiel I-a-2 + Mefenpyr | 50 + 100<br>25 + 100<br>12,5 + 100 | 30 | 30<br>20<br>10 |

**Beispiel E**

Grenzkonzentrations-Test / Bodeninsekten - Behandlung transgener Pflanzen

| | |
|---|---|
| Testinsekt: | Diabrotica balteata - Larven im Boden |
| Lösungsmittel: | 7 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird auf den Boden gegossen. Dabei spielt die Konzentration des Wirkstoffs in der Zubereitung praktisch keine Rolle, entscheidend ist allein die Wirkstoffgewichtsmenge pro Volumeneinheit Boden, welche in ppm (mg/l) angegeben wird. Man füllt den Boden in 0,25 l Töpfe und lässt diese bei 20˚C stehen.

Sofort nach dem Ansatz werden je Topf 5 vorgekeimte Maiskörner der Sorte YIELD GUARD (Warenzeichen von Monsanto Comp., USA) gelegt. Nach 2 Tagen werden in den behandelten Boden die entsprechenden Testinsekten gesetzt. Nach weiteren 7 Tagen wird der Wirkungsgrad des Wirkstoffs durch Auszählen der aufgelaufenen Maispflanzen bestimmt (1 Pflanze = 20 % Wirkung).

**Beispiel F**

Heliothis virescens - Test - Behandlung transgener Pflanzen

| | |
|---|---|
| Lösungsmittel: | 7 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) der Sorte Roundup Ready (Warenzeichen der Monsanto Comp. USA) werden durch Tauchen in die Wirkstoffzubereitung der gewünschten Konzentration behandelt und mit der Tabakknospenraupe Heliothis virescens besetzt, solange die Blätter noch feucht sind.

Nach der gewünschten Zeit wird die Abtötung der Insekten bestimmt.

## Patentansprüche

**1.** Verbindungen der Formel (XVIII)

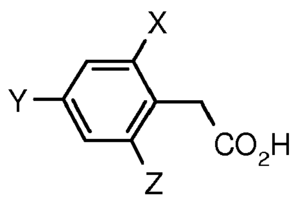

(XVIII)

in welcher

X für Alkoxy steht,
Y für Alkyl steht,
Z für $C_2$-$C_6$-Alkyl steht.

**2.** Verbindungen der Formel (XVIII) gemäß Anspruch 1, in welcher

X für $C_1$-$C_4$-Alkoxy steht,
Y für $C_1$-$C_3$-Alkyl steht,
Z für Ethyl, n-Propyl oder n-Butyl steht.

**3.** Verbindungen der Formel (XVIII) gemäß Anspruch 1, in welcher

X für Methoxy, Ethoxy, n-Propoxy steht,
Y für Methyl oder Ethyl steht,
Z für Ethyl oder n-Propyl steht.

**4.** Verbindungen der Formel (XVIII) gemäß Anspruch 1, in welcher

X für Methoxy oder Ethoxy steht,
Y für Methyl steht,
Z für Ethyl steht.

**5.** Verbindungen der Formel (XVIII) gemäß Anspruch 1, in welcher

X für Methoxy oder Ethoxy steht,
Y für Methyl steht,
Z für Ethyl steht.

Europäisches Patentamt
European Patent Office
Office européen des brevets

# EUROPÄISCHER RECHERCHENBERICHT

Nummer der Anmeldung
EP 10 15 9644

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | WO 97/02243 A1 (BAYER AG [DE]; FISCHER REINER [DE]; BRETSCHNEIDER THOMAS [DE]; HAGEMAN) 23. Januar 1997 (1997-01-23)<br>* Seite 94, 1. Absatz (Formel XXV); Seite 171, Tabelle 36; Anspruch 9 *<br>----- | 1-5 | INV.<br>C07C57/32 |
| A | EP 0 160 962 A2 (CONSORTIUM ELEKTROCHEM IND [DE]) 13. November 1985 (1985-11-13)<br>* Seite 4, 2. Abschnitt;Anspruch 3 *<br>----- | 1-5 | |

RECHERCHIERTE SACHGEBIETE (IPC)

C07C
A01N

Der vorliegende Recherchenbericht wurde für alle Patentansprüche erstellt

1

| Recherchenort | Abschlußdatum der Recherche | Prüfer |
|---|---|---|
| München | 23. Juni 2010 | Schmid, Arnold |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
........................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 10 15 9644

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

23-06-2010

| Im Recherchenbericht angeführtes Patentdokument | Datum der Veröffentlichung | Mitglied(er) der Patentfamilie | Datum der Veröffentlichung |
|---|---|---|---|
| WO 9702243 A1 | 23-01-1997 | AU 707357 B2 | 08-07-1999 |
| | | BR 9609301 A | 25-05-1999 |
| | | CA 2225830 A1 | 23-01-1997 |
| | | CA 2532743 A1 | 23-01-1997 |
| | | CN 1362397 A | 07-08-2002 |
| | | CN 1193960 A | 23-09-1998 |
| | | DE 59610095 D1 | 06-03-2003 |
| | | DK 0835243 T3 | 19-05-2003 |
| | | EP 0835243 A1 | 15-04-1998 |
| | | ES 2189877 T3 | 16-07-2003 |
| | | HU 9802279 A2 | 28-01-1999 |
| | | TR 9701740 T1 | 21-04-1998 |
| | | TW 410141 B | 01-11-2000 |
| | | US 6251830 B1 | 26-06-2001 |
| | | US 5994274 A | 30-11-1999 |
| EP 0160962 A2 | 13-11-1985 | AU 575938 B2 | 11-08-1988 |
| | | AU 4201485 A | 14-11-1985 |
| | | DE 3416772 A1 | 07-11-1985 |
| | | GR 850238 A1 | 25-11-1985 |
| | | HU 37906 A2 | 28-03-1986 |
| | | IL 74643 A | 29-02-1988 |
| | | JP 2047976 B | 23-10-1990 |
| | | JP 60239441 A | 28-11-1985 |
| | | ZA 8503377 A | 24-12-1985 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0262399 A
- GB 2266888 A
- EP 355599 A
- EP 415211 A
- EP 377893 A
- EP 442077 A
- EP 442073 A
- EP 456063 A
- EP 521334 A
- EP 596298 A
- EP 613884 A
- EP 613885 A
- WO 9401997 A
- WO 9526954 A
- WO 9520572 A
- EP 0668267 A
- WO 9625395 A
- WO 9635664 A
- WO 9701535 A
- WO 9702243 A
- WO 9736868 A
- WO 9743275 A
- WO 9805638 A
- WO 9806721 A
- WO 9825928 A
- WO 9916748 A
- WO 9924437 A
- WO 9943649 A
- WO 9948869 A
- WO 9955673 A
- WO 0109092 A
- WO 0117972 A
- WO 0123354 A
- WO 0174770 A
- WO 03013249 A
- WO 04007448 A
- WO 04024688 A
- WO 04065336 A
- WO 04080962 A
- WO 04111042 A
- WO 05044791 A
- WO 05044796 A
- WO 05048710 A
- WO 05049569 A
- WO 05066125 A
- EP 86750 A
- EP 94349 A
- EP 191736 A
- EP 492366 A
- EP 174562 A
- EP 346620 A
- WO 9507897 A
- WO 9107874 A
- EP 269806 A
- EP 333131 A
- WO 9108202 A
- EP 582198 A
- EP 613618 A
- DE 2218097 A
- DE 2350547 A
- DE 19621522 A
- US 6235680 A
- WO 9966795 A
- US 6251827 A
- DE 04030753 A
- WO 9429268 A


### In der Beschreibung aufgeführte Nicht-Patentliteratur


- **S. Suzuki et al.** *Chem. Pharm. Bull.,* 1967, vol. 15, 1120
- **R. Schmierer ; H. Mildenberger.** *Liebigs Ann. Chem.,* 1985, 1095
- *Chem. Reviews,* 1953, vol. 52, 237-416
- **Bhattacharya.** *Indian J. Chem.,* 1968, vol. 6, 341-5
- *Chem. Ind.,* 1968, 1568
- Organikum. VEB Deutscher Verlag der Wissenschaften, 1977, 505
- **Compagnon.** *Miocque Ann. Chim.,* 1970, vol. 5, 11-2223-27
- **L. Munday.** *J. Chem. Soc.,* 1961, 4372
- **J.T. Eward ; C. Jitrangeri.** *Can. J. Chem.,* 1975, vol. 53, 3339
- **Ungerer.** *Chem. Ind.,* 1985, vol. 37, 730-732
- **Williams.** Antifouling Marine Coatings. Noyes, 1973